(19) Europäisches Patentamt / European Patent Office / Office européen des brevets

(11) **EP 4 274 893 B1**

(12) **EUROPEAN PATENT SPECIFICATION**

(45) Date of publication and mention
of the grant of the patent:
**01.01.2025 Bulletin 2025/01**

(21) Application number: **22704407.0**

(22) Date of filing: **05.01.2022**

(51) International Patent Classification (IPC):
**C12N 9/12** *(2006.01)*    **C12N 15/11** *(2006.01)*
**C12N 15/90** *(2006.01)*    **C12N 9/78** *(2006.01)*

(52) Cooperative Patent Classification (CPC):
(C-Sets available)
**C12N 15/11; C12N 9/1276; C12N 9/78;**
**C12Y 207/07049; C12Y 305/04004;**
**C12Y 305/04005; C12Y 305/04006;** C12N 15/907;
C12N 2310/16; C12N 2310/20; C12N 2310/315;
C12N 2310/3519; C12N 2320/34         (Cont.)

(86) International application number:
**PCT/GB2022/050004**

(87) International publication number:
**WO 2022/148955 (14.07.2022 Gazette 2022/28)**

(54) **METHOD FOR PRODUCING GENETICALLY MODIFIED CELLS**

VERFAHREN ZUR HERSTELLUNG GENETISCH MODIFIZIERTER ZELLEN

PROCÉDÉ DE PRODUCTION DE CELLULES GÉNÉTIQUEMENT MODIFIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.01.2021   US 202163133942 P
05.01.2021   US 202163133945 P
19.05.2021   GB 202107188**

(43) Date of publication of application:
**15.11.2023   Bulletin 2023/46**

(73) Proprietor: **REVVITY DISCOVERY LIMITED
Cambridge, Cambridgeshire, CB25 9TL (GB)**

(72) Inventors:
• **LAMBOURNE, John**
  **Cambridge CB25 9TL (GB)**
• **PORRECA, Immacolata**
  **Cambridge CB25 9TL (GB)**
• **SMITH, Amanda**
  **Cambridge CB25 9TL (GB)**

(74) Representative: **Hoffmann Eitle
Patent- und Rechtsanwälte PartmbB
Arabellastraße 30
81925 München (DE)**

(56) References cited:
**WO-A1-2018/129129     WO-A1-2021/055459**

• **TADIC VANJA ET AL: "CRISPR/Cas9-based
epigenome editing: An overview of dCas9-based
tools with special emphasis on off-target
activity", METHODS, ACADEMIC PRESS, NL, vol.
164, 6 May 2019 (2019-05-06), pages 109 - 119,
XP085754554, ISSN: 1046-2023, [retrieved on
20190506], DOI: 10.1016/J.YMETH.2019.05.003**
• **JESSE G. ZALATAN ET AL: "Engineering
Complex Synthetic Transcriptional Programs
with CRISPR RNA Scaffolds", CELL, vol. 160, no.
1-2, 1 January 2015 (2015-01-01), Amsterdam NL,
pages 339 - 350, XP055278878, ISSN: 0092-8674,
DOI: 10.1016/j.cell.2014.11.052**

Remarks:
The complete document including Reference
Table(s) and the Sequence Listing(s) can be
downloaded from the EPO website

(52) Cooperative Patent Classification (CPC): (Cont.)

C-Sets
C12N 2310/321, C12N 2310/3521

**Description**

**FIELD OF THE INVENTION**

The invention relates to a method for producing genetically modified cells, including plant cells, animal cells, mammalian cells, immune cells and stem cells, using RNA-mediated base editing.

**BACKGROUND TO THE INVENTION**

Clustered Regularly Interspaced Short Palindromic Repeat (CRISPR)-Cas technologies have emerged as a revolutionary nucleic acid-guided gene editing tool, enabling precise editing of a gene of interest. Directed by a guide RNA (gRNA), CRISPR-Cas systems induce a double stranded break (DSB) within a gene of interest, thereby resulting in the formation of small insertions or deletions (collectively referred to as 'indels') created by highly variable repair via the Non-Homologous End Joining (NHEJ) pathway. Alternatively, precise genome alterations can be achieved by introduction of a DSB along with co-delivery of a DNA template for repair via homology directed repair (HDR). While this approach is efficient and reliable when simply disrupting a single gene by NHEJ, precision alteration of single nucleotides by HDR is far less efficient. Furthermore, inducing multiple DSBs during multiplexed gene editing procedures can cause undesirable genotoxicity and the formation of potentially oncogenic gross chromosomal translocations. Accordingly, there remains a need in the field for more controlled and safer methods of multiplexed genetic engineering of cells, in particular human cells, with limited induction of toxic DSBs.

Recently an RNA-mediated base editing system was also developed and is described in WO2017/011721 (Rutgers) entitled: 'Nuclease-independent targeted gene editing platform and uses thereof'. This system recruits a base editing enzyme to a target DNA sequence through the guide RNA component of a CRISPR-Cas complex. This system contains a modified guide RNA with a reprogrammable RNA motif (also referred to as an RNA aptamer in the art) at the 3' end, which recruits the cognate RNA binding domain (also referred to as an RNA aptamer ligand in the art) fused to an effector (such as a deaminase effector). Using this system, targeted nucleotide modification was achieved with high precision in prokaryotic cells and eukaryotic cells including mammalian cells; see WO2018129129 and WO2017011721. As disclosed in US application 62/901,584, a new, second generation base editing system with increased specificity and efficacy in prokaryotic cells was tested and further improved in mammalian cells. The second generation system/platform exhibits high specificity, high efficiency, and low off-target liability. With a modular design that fully separates the nucleic acid modification module from the nucleic acid recognition module as well as other advantages disclosed herein, the RNA-mediated base editing platform provides an alternative to recruitment of the effector through fusion to or direct interaction with the sequence-targeting protein, which could not effectively separate sequence-targeting function from nucleic acid modification function. Devoid of the requirements of DNA DSB and HDR, the new system provides powerful tools for genetic engineering and for therapeutic development.

Comparing to the recruitment by direct protein fusion (prior art BE system) or other recruiting approaches by the protein component, the RNA scaffold mediated effector recruitment mechanism has a number of distinct features potentially advantageous both for system engineering and for achieving better functionality. For example, it has a modular design in which the nucleic acid sequence targeting function and effector function reside in different molecules, making it possible to independently reprogram the functional modules. It does not require re-engineering of an individual functional Cas9 fusion protein. Thus, combinations of different modules can be achieved by swapping the nucleotide sequence of the recruiting RNA motif and the cognate RNA binding domain. Recruitment of an effector by direct fusion or direct interaction with the protein component of the sequence-targeting unit, on the other hand, always requires a re-engineering of a new fusion protein, which is technically more difficult with a less predictable outcome. In addition, the fusion effector is smaller in size which could potentially allow more efficient oligomerisation of the functional effector, while direct fusion would preclude the formation of oligomers due to steric hindrance. Moreover, as RNA scaffold mediated base editing does not require generation of a Cas fusion protein, which further increases the gene/transcription size of Cas, the RNA mediated base editing system could potentially be constructed in a way that is more efficient for packaging and delivery by viral vectors.

Despite the advantages of the above-described RNA-mediated base editing system, significant optimisation is still required. The efficiency of base editing enzymes such as deaminases is highly dependent on the sequence of the target nucleic acid. Therefore, when using the RNA-mediated base editing system, the effector must be carefully selected based on the target sequence. The process of selecting the optimal effector and testing the system can be time-consuming, and therefore it is desirable to develop a more efficient base editing system.

**SUMMARY OF THE INVENTION**

The inventors of the present invention have a developed a novel RNA mediated base editing system that enables recruitment of multiple effectors to a single target site, allowing for highly efficient base editing.

Previously, it was assumed that the RNA-aptamer mediated base editing system described in WO2017/011721 involved irreversible recruitment of a single aptamer ligand enabling precise modification to a target genomic locus. However, surprisingly the inventors have found that the recruitment mechanism is in fact reversible, which has implications for improving the efficacy of base editing. Based on this finding, the inventors have carefully devised a system which enables recruitment of multiple effectors to a single genomic locus to enable a single or multiple highly efficient edits within this region. Further, the system can be simultaneously applied to edit multiple different genomic loci (multiplexed base editing).

The present invention is defined by the claims.

In a first aspect, the invention provides a method for genetically modifying a cell by base editing, the method comprising introducing into the cells:

    i) a sequence-targeting protein;
    ii) an RNA-ligand binding complex comprising

        a) a guide RNA, and
        b) a ligand binding moiety; and

    iii) two or more effector proteins comprising

        c) a ligand capable of reversibly binding to the ligand binding moiety, and
        d) an effector domain,

wherein the two or more effector proteins each comprise different effector domains.

In an embodiment, a method for genetically modifying a cell by base editing, the method comprising providing within the cell for example, through introduction into the cell:

    i) a sequence-targeting protein;
    ii) an RNA-ligand binding complex comprising

        e) a guide RNA, and
        f) a ligand binding moiety; and

    iii) two or more effector proteins comprising

        g) a ligand capable of reversibly binding to the ligand binding moiety, and
        h) an effector domain,

wherein the two or more effector proteins each comprise different effector domains.

In an embodiment, the guide RNA (or gRNA) comprises a crRNA comprising a targeting sequence that is complementary to a target nucleic acid. In an embodiment, the guide RNA further comprises a tracrRNA or scoutRNA capable of binding to the sequence-targeting protein. Thus, in an embodiment the guide RNA comprises solely a crRNA. In another embodiment the guide RNA comprises a crRNA and a tracrRNA. In an alternative embodiment the guide RNA comprises a crRNA and a scoutRNA.

In an embodiment, the sequence-targeting protein is a Cas protein.

When an effector is attached to (or contains) a ligand, the system has a modular design. The presence of the ligand binding moiety within the RNA-ligand binding complex allows the complex to associate with the corresponding ligand associated with (or contained with) the effector. Thus, the ligand binding moiety is associated with the guide RNA in a manner and orientation that allows it to be capable of reversibly associating with a ligand. Similarly, the ligand is attached to or associated with the effector in a manner that renders it capable of reversibly associating with the ligand binding moiety.

When an effector (or 'effector domain') is attached to (or contains) a ligand, the system has a modular design. The presence of the ligand binding moiety within the RNA-ligand binding complex allows the complex to associate with the corresponding ligand associated with (or contained within) the effector domain. Thus, the ligand binding moiety is associated with the guide RNA in a manner and orientation that allows it to be capable of reversibly associating with a ligand. Similarly, the ligand is attached to or associated with the effector domain in a manner that renders it capable of reversibly associating with the ligand binding moiety.

When the ligand and the ligand binding moiety are associated with each other, the effector that is attached to or associated with the ligand will become part of any base editing complex that contains the RNA-ligand binding complex. When the base editing complex also contains a Cas protein, that Cas protein and the effector can be retained in the same locality, e.g. at or near a target site of interest.

Thus, to use a particular effector with a Cas protein, the effector needs to be associated with the ligand that is capable of reversibly associating with the ligand binding moiety that is part of the base editing complex that contains that Cas protein. To change the effector from one system to the next, only the effector-ligand needs to be changed. Consequently, the same RNA-ligand binding complex and its associated Cas protein can be used with a plurality of different effectors. The plurality of different effectors can be used simultaneously or sequentially in the same system by associating and dissociating their ligands with the ligand binding moieties or simultaneously or sequentially in different systems.

In a preferred embodiment, the ligand binding moiety of the RNA-ligand binding complex is an RNA motif, and the ligand of the effector protein is an RNA binding domain. In a preferred embodiment, the ligand binding moiety of the RNA-ligand binding complex is an RNA motif, and the ligand of the effector protein is an RNA binding domain that recognises and is capable of binding to the RNA motif. In embodiments where the RNA-ligand binding complex comprises a guide RNA and an RNA motif, it is referred to as an RNA scaffold.

When an effector is attached to (or contains) an RNA binding domain, the system has a modular design. The presence of the RNA motif within the RNA-ligand binding complex (RNA scaffold) allows the complex to associate with the corresponding RNA binding domain associated with (or contained with) the effector. Thus, the RNA motif is associated with the guide RNA in a manner and orientation that allows it to be capable of reversibly associating with the RNA binding domain. Similarly, the RNA binding domain is attached to or associated with the effector in a manner that renders it capable of reversibly associating with the RNA motif.

When an effector (or 'effector domain') is attached to (or contains) an RNA binding domain, the system has a modular design. The presence of the RNA motif within the RNA-ligand binding complex (RNA scaffold) allows the complex to associate with the corresponding ligand associated with (or contained within) the effector domain. Thus, the RNA motif is associated with the guide RNA in a manner and orientation that allows it to be capable of reversibly associating with the RNA binding domain. Similarly, the RNA binding domain is attached to or associated with the effector domain in a manner that renders it capable of reversibly associating with the RNA motif.

When the RNA binding domain and the RNA motif are associated with each other, the effector that is associated with the RNA binding domain will become part of any base editing complex that contains the RNA-ligand binding complex (RNA scaffold). When the base editing complex also contains a Cas protein, that Cas protein and the effector can be retained in the same locality, e.g. at or near a target site of interest.

Thus, the invention provides a method in which a particular effector can be used with a Cas protein, and wherein the effector protein comprises an RNA binding domain that is capable of reversibly associating with the RNA motif that is part of the base editing complex that contains that Cas protein. To change the effector from one system to the next, only the effector-RNA binding domain needs changing. Consequently, the same RNA-ligand binding complex (RNA scaffold) and its associated Cas protein can be used with a plurality of different effectors. The plurality of different effectors can be used simultaneously or sequentially in the same system by associating and dissociating their RNA binding domains with the RNA motifs or simultaneously or sequentially in different systems.

In any embodiment of the invention, the two or more effector proteins each comprise a ligand capable of reversibly binding to the same ligand binding moiety in the RNA-ligand binding complex. In other words, the two or more effector proteins are capable of interacting with a common ligand binding moiety, and thus can each be recruited by the same RNA-ligand binding complex. The two or more effector proteins each comprise different effector domains, and each comprise a ligand capable of reversibly binding to the same ligand binding moiety.

In an embodiment of the invention, the two or more effector proteins each comprise an RNA binding domain capable of reversibly binding to the same RNA motif in the RNA scaffold. In other words, the two or more effector proteins are capable of interacting with a common RNA motif, and thus can each be recruited by the same RNA scaffold. The two or more effector proteins each comprise different effector domains, and each comprise an RNA binding domain capable of reversibly binding to the same RNA motif.

In an embodiment, the RNA binding domain may be a monomer, dimer or multimer. In a preferred embodiment the RNA binding domain may form a dimer, such that a dimeric RNA binding protein and its associated effector domain(s) reversibly associate with a single RNA motif.

The RNA motif and/or the RNA binding domain has a binding affinity with a dissociation constant ($K_d$) of less than 150 nM. Typically, the $K_d$ is between 1-150 nM, 5 - 150 nM, 10 - 150 nM; 15 - 150 nM; 20 - 150 nM, 25 - 150 nM, 30 -150 nM 40 - 150 nM, 50 - 150 nM 60 -150 nm, more typically the $K_d$ is between, 1- 10 nM: 1-20 nM, 1- 30 nM, 1- 40 nM 1 - 50 nM; 1-60 nM 1- 70 nM; 1-80 nM; 1-90 nM. A dissociation constant greater than 150 nM leads to reduced efficiency of base editing.

In an embodiment of the invention there is provided a method wherein the RNA motif and the RNA binding domain pair are selected from the group consisting of: a telomerase Ku binding motif and Ku protein or an RNA-binding section thereof, a telomerase Sm7 binding motif and Sm7 protein or an RNA-binding section thereof, a MS2 phage operator stem-loop and MS2 coat protein (MCP) or an RNA-binding section thereof, a PP7 phage operator stem-loop and PP7 coat protein (PCP) or an RNA-binding section thereof, a SfMu phage Com stem-loop and Com RNA binding protein or an RNA-binding section thereof.

In an embodiment, the effector domains of the two or more effector proteins have the same or different functions. In an embodiment the effector domain is selected from the group consisting of an enzyme or protein fragment. The effector domain in one embodiment is an enzyme or protein fragment with enzymatic activity, for example in an embodiment the effector domain has deamination activity such as, in one embodiment cytidine or adenine deamination activity.

In one embodiment the methods provided herein that use an effector domain having cytidine deamination activity are a wild type or genetically engineered version of AID, CDA, APOBEC1, APOBEC3A, APOBEC3B, APOBEC3C, APOBEC3D, APOBEC3F, APOBEC3G, APOBEC3H or other APOBEC family enzymes. The effector domain having adenine deamination activity are in an embodiment, a wild type or genetically engineered version of ADA, ADAR, ADAR1 family enzymes, or tRNA adenosine deaminases TadA, TADA, TAD3.

In an embodiment, the effector protein further comprises a linker. Advantageously, the linker allows for greater control in the three dimensional space of the ligand and in turn the effector to be used. In an embodiment, the effector protein further comprises a linker configured to link the ligand and the effector domain.

In an embodiment, the sequence-targeting component and/or the effector protein comprises one or more nuclear localisation signals (NLSs).

In an embodiment, the target nucleic acid is extrachromosomal DNA or genomic DNA on a chromosome.

In an embodiment of the invention there is provided a method wherein the sequence-targeting component comprising a sequence-targeting protein is fused to one or more uracil DNA glycosylase (UNG) inhibitor peptide(s) (UGI).

In an embodiment, the sequence-targeting protein is a Type II Cas protein. In an embodiment, the sequence-targeting protein is a Type II Cas protein that is nuclease null or has nickase activity and/or comprises the sequence of dCas9 or nCas9 of a species selected from the group consisting of *Streptococcus pyogenes, Streptococcus agalactiae, Staphylococus aureus, Streptococcus thermophiles, Neisseria meningitides* and *Treponema denticola.* In an embodiment, the sequence-targeting protein comprises the sequence of dCas9 or nCas9 of *Staphylococus auricularis.*

In an embodiment, the sequence-targeting protein is a Type V Cas protein. In an embodiment, the sequence-targeting protein is a Type V Cas protein that is nuclease null or has nickase activity and/or comprises the sequence of dCas12 or nCas12 of a species selected from the group consisting of *Francisella cf. novicida Fx1, Deltaproteobacteria* bacterium, *Alicyclobacillus acidoterrestri, Planctomycetes* bacterium, *Oleiphilus* sp., Bacterium CG09_39_24, Uncultured archaeon, *Bacillus thuringiensis, Cyanothece* sp., *Rothic dentocariosa, Candidatus Microrchoeoto archaeon, Gordonia otitidis, Freshwater metagenome, Hypersaline lake sediment metagenome* or *Hot springs metagenome.* In an embodiment, the sequence-targeting protein is a Type V Cas protein that is nuclease null or has nickase activity and/or comprises the sequence of dCas12 or nCas12 of a species selected from the group consisting of *Francisella cf. novicida Fx1, Deltaproteobacteria* bacterium, *Alicyclobacillus acidoterrestri, Planctomycetes* bacterium, *Oleiphilus* sp., Bacterium CG09_39_24, *Bacillus thuringiensis, Cyanothece* sp., *Rothic dentocariosa, Candidatus Micrarchaeota archaeon, Gordonia otitidis.*

In an embodiment of the invention there is provided a method wherein the cell undergoes base editing at a single genomic locus. In an embodiment, the cell undergoes base editing at two or more genomic loci. In an embodiment, the cell is a eukaryotic cell or a prokaryotic cell. In an embodiment, the cell is a mammalian cell or plant cell. In an embodiment, the cell is a human cell. In an embodiment the cell is an immune cell (for example, a T cell, B cell, Natural Killer (NK cell), CD34+ hematopoietic stem progenitor cell (HSPC) or macrophage), stem cell or cancer cell. In a preferred embodiment, the cell is a T cell.

In an embodiment of the invention there is provided a method wherein the genetic modification involves at least two genetic modifications within the same genetic locus. In an embodiment, the genetic modification is a point mutation. The point mutation introduces a premature stop codon, disrupts a start codon, disrupts a splice site or corrects a genetic mutation.

In an embodiment of the invention there is provided a method wherein the RNA-ligand binding complex is introduced into the cell as chemically synthesised RNA. In an embodiment, the RNA-ligand binding complex comprises one or more modifications. In an embodiment, the RNA-ligand binding complex may comprise one or more modifications as described herein below. For example, the guide RNA may be chemically modified to comprise 2'-O-methyl phosphorothioate modification on at least one 5' nucleotide and at least one 3' nucleotide of the guide RNA. In an embodiment, the RNA-ligand binding complex is synthesised as a single component or two separate components. In an embodiment, the RNA-ligand binding complex is synthesised as two separate components, wherein the first component comprises a) the crRNA and/or b) the ligand binding moiety and the second component comprises c) the tracrRNA or scoutRNA and/or d) the ligand binding moiety. The two components are allowed to hybridise prior to introduction into the cell. In an embodiment, the RNA-ligand binding complex is a synthetic RNA component(s).

In an embodiment of the invention, the RNA scaffold used in the methods herein is introduced into the cell as chemically synthesised RNA. In an embodiment, the RNA scaffold comprises one or more modifications. In an embodiment, the RNA scaffold comprises one or more modifications as described herein below. For example, the guide RNA sequence may be chemically modified to comprise 2'-O-methyl phosphorothioate modification on at least one 5' nucleotide and at least one 3' nucleotide of the guide RNA. In an embodiment, the RNA scaffold is synthesised as a single component or two separate components. In an embodiment, the RNA scaffold is synthesised as two separate components, wherein the first component comprises a) the crRNA and/or b) the RNA motif and the second component comprises c) the tracrRNA or scoutRNA and/or d) the RNA motif. The two components are allowed to hybridise prior to introduction into the cell. In an embodiment, the RNA scaffold is a synthetic RNA component(s).

In an embodiment of the invention, the RNA-ligand binding complex comprises one or more modifications. In an embodiment of the invention, the one or more modifications may increase the stability of the RNA-ligand binding complex. In an embodiment the one or modifications may increase the stability of the RNA-ligand binding complex, by increasing resistance of the complex to nuclease degradation. In an embodiment, the guide RNA is chemically modified to comprise at least one 2'-(O-methyl phosphorthioate) modification.

In an embodiment of the invention, there is provided a method wherein one or more ligand binding moieties are located at the 3' end of, 5' end of or within the guide RNA. In a preferred embodiment, the RNA-ligand binding complex (or RNA scaffold) may comprise one or more RNA motifs enabling recruitment of one or more effectors. In a preferred embodiment, the RNA-ligand binding complex (or RNA scaffold) may comprise one or more RNA motifs enabling recruitment of one or more effector proteins. In an embodiment, each RNA motif recruits either a single or multiple effector proteins. In an embodiment where the RNA-ligand binding complex (RNA scaffold) comprises more than one RNA motif, the RNA motifs are the same or different. In an embodiment where there is more than one RNA motif and they are different, each RNA motif recruits either a single or multiple effector proteins. In an embodiment where there is more than one RNA motif and they are different, at least one RNA motif recruits multiple effector proteins. In an embodiment where multiple effector proteins are recruited, these proteins have the same or different functions. In any embodiment where multiple effector proteins are recruited, the effector domains of the effector proteins have the same or different functions.

In a preferred embodiment, at least one RNA motif is an MS2 aptamer, optionally wherein the MS2 aptamer has an extended stem. In an embodiment, the extended stem comprises 2 to 24 nucleotides. In an embodiment, at least one RNA motif is a PP7 aptamer. In an embodiment, the MS2 aptamer and PP7 aptamer recruit a single effector protein or multiple effector proteins each. In an embodiment, the MS2 aptamer and PP7 aptamer recruit the same or different effector proteins.

In an embodiment, the sequence targeting protein comprises nCas9 with one or two UGIs and the ligand binding moiety is a single MS2 RNA motif or PP7 RNA motif located at the 3' end of the RNA-ligand binding complex.

In an embodiment, the sequence targeting protein comprises nCas9 with one or more UGIs and the ligand binding moiety is a single MS2 RNA motif or PP7 RNA motif located at the 3' end of the RNA-ligand binding complex.

In an embodiment of the invention, there is provided a method wherein the components are in the form of RNA or protein, for example the sequence-targeting component, the RNA-ligand binding complex and the effector protein may all be introduced into the cell as RNA or protein; this means that the method/system provided herein could, if desired, be used a vector-free system.

A population of genetically modified cells obtained according to the method for genetically modifying a cell by base editing, may comprise at least 10% of the cells comprising the genetic modification(s).

In a further aspect, the invention provides a kit for genetically modifying a cell by base editing comprising:

    i) a sequence-targeting protein;
    ii) an RNA-ligand binding complex comprising

        a) a crRNA comprising a targeting sequence that is complementary to a target nucleic acid,
        b) a tracrRNA capable of binding to the sequence-targeting protein, and
        c) a ligand binding moiety; and

    iii) two or more effector proteins comprising

        d) a ligand capable of reversibly binding to the ligand binding moiety,
        e) an effector domain having a cytidine deamination activity or adenine deamination activity,

wherein the two or more effector proteins each comprise different effector domains.

In an embodiment of the kit described in the aspect of the invention, the effector proteins further comprise a linker.

In an embodiment of the kit described in the aspect of the invention, the ligand binding moiety is an RNA motif, and the ligand is an RNA binding domain.

In a further aspect, the invention provides a kit for genetically modifying a cell by base editing comprising:

i) a sequence-targeting protein;
ii) an RNA-ligand binding complex

a) a guide RNA, and
b) a ligand binding moiety; and

iii) two or more effectors comprising

c) a ligand capable of reversibly binding to the ligand binding moiety, and
d) an effector domain,

wherein the two or more effector proteins each comprise different effector domains.

In an embodiment of the kit described in the aspect of the invention, the guide RNA comprises a crRNA comprising a targeting sequence that is complementary to a target nucleic acid. In an embodiment of the kit described in the aspect of the invention, the guide RNA further comprises a tracrRNA or scoutRNA capable of binding to the sequence-targeting protein.

In an embodiment of the kit described in the aspect of the invention, the ligand binding moiety is an RNA motif, and the ligand is an RNA binding domain.

In a further aspect, the invention provides a method for genetically modifying a cell by base editing, the method comprising introduction into the cells and/or expression in the cells of:

i) a sequence-targeting component comprising

a) a sequence-targeting protein comprising an SH3 domain, and
b) an effector domain comprising an SH3 domain, wherein (a) and (b) are joined by an SH3-mediated link;

ii) a RNA-ligand binding complex comprising

c) a guide RNA, and
d) a ligand binding moiety; and

iii) two or more effector proteins comprising

e) a ligand capable of reversibly binding to the ligand binding moiety, and
f) an effector domain,

wherein the two or more effector proteins each comprise different effector domains.

In an embodiment of the kit described in the fifth aspect of the invention, the guide RNA comprises a crRNA comprising a targeting sequence that is complementary to a target nucleic acid. In an embodiment of the kit described in the fifth aspect of the invention, the guide RNA further comprises a tracrRNA or scoutRNA capable of binding to the sequence-targeting protein.

In an embodiment of the kit described in the fifth aspect of the invention, the ligand binding moiety is an RNA motif, and the ligand is an RNA binding domain.

In a further aspect, the invention provides a method for genetically modifying a cell by base editing, the method comprising introduction into the cells and/or expression in the cells of:

i) a sequence-targeting component comprising

a) sequence-targeting protein fused to
b) an effector domain;

ii) an RNA-ligand binding complex comprising

c) a guide RNA, and

d) a ligand binding moiety; and

iii) two or more effector proteins comprising

e) a ligand capable of reversibly binding to the ligand binding moiety, and
f) an effector domain,

wherein the two or more effector proteins each comprise different effector domains.

In an embodiment of the kit described in the sixth aspect of the invention, the guide RNA comprises a crRNA comprising a targeting sequence that is complementary to a target nucleic acid. In an embodiment of the kit described in the sixth aspect of the invention, the guide RNA further comprises a tracrRNA or scoutRNA capable of binding to the sequence-targeting protein.

In an embodiment of the kit described in the sixth aspect of the invention, the ligand binding moiety is an RNA motif, and the ligand is an RNA binding domain.

A cell line derived from at least one cell may be prepared according to the methods disclosed herein.

## BRIEF DESCRIPTION OF THE FIGURES

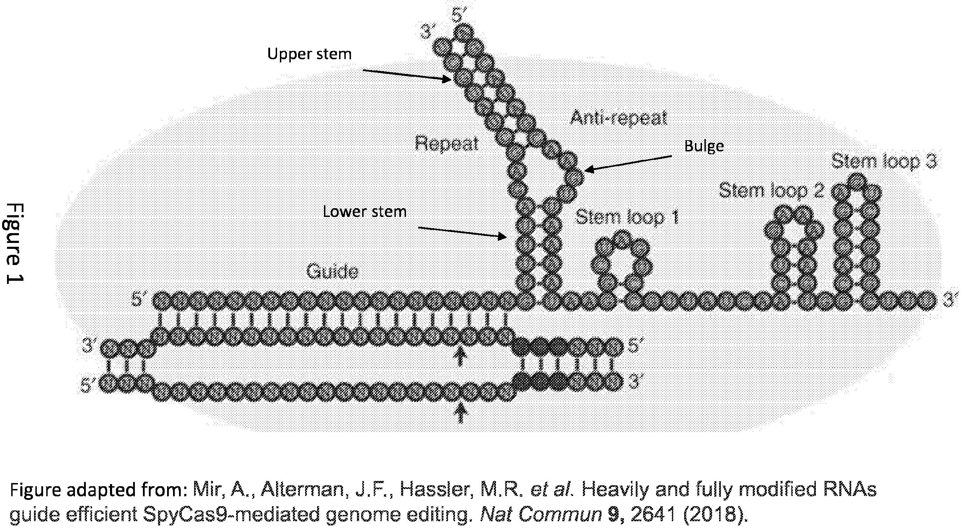

Figure 1- Depiction of an exemplary gRNA molecule for use with a type II Cas enzyme that details the key features; lower stem, upper stem, repeat, anti-repeat, bulge, stem loop 1, stem loop 2, stem loop 3.

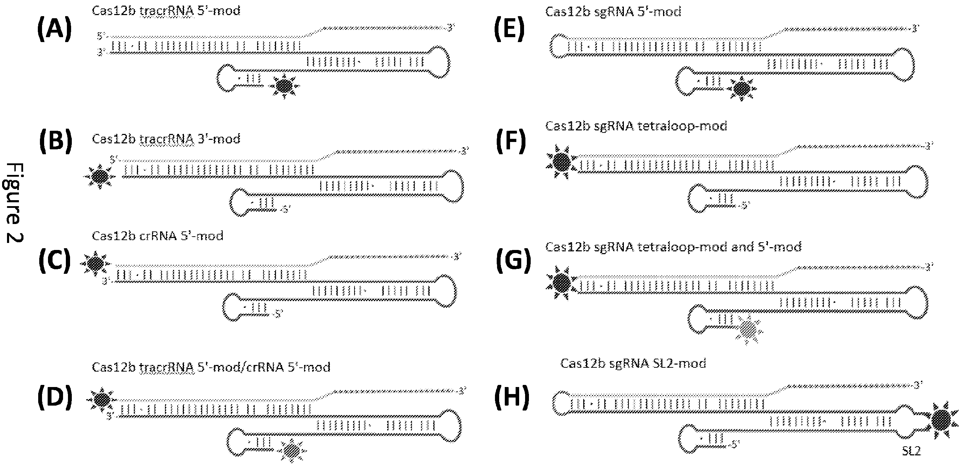

Figure 2 - Depictions of exemplary gRNA sequences for use with Cas12b proteins showing the potential location of one or more modifications to include aptamer sequences, indicated with a star. (A) A gRNA with an aptamer sequence positioned at the 5' of the tracrRNA. (B) A gRNA with an aptamer sequence positioned at the 3' of the tracrRNA. (C) A gRNA with an aptamer sequence positioned at the 5' of the crRNA. (D) A gRNA with an aptamer sequence positioned at the 5' of the crRNA and a second aptamer sequence positioned at the 5' of the tracrRNA. (E) A sgRNA with an aptamer sequence positioned at the 5' end of the sgRNA. (F) A sgRNA with an aptamer sequence positioned as an extension of the tetraloop. (G) A sgRNA with an aptamer sequence positioned as an extension on the tetraloop and a second aptamer sequence positioned at the 5' end of the sgRNA. (H) A sgRNA with an aptamer sequence positioned as an extension to stem loop 2. An RNA-ligand binding complex comprising a gRNA (guide RNA) and aptamer sequence(s), wherein the aptamer sequence(s) is an RNA motif, may also be referred to as an RNA scaffold.

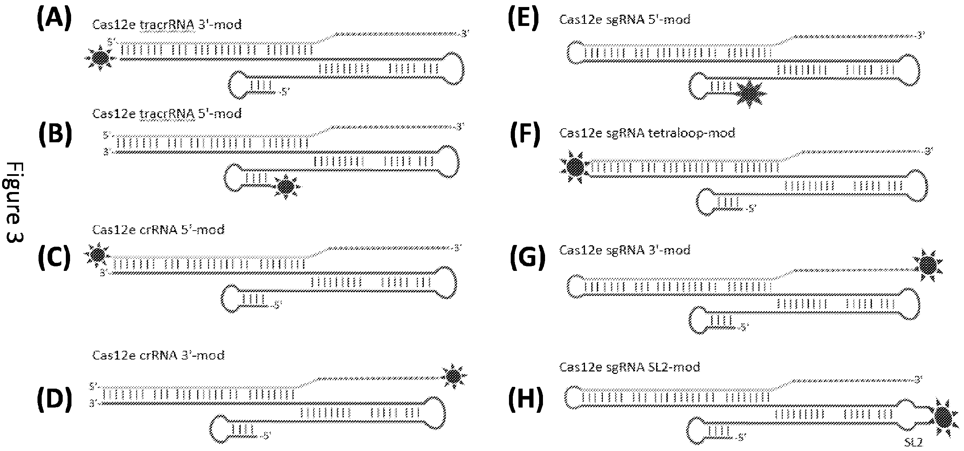

Figure 3 - Depictions of exemplary gRNA sequences for use with Cas12e enzymes showing the potential location of a modification to include an aptamer sequence, indicated with a star. (A) A gRNA with an aptamer sequence positioned at the 3' of the tracrRNA. (B) A gRNA with an aptamer sequence positioned at the 5' of the tracrRNA. (C) A gRNA with an aptamer sequence positioned at the 5' of the crRNA. (D) A gRNA with an aptamer sequence positioned at the 3' of the crRNA (E) A sgRNA with an aptamer sequence positioned at the 5' end of the sgRNA. (F) A sgRNA with an aptamer sequence positioned as an extension of the tetraloop. (G) A sgRNA with an aptamer sequence positioned at the 3' end of the sgRNA. (H) A sgRNA with an aptamer sequence positioned as an extension to stem loop 2. An RNA-ligand binding complex comprising a gRNA (guide RNA) and aptamer sequence(s), wherein the aptamer sequence(s) is an RNA motif, may also be referred to as an RNA scaffold.

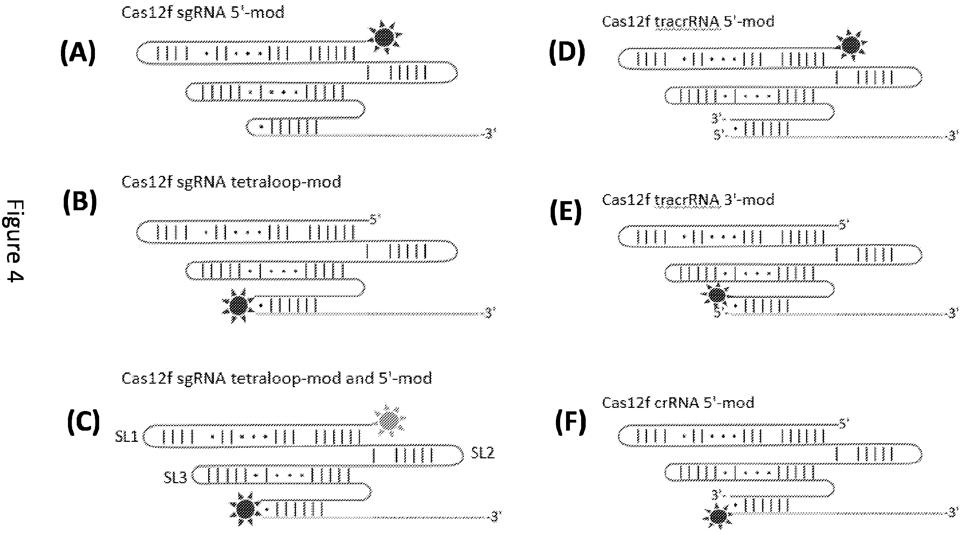

Figure 4 - Depictions of exemplary gRNA sequences for use with Cas12f enzymes showing the potential location of one or more modifications to include aptamer sequences, indicated with a star. (A) A sgRNA with an aptamer sequence positioned at the 3' of the sgRNA. (B) A sgRNA with an aptamer sequence positioned as an extension of the tetraloop. (C) A sgRNA with an aptamer sequence positioned as an extension on the tetraloop and a second aptamer sequence positioned at the 5' end of the sgRNA. (D) A gRNA with an aptamer sequence positioned at the 5' of the tracrRNA (E) A gRNA with an aptamer sequence positioned at the 3' end of the tracrRNA. (F) A gRNA with an aptamer sequence positioned at the 5' of the crRNA An RNA-ligand binding complex comprising a gRNA (guide RNA) and aptamer sequence(s), wherein the aptamer sequence(s) is an RNA motif, may also be referred to as an RNA scaffold.

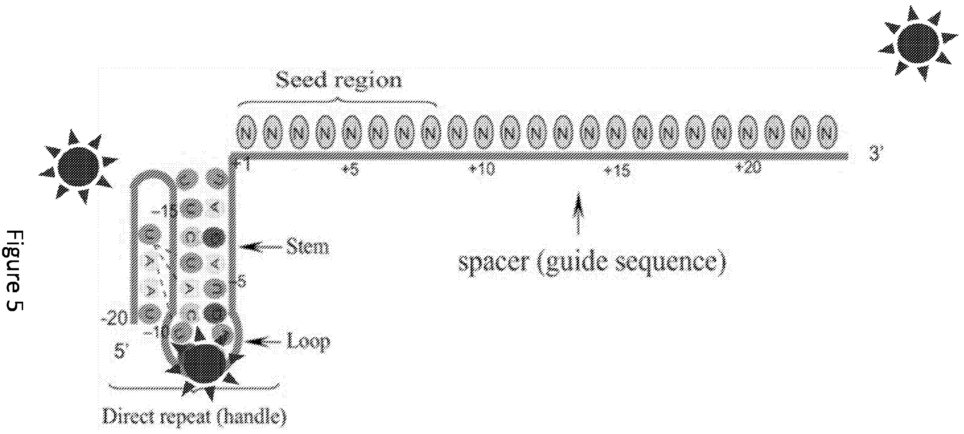

Figure 5 - A depiction of an exemplary gRNA for use with Cas12a showing the potential locations for the inclusion of the aptamer sequence. Locations of one or more modifications to include an aptamer sequence, indicated with a star: at the 5' base of the direct repeat, positioned at the loop of the direct repeat or at the 3' end of the gRNA. An RNA-ligand binding complex comprising a gRNA (guide RNA) and aptamer sequence(s), wherein the aptamer sequence(s) is an RNA motif, may also be referred to as an RNA scaffold.

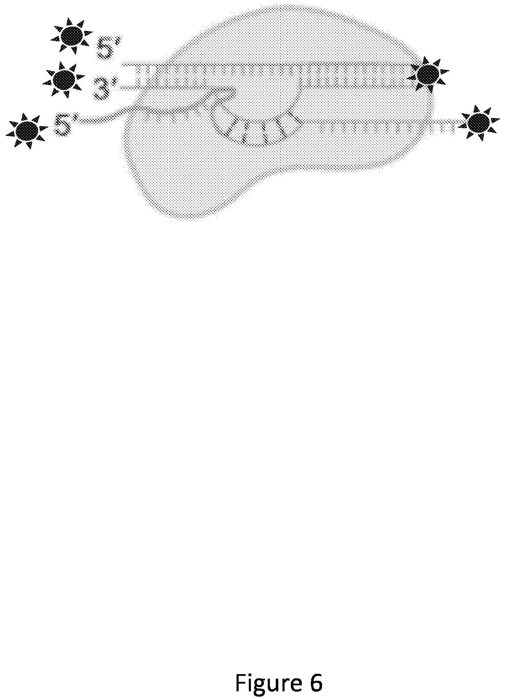

Figure 6 - A depiction of an exemplary gRNA for use with Cas12c and Cas12d systems showing the potential locations for the inclusion of the aptamer sequence. Locations of one or more modifications to include an aptamer sequence, indicated with a star: at the 5' of the scoutRNA, 3' of the scoutRNA, at the stem of the scoutRNA. An RNA-ligand binding complex comprising a gRNA (guide RNA) and aptamer sequence(s), wherein the aptamer sequence(s) is an RNA motif, may also be referred to as an RNA scaffold.

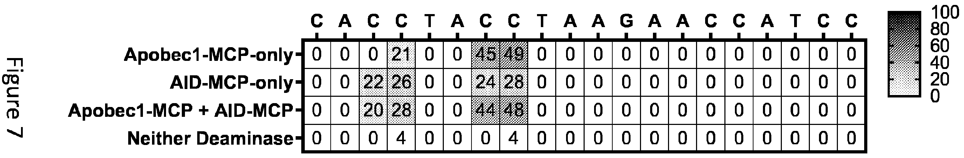

Figure 7 - Editing efficiency of pin point system employing a single aptamer recruiting, Apobec only, AID only and Apobec and AID combined at PCD1 locus. Editing efficiency of pin point system employing a single ligand binding moiety (RNA motif) recruiting, Apobec only, AID only and Apobec and AID combined. Depicting the percentage of cytosine to thymine nucleobase transitions.

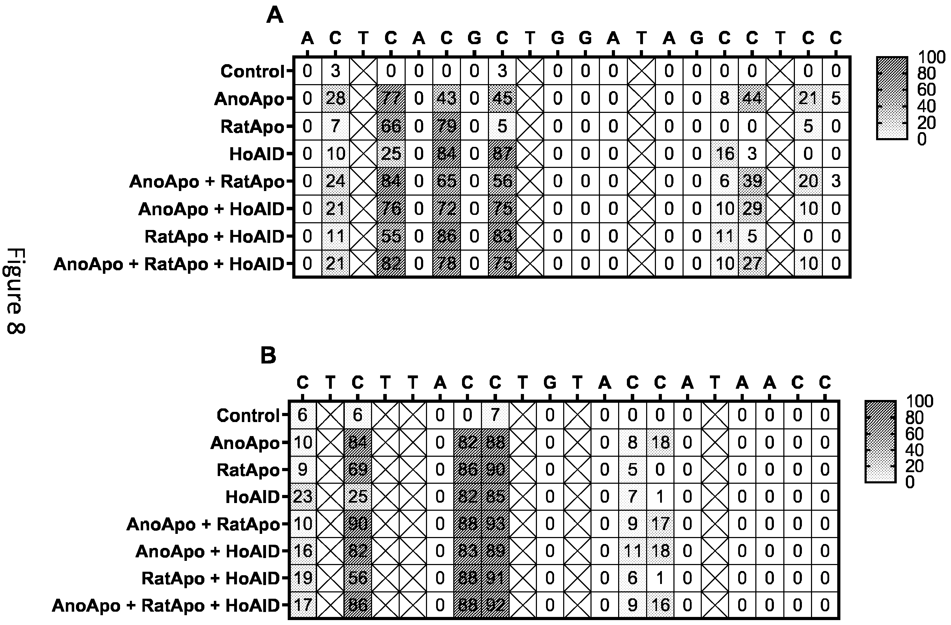

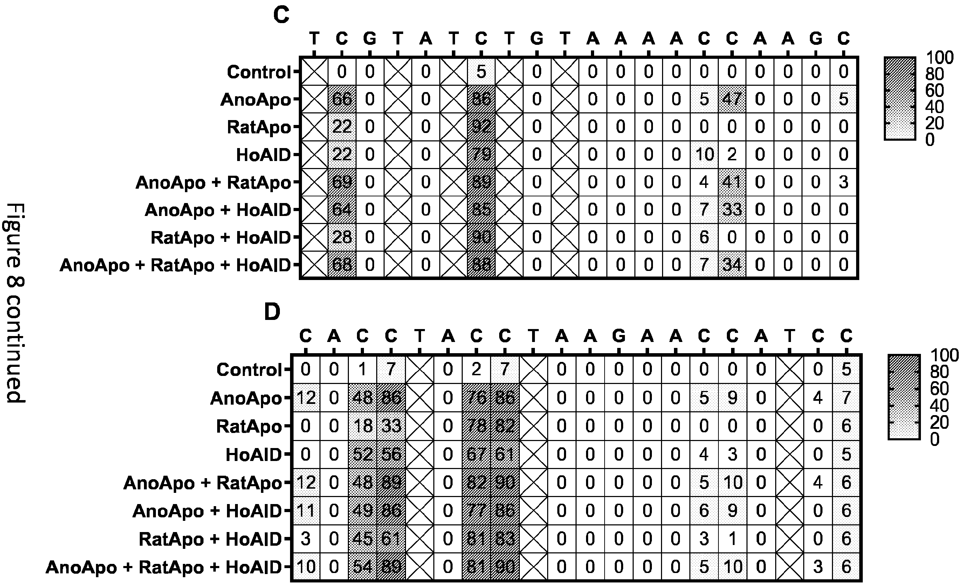

Figure 8 - Single Aptamer (ligand binding moiety) multi-deaminase recruitment base editing at multiple loci simultaneously for novel base editing spacer profiles. Tetraplex gene KO base editing with synthetic sgRNA-Aptamers (RNA-ligand binding complexes) by Cytosine to Thymine Base editing in primary human cells. **A)** Quantification of C to T conversion of target base within the target guide sequence for the gene KO of B2M. **B)** Quantification of C to T conversion of target base within the target guide sequence for the gene KO of CD52. **C)** Quantification of C to T conversion of target base within the target guide sequence for the gene KO of TRAC. **D)** Quantification of C to T conversion of target base within the target guide sequence for the gene KO of PDCD-1. Synthetic RNA-ligand binding complexes and Deaminase-based editor mRNAs (effector proteins) were co-delivered, via electroporation, into CD3 positive T-cells and base conversion was determined 5-7 days post-delivery by analysis of Sanger sequencing traces. The entire protospacer is reported in the drawing to display the different profiles of each of the deaminase enzymes. The control included contains all three deaminases (AnoApo, RatApo, and HoAID) but includes a non-targeting/scramble guide. AnoApo = NLS-Anolis(P16A-E17A)-Apobec1-Linker-MCP. RatApo = NLS-Rattus-Apobec1-Linker-MCP, HoAID = NLS-Homo-AID-Linker-MCP. Depicting the percentage of cytosine to thymine nucleobase transitions.

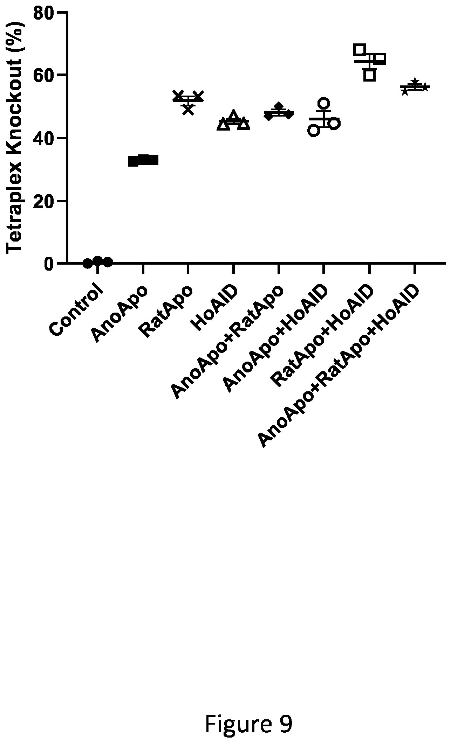

Figure 9 - Single Aptamer (ligand binding moiety) multi-deaminase recruitment base editing at multiple loci simultaneously for novel base editing functionality in a therapeutically relevant immune cell model. Tetraplex functional KO by base editing with synthetic sgRNA-Aptamers (RNA-ligand binding complexes) by Cytosine to Thymine Base editing in primary human cells. Flow cytometry quantification of the percentage of live cells that do not contain any of the four surface markers: B2M, CD52, TCR (reduction as a result of TRAC editing), and PD1 (reduction as a result of PDCD-1 editing); the results show the successfully edited tetraplex edited cells in the live cell population. The control included contains all three deaminases (AnoApo, RatApo, and HoAID) but includes a non-targeting/scramble guide. AnoApo = NLS-Anolis(P16A-E17A)-Apobec1-Linker-MCP. RatApo = NLS-Rattus-Apobec1-Linker-MCP, HoAID = NLS-Homo-AID-Linker-MCP.

## DETAILED DESCRIPTION OF THE INVENTION

This invention relates to a novel method for genetically modifying a cell by base editing. This invention is based on the use of an RNA mediated base editing system in which two or more effectors as defined by the claims are reversibly recruited to a target genomic locus.

In an aspect, provided herein is a method for genetically modifying a cell by base editing, the method comprising introduction into the cells and/or expression in the cells of i) a sequence-targeting component (or sequence-targeting protein) comprising a sequence-targeting protein; ii) an RNA-ligand binding complex comprising (a) a guide RNA, and (b) a ligand binding moiety; and iii) two or more effector proteins comprising (c) a ligand capable of binding to the ligand binding moiety, and (d) an effector domain. The RNA-ligand binding complex guides the sequence-targeting protein and the effector protein to a target polynucleotide at a target site and the effector domain of the effector protein modifies the sequence. As disclosed herein, the sequence-targeting protein, such as a Cas9 protein or a Cas12 protein, is modified such that its double-strand cleavage activity is eliminated.

## RNA-MEDIATED BASE EDITING SYSTEM

The system of the present invention is related to, at least in part, the previously disclosed RNA-mediated base editing system (see WO2018129129, WO2017011721 and US application 62/901,584).

The RNA-mediated base editing used in the methods provided herein take a different approach to the prior art.

More specifically, in the prior art RNA mediated base editing, the RNA-ligand binding complex of the CRISPR/Cas complex serves as an anchor for the recruitment of a single effector by including an RNA motif into the guide RNA molecule. Thus the RNA motif recruits a single effector, e.g. a base editing enzyme, fused to the RNA binding domain (ligand).

In the RNA-mediated base editing complex of the present invention, the recruitment of the effector fused to the RNA binding domain (ligand) is reversible, i.e. the effector has the ability to reversibly associate with its cognate RNA motif (ligand binding moiety). The reversible nature of this system allows the recruitment of more than one effector to a target site of interest where the CRISPR/Cas system has been guided to. Therefore, one can use the same RNA-ligand binding complex and its associated Cas protein with a plurality of different effectors to edit within the same genomic locus.

This system can be used to increase efficiency of base editing, whereby multiple effector proteins with the same RNA binding domain and different effector domains performing the same function are recruited to a single genomic locus. By reversibly associating with the cognate RNA motif (residing in the RNA-ligand binding complex) and its associated sequence-targeting protein, multiple effector proteins are able to be transiently incorporated into the base editing complex until an effector protein with optimal activity associates and makes the appropriate edit. Thus, two, three, four, five or a plurality of effectors with the same function may be employed in the method or kits disclosed herein.

Alternatively, multiple effector proteins with the same RNA binding domain and different effector domains with different functions may be guided to a single genomic locus. By reversibly associating with the cognate RNA motif (residing in the RNA-ligand binding complex) and its associated sequence-targeting protein, multiple effector proteins are able to be transiently incorporated into the base editing complex and perform the appropriate edit. Thus, two, three, four, five or a plurality of effectors with different functions may be employed in the method or kits disclosed herein.

By employing multiple RNA-ligand binding complexes with different guide RNA sequences, the methods and kits disclosed herein can also be used to perform edits at multiple different genomic loci. A single edit, or multiple edits can be performed at each genomic locus by employing multiple effector proteins as described above.

As demonstrated herein, the RNA-ligand binding complex mediated base editing methods and system, and particularly the RNA scaffold mediated base editing methods and system provided herein exhibit a number of important different features compared to the previous system (first generation) described in WO2018129129 and WO2017011721.

First, the present methods and system exhibit substantially increased on-target efficacy compared to the first generation system, but still maintains low or absent detectable off-target effect. Second, the present methods and system may utilise a wide variety of cytidine deaminases from different species and different deaminase families. Many of them show clear different activity windows and preference positions from any previously described first generation constructs. Third, the ability to provide a plurality of different effector proteins (e.g. deaminases) to all sites where edits are to take place leads to the benefit of increased editing efficiency without wasting time to determine the optimal deaminase for each genomic loci. Fourth, being able to provide both cytidine and adenosine deaminases at the same time, to the same site, allows both C-T and A-G edits to be performed simultaneously within the same genomic locus.

The various components that make up the RNA-mediated base editing system of the present invention will now be discussed in detail.

### i) Sequence-Targeting Component

The sequence-targeting component (also referred to as a sequence-targeting protein) of the methods and systems provided herein typically utilise a Cas protein of CRISPR-Cas systems from bacterial species as the sequence targeting protein. In some embodiments the Cas protein is from a Type II CRISPR-Cas system. In alternative embodiments the Cas protein is from a Type V CRISPR-Cas system.

In general, a Cas protein includes at least one Cas RNA binding domain. The Cas RNA binding domain interacts with the guide RNA at the Cas association region. The Cas protein can be a wild type Cas protein or a modified version with no nuclease activity (dCas protein) or just single-strand nicking activity (nCas protein). The Cas protein can be modified to increase nucleic acid binding affinity and/or specificity, alter an enzymatic activity, and/or change another property of the protein. For example, nuclease (i.e., DNase, RNase) domains of the protein can be modified, deleted, or inactivated. Alternatively, the protein can be truncated to remove domains that are not essential for the function of the protein. The protein can also be truncated or modified to optimise the activity.

### Cas Proteins

Various Cas proteins may be used in this invention. A Cas protein, CRISPR-associated protein, or CRISPR protein, used interchangeably, refers to a protein of or derived from a CRISPR-Cas type I, type II, type III or type V system, which has an RNA-guided DNA-binding activity. In general, a Cas protein includes at least one RNA binding domain, herein referred to as a Cas RNA binding domain. The Cas RNA binding domain interacts with the guide RNA at the Cas association region. Non-limiting examples of suitable CRISPR-Cas proteins include Cas3, Cas4, Cas5, Cas5e (or CasD), Cas6, Cas6e, Cas6f, Cas7, Cas8a1, Cas8a2, Cas8b, Cas8c, Cas9, Cas10, Cas10d, Cas12a (or Cpf1), Cas12b (or C2c1), Cas12c, Cas12d (or CasY), Cas12e (or CasX), Cas12f, Cas12g, Cas12g1, Cas12h, Cas12i, Cas12j, Cas12k, CasF, CasG, CasH, Csy1, Csy2, Csy3, Cse1 (or CasA), Cse2 (or CasB), Cse3 (or CasE), Cse4 (or CasC), Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csz1, Csx15, Csf1, Csf2, Csf3, Csf4, and Cu1966. See e.g. Koonin and Makarova, 2019, origins and evolution of crispr cas systems, Review Philos Trans R Soc Lond B Biol Sci. 2019 May 13;374(1772); for Type-V Yan et al, Science, 363 pg 88-92, 2019; and for Miniature Cas14, Harrington et al, 2018, Science, vol 362 pg 839-842.

A Cas protein (as well as other protein components described in this invention) can be obtained as a recombinant polypeptide. Alternatively, the proteins can be chemically synthesised (see e.g. Creighton, "Proteins: Structures and Molecular Principles," W.H. Freeman & Co., NY, 1983), or produced by recombinant DNA technology as described herein. For additional guidance, skilled artisans may consult Frederick M. Ausubel et al., Current Protocols in Molecular Biology, John Wiley & Sons, 2003; and Sambrook et al., Molecular Cloning, A Laboratory Manual," Cold Spring Harbor Press, Cold Spring Harbor, NY, 2001).

The Cas protein described in the invention can be provided in purified or isolated form, or can be part of a composition. Preferably, where in a composition, the proteins are first purified to some extent, more preferably to a high level of purity (e.g., about 80%, 90%, 95%, or 99% or higher). Compositions according to the invention can be any type of composition desired, but typically are aqueous compositions suitable for use as, or inclusion in, a composition for RNA-guided targeting. Those of skill in the art are well aware of the various substances that can be included in such nuclease reaction compositions.

The Cas protein described in the invention can be directly fused to an effector protein, such that in addition to the two or more effector proteins reversibly recruited to the base-editing complex via the RNA-ligand binding complex (or RNA scaffold), the directly-fused effector domain is also able to carry out its activity at the target site.

Alternatively the Cas protein described in the invention can be associated to an effector domain via a ligand as described in Science 16 Sep 2016: Vol. 353, Issue 6305, such that in addition to the two or more effector proteins reversibly recruited to the base-editing complex via the RNA-ligand binding complex (or RNA scaffold), the ligand associated effector domain is also able to carry out its activity at the target site.

To practice the method disclosed herein for modifying a target nucleic acid, one can produce the proteins in a target cell via mRNA, protein RNA complexes (RNP), or any suitable expression vectors. Examples of expression vectors include chromosomal, non-chromosomal and synthetic DNA sequences, bacterial plasmids, minicircles, phage DNA, baculovirus, yeast plasmids, vectors derived from combinations of plasmids and phage DNA, viral DNA such as vaccinia, adenovirus, fowl pox virus, and pseudorabies. More details are described in the Expression System and Methods sections below.

### Application to a Type II CRISPR-Cas System

In some embodiments, the Cas protein is derived from a Type II CRISPR-Cas system. In exemplary embodiments, the Cas protein is or is derived from a Cas9 protein. The Cas9 protein can be from *Streptococcus pyogenes, Streptococcus thermophilus, Streptococcus sp., Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetoholobium arabaticum, Ammonifex degensii, Coldicelulosiruptor becscii, Candidatus Desulforudis, Clostridium botulinum, Clostridium difficile, Finegoldic magna, Natranaerobius thermophilus, Pelotomaculum the rmopropionicum, Acidithiobacillus caldus, Acidithiobacillus ferrooxidans, Allochromatium vinosum, Marinobacter sp., Nitrosococcus holophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus,* or *Acaryochloris marina.*

In some embodiments, the Cas protein can be a mutant of a wild type Cas9 protein or a fragment thereof. In other embodiments, the Cas protein can be derived from a mutant Cas9 protein. For example, the amino acid sequence of the Cas9 protein can be modified to alter one or more properties *(e.g.,* nuclease activity, affinity, stability, etc.) of the protein. Alternatively, domains of the Cas9 protein not involved in RNA targeting can be eliminated from the protein such that the modified Cas9 protein is smaller than the wild type Cas9. In some embodiments, the present system utilises the Cas9 protein from *S. pyogenes,* either as encoded in bacteria or codon-optimised for expression in mammalian cells.

A mutant Cas9 protein refers to a polypeptide derivative of the wild type protein, *e.g.* a protein having one or more point mutations, insertions, deletions, truncations, a fusion protein, or a combination thereof. The mutant has at least one of the RNA-guided DNA binding activity, or RNA-guided nuclease activity, or both. In general, the modified version is at least 50% *(e.g.,* any number between 50% and 100%, inclusive, *e.g.*, 50%, 60%, 70 %, 75%, 80%, 85%, 90%, 95%, and 99%) identical to the wild type Cas9 protein such as SEQ ID No. 44 below.

As disclosed here, one can use the nuclease dead Cas9 (dCas9, for example from *S. pyogenes* D10A, H840A mutant protein, SEQ ID No. 17), or the nuclease defective nickase Cas9 (nCas9, for example from *S. pyogenes* D10A mutant protein, SEQ ID No. 46). dCas9 or nCas9 could also be derived from various bacterial species. Table 1 lists a non-exhausting list of examples of Cas9, and their corresponding PAM requirements. One can also use synthetic Cas substitutes such as those described in Rauch et al., Programmable RNA-Guided RNA Effector Proteins Built from Human Parts. Cell Volume 178, Issue 1, 27 June 2019, Pages 122-134.e12.

**Table 1. Non-exhaustive list of examples of Cas9 proteins and their corresponding PAM requirements**

| Species | PAM |
|---|---|
| *Streptococcus pyogenes* | NGG |
| *Streptococcus agalactiae* | NGG |
| *Staphylococcus aureus* | NNGRRT |
| *Streptococcus thermophiles* | NNAGAAW |
| *Streptococcus thermophiles* | NGGNG |
| *Neisseria meningitidis* | NNNNGATT |
| *Treponema denticola* | NAAAAC |

### *Application to a Type V CRISPR-Cas System*

In some embodiments, the Cas protein is derived from a Type V CRISPR-Cas system (see for example Wang, J., Zhang, C., and Feng, B. (2020). The rapidly advancing Class 2 CRISPR-Cas technologies: a customizable toolbox for molecular manipulations. J. Cell. Mol. Med. 24, 3256-3270. doi: 10.1111/jcmm.15039; and Makarova, K.S., Wolf, Y.I., Iranzo, J. et al. Evolutionary classification of CRISPR-Cas systems: a burst of class 2 and derived variants. Nat Rev Microbiol 18, 67-83 (2020); and Tong, B. et al. (2021) The versatile type V CRISPR effectors and their application prospects. Front. Cell Dev. Biol. 8, 1835. doi: 10.3389/fcell.2020.622103). The Cas12 protein can be from *Francisella cf. novicida Fx1, Deltaproteobacteria bacterium, Alicyclobacillus acidoterrestri, Planctomycetes bacterium, Oleiphilus sp., Bacterium CG09_39_24, Uncultured archaeon, Bacillus thuringiensis, Cyanothece sp., Rothic dentocariosa, Candidatus Micrarchaeota archaeon, Gordonia otitidis, Freshwater metagenome, Hypersaline lake sediment metagenome or Hot springs metagenome.* The Cas protein can be Cas12a (or Cpf1), Cas12b (or C2c1), Cas12c, Cas12d (or CasY), Cas12e (or CasX), Cas12f, Cas12g, Cas12g1, Cas12h, Cas12i, Cas12j, or Cas12k.

In some embodiments, the Cas protein can be a mutant of a wild type Cas12 protein (such as Cas12a from *Acidaminococcus* sp.) or a fragment thereof. In other embodiments, the Cas12 protein can be derived from a mutant Cas12 protein. For example, the amino acid sequence of the Cas12 protein can be modified to alter one or more properties (e.g., nuclease activity, affinity, stability, etc.) of the protein. Alternatively, domains of the Cas12 protein not involved in RNA targeting can be eliminated from the protein such that the modified Cas12 protein is smaller than the wild type Cas12 protein. In some embodiments, the present system utilises the Cas12a protein from Lachnospiraceae bacterium ND2006, Cas12d from Candidate Phyla Radiation group of bacteria or Cas12b from *A. acidoterrestris,* either as encoded in bacteria or codon-optimized for expression in mammalian cells.

A mutant Cas12 protein refers to a polypeptide derivative of the wild type protein, e.g. a protein having one or more point mutations, insertions, deletions, truncations, a fusion protein, or a combination thereof. The mutant has at least one of the RNA-guided DNA binding activity, or RNA-guided nuclease activity, or both. In general, the modified version is at least 50% (e.g., any number between 50% and 100%, inclusive, e.g., 50%, 60%, 70%, 75%, 80%, 85%, 90%, 95%, and 99%) identical to the wild type Cas12 protein such as AsCas12a (SEQ ID No. 48), AaCas12b (SEQ ID No. 49) or Cas12d15 (SEQ ID No. 50) below.

As disclosed here, one can use the nuclease dead AsCas12a (dAsCas12a, for example from *Acidaminococcus* sp. D908A mutant protein), dCas12 or nCas12 could also be derived from various bacterial species. Table 2 lists a non-exhausting list of examples of Cas12 proteins, and their corresponding PAM requirements.

The humanised nucleotide sequence of dCas12a (SEQ ID No. 54), dCas12b (SEQ ID No. 55) and dCas12d (SEQ ID No. 56) and amino acid sequence of dAsCas12a (SEQ ID No. 57), dAaCas12b (SEQ ID No. 58) and dCas12d15 (SEQ ID No. 59) is provided.

Table 2

| Biological Origin | Cas | PAM |
|---|---|---|
| *Francisella novicida* | FnCas12a (Cpf1) | 5' (T)TTV-(PS) |
| *Lachnospiraceae* bacterium | LbCas12a (Cpf1) | 5'TTTV-(PS) |
| *Acidaminococcus* sp. | AsCas12a (Cpf1) | 5' TTTN-(PS) |
| *Alicyclobacillus acidoterrestris* | AacCas12b (C2c1) | 5' TTN-(PS) |
| *Bacillus hisashii* | BhCas12b v4 | 5' ATTN-(PS) |
| *Oleiphilus sp.* | OspCas12c (C2c3) | 5' TG-(PS) |
| - | Cas12c1 (C2c3)a | 5' TG-(PS) |
| - | Cas12c2 (C2c3) | 5' TN-(PS) |
| - | Cas12d (CasY) | 5' TA-(PS) |
| - | Cas12e (CasX) | 5' TTCN-(PS) |
| - | Cas14a1 | 5' TTTR-(PS) |
| - | Cas12g1 | No PAM requirement |
| - | Cas12h1 | 5' RTR-(PS) |
| - | Cas12i1 | 5' TTN-(PS) |

PS = protospacer; N = any base; R = A/G; V = A/C/G.

### UGI

In some aspects of this disclosure, the above-described sequence-targeting component comprises a fusion between (a) a sequence-targeting protein, and (b) a first uracil DNA glycosylase (UNG) inhibitor peptide (UGI). For example, the fusion protein can include a Cas protein, e.g. Type II or Type V Cas proteins, fused to a UGI. Such fusion proteins may exhibit an increased nucleic acid editing efficiency as compared to fusion proteins not comprising an UGI domain. In some embodiments, the UGI comprises a wild type UGI sequence from the Bacillus phage PBS2 or one having the following amino acid sequence (SEQ ID NO. 60).

In some embodiments, the UGI proteins provided herein include fragments or variants of UGI and proteins homologous to a UGI or a UGI fragment. For example, in some embodiments, a UGI comprises an amino acid sequence homologous to a fragment of SEQ ID No. 60. In some embodiments, a UGI comprises an amino acid sequence homologous to (SEQ ID No. 60) or an amino acid sequence homologous to a fragment of (SEQ ID No. 60). In some embodiments, proteins comprising UGI or fragments of UGI or homologs of UGI or UGI fragments are referred to as "UGI variants." A UGI variant shares homology to UGI, or a fragment thereof. For example, a UGI variant is at least about 70% (e.g., at least about 80%, 90%, 95%, 96%, 97%, 98%, 99%) to a wild type UGI or the UGI sequence (SEQ ID No. 60).

In some embodiments, the active or deactivated Cas protein comprises a fusion with two or more UGI peptides or variants. The UGI peptides or variants of the UGI peptide can be connected directly to another UGI peptide or Cas protein or via a linker of 1 to 100 amino acid residues to another UGI peptide or Cas protein.

Suitable UGI protein and nucleotide sequences are provided herein and additional suitable UGI sequences are known to those in the art, and include, for example, those published in Wang et al., Uracil-DNA glycosylase inhibitor gene of bacteriophage PBS2 encodes a binding protein specific for uracil-DNA glycosylase. J Biol. Chem. 264:1163-1171(1989); Lundquist et al., Site-directed mutagenesis and characterization of uracil-DNA glycosylase inhibitor protein. Role of specific carboxylic amino acids in complex formation with Escherichia coli uracil-DNA glycosylase. J Biol. Chem. 272:21408-21419(1997); Ravishankar et al., X-ray analysis of a complex of Escherichia coli uracil DNA glycosylase (EcUDG) with a proteinaceous inhibitor. The structure elucidation of a prokaryotic UDG. Nucleic Acids Res. 26:4880-4887(1998); and Putnam et al., Protein mimicry of DNA from crystal structures of the uracil-DNA glycosylase inhibitor protein and its complex with Escherichia coli uracil-DNA glycosylase. J Mol. Biol. 287:331-346(1999).

### ii) RNA-Ligand Binding Complex

The second component of the platform disclosed herein is an RNA-ligand binding complex, which plays an essential role for sequence recognition and effector recruitment. The RNA-ligand binding complex comprises two sub-components: (a) a guide RNA and (b) a ligand binding moiety. In a preferred embodiment, the ligand binding moiety is an RNA motif.

The nucleotides within the RNA strand(s) may be entirely ribonucleotides or a combination of ribonucleotides and other nucleotides such as deoxyribonucleotides. Each nucleotide may be unmodified, or one or more nucleotides, if not all nucleotides may be modified, e.g. with one or more of the following modifications: 2'-O-methyl, 2' fluoro or 2' aminopurine. In some embodiments over one or more ranges of one to forty or two to twenty or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 35, or 36 nucleotides, there are consecutively modified nucleotides or a modification pattern of every second, or every third or every fourth nucleotide being modified at its 2' position with all other nucleotides being unmodified. Additionally, or alternatively, between one or more pairs or every pair of consecutive nucleotides, there may be modified or unmodified internucleotide linkages.

## (a) Programmable Guide RNA

Due to its simplicity and efficiency, the CRISPR-Cas system has been used to perform genome-editing in cells of various organisms. The specificity of this system is dictated by base pairing between a target DNA and a custom-designed guide RNA. By engineering and adjusting the base-pairing properties of guide RNAs, one can target any sequences of interest. For some CRISPR-Cas systems, the target sequence of interest must include a PAM sequence to be successfully targeted by the base-editing complex, as detailed below.

The guide RNA may comprise any one or more of the following: a crRNA, a tracrRNA and/or a scoutRNA.

For embodiments wherein a Type II Cas protein is utilised, the guide RNA may comprise crRNA and tracrRNA. For embodiments wherein a Type V Cas protein is utilised, the guide RNA may comprise crRNA, crRNA and tracrRNA, or crRNA and scoutRNA.

## Type II CRISPR-Cas System: crRNA:tracrRNA Guide

Type II CRISPR-Cas systems utilise a guide RNA comprising a crRNA and a tracrRNA (Type II crRNA:tracrRNA guide). The crRNA comprises a sequence that is complementary to a target nucleic acid. The guide RNA may further comprise a tracrRNA that at minimum can hybridise with the crRNA over a range of at least three nucleotides, and when hybridised over that region can retain association with a Type II Cas protein. The guide RNA can be either a single RNA molecule or a complex of multiple RNA molecules.

Thus, in embodiments wherein the sequence targeting component comprises a Type II Cas protein, it is preferable that the RNA-ligand binding complex comprises a Type II crRNA:tracrRNA guide.

Among the sub-components of the RNA-ligand binding complex (or RNA scaffold) disclosed herein, the crRNA provides the targeting specificity. The crRNA comprises a programmable spacer that is complementary and capable of hybridisation to a pre-selected target site of interest. In various embodiments, this spacer can comprise from about 10 nucleotides to more than about 25 nucleotides. For example, the region of base pairing between the spacer and the corresponding target site sequence can be about 10, 11, 12, 13, 14, 15, 16, 17, 18, 19, 20, 22, 23, 24, 25, or more than 25 nucleotides in length. In an exemplary embodiment, the spacer is about 17-20 nucleotides in length, such as 20 nucleotides. The spacer is variable and may be selected based on the target sequence of interest for the Cas protein and/or effector to cause base editing. The spacer does not hybridise with tracrRNA, and it may be downstream of the Cas association region.

One requirement for selecting a suitable target nucleic acid for Type II CRISPR-Cas systems is that it has a PAM site/sequence. Each target sequence and its corresponding PAM site/sequence are referred herein as a Cas-targeted site. The Type II CRISPR-Cas system, one of the most well characterised systems, needs only a Cas9 protein and a guide RNA complementary to a target sequence to affect target cleavage. The Type II CRISPR-Cas system of S. pyogenes uses target sites having N12-20NGG, where NGG represents the PAM site from *S. pyogenes,* and N12-20 represents the 12-20 nucleotides directly 5' to the PAM site. Additional PAM site sequences from other species of bacteria include NGGNG, NNNNGATT, NNAGAA, NNAGAAW, and NAAAAC. See, e.g., US 20140273233, WO 2013176772, Cong et al., (2012), Science 339 (6121): 819-823, Jinek et al., (2012), Science 337 (6096): 816-821, Mali et al, (2013), Science 339 (6121): 823-826, Gasiunas et al., (2012), Proc Natl Acad Sci USA. 109 (39): E2579-E2586, Cho et al., (2013) Nature Biotechnology 31, 230-232, Hou et al., Proc Natl Acad Sci USA. 2013 Sep 24;110(39):15644-9, Mojica et al., Microbiology. 2009 Mar;155(Pt 3):733-40, and www.addgene.org/CRISPR/. A PAM site/sequence is also a requirement for selecting a suitable target nucleic acid for a Type V CRISPR system.

The target nucleic acid strand can be either of the two strands on a genomic DNA in a host cell. Examples of such genomic dsDNA include, but are not necessarily limited to, a host cell chromosome, mitochondrial DNA and a stably maintained plasmid. However, it is to be understood that the present method can be practiced on other dsDNA present in a host cell, such as non-stable plasmid DNA, viral DNA, and phagemid DNA, as long as there is Cas-targeted site regardless of the nature of the host cell dsDNA. The present method can be practiced on RNAs too.

In addition to the spacer, the crRNA also comprises a repeat region. The repeat region hybridises with the anti-repeat region of the tracrRNA, described below, to form a repeat:antirepeat duplex. In various embodiments, the repeat region can comprise from about 10 nucleotides to more than about 25 nucleotides. For example, the repeat region can be about 10, 11, 12, 13, 14, 15, 16, 17, 18, 19, 20, 22, 23, 24, 25, or more than 25 nucleotides in length. In an exemplary embodiment, the repeat region is about 21-25 nucleotides in length, such as 23 nucleotides.

The repeat:antirepeat duplex acts as a Cas association region, and is designed on the Cas RNA binding domain of a Cas protein with which it is intended to associate. Not all of the nucleotides within the Cas association region need directly associate with the Cas protein. Not all of the nucleotides within the repeat:antirepeat duplex hybridise. The repeat:antirepeat duplex comprises a lower stem, bulge and upper stem. The bulge is essential for interaction with the Cas protein. In a non-limiting example, the repeat:antirepeat duplex of a guide RNA capable of interacting with Cas9 from *S. pyogenes* may comprise a lower stem of about 6 nucleotide in length, a bulge of a about 6 nucleotides in length and a upper stem of 1, 2, 3, 4, 5, 6, 7, 8, 9, 10, or more than 10 nucleotides in length. Optionally, the upper stem may be absent from the repeat:antirepeat duplex.

In addition to a crRNA, the guide RNA of Type II CRISPR-Cas systems further comprises a trans-activating CRISPR RNA (tracrRNA). The tracrRNA sequence may comprise from about 40 nucleotides to more than about 100 nucleotides. For example, the tracrRNA can be about 40, 50, 60, 70 80, 90 or more than 100 nucleotides in length. In an exemplary embodiment, the tracrRNA is about 88-90 nucleotides in length, such as 89 nucleotides. The tracrRNA sequence comprises an anti-repeat region, a nexus, stem loop 2 and stem loop 3. The tracrRNA further comprises a distal region that does not hybridise with the crRNA, and it may be upstream of the anti-repeat region. The anti-repeat region is at least 80%, at least 85%, at least 90%, at least 95%, or 100% complementary to the crRNA repeat region over at least 7 consecutive nucleotides. Thus, the repeat region of the above-described programmable crRNA and the anti-repeat region of the tracrRNA are capable of hybridising to form a hybridisation region, herein referred to as a repeat:anti-repeat duplex.

In some embodiments the tracrRNA is from *Streptococcus pyogenes.*

In some embodiments, the tracrRNA activity and crRNA activity are part of a single continuous strand of nucleotides, known as single guide RNA (or sgRNA). In some embodiments, the crRNA may be immediately upstream of the tracrRNA or it may be upstream of the tracrRNA with an intervening sequence or moiety between the tracrRNA and crRNA. If the tracrRNA and crRNA are part of a contiguous strand of nucleotides (sgRNA), there may be a loop region between the tracrRNA and the crRNA of for example 3 to 6 nucleotides, herein referred to as a tetraloop. See e.g., WO2014099750, US 20140179006, and US 20140273226. Methods for generating crRNA:tracrRNA guide RNAs and sgRNAs are well known in the art.

In some embodiments, the tracrRNA activity and the crRNA comprising the guide RNA are in two separate RNA molecules, which together form the functional guide RNA and part of the RNA-ligand binding complex (or RNA scaffold). In this case, the molecule with the tracrRNA activity should be able to interact with (usually by base pairing) the molecule with the crRNA activity to form a two-part guide crRNA:tracrRNA.

Shown below is an exemplary hybrid Type II crRNA:tracrRNA guide RNA sequence (SEQ ID No. 61; Chen et al. Cell. 2013 Dec 19;155(7):1479-91):

5'-(20nt guide)-

GUUUAAGAGCUAUGCUGGAAACAGCAUAGCAAGUUUAAAUAAGGCUAGUCCGUUAUCAACUUGAAAAA

GUGGCACCGAGUCGGUGCUUUUUUU-3'

Various tracrRNA and crRNA sequences are known in the art, and non-limiting examples of crRNAs and tracrRNAs that may be used in connection to the present invention are provided below (SEQ ID No. 1, SEQ ID No. 2). As used herein, an active portion of a tracrRNA retains the ability to form a complex with a Cas protein, such as Cas9 or dCas9 or nCas9. See, e.g., WO2014144592.

Type II crRNA (Ns denote target-specific spacer) (SEQ ID No. 1)
5' NNNNNNNNNNNNNNNNNNNNGUUUUAGAGCUAUGCUGUUUUG 3'-crRNA (Ns denote target specific spacer).

Type II tracrRNA (SEQ ID No. 2)

5'AACAGCAUAGCAAGUUAAAAUAAGGCUAGUCCGUUAUCAACUUGAAAAAGUGGCACCGAGUC

GGUGCGCGCACAUGAGGAUCACCCAUGUGCUUUUUUU 3'

Thus, in embodiments wherein the sequence targeting component of the present invention comprises a Type II Cas protein, it is preferable that the RNA-ligand binding complex comprises a Type II crRNA:tracrRNA guide RNA.

**Application to a Type V CRISPR-Cas System**

Type V CRISPR-Cas systems may utilise a guide RNA comprising a crRNA (Type V crRNA-only guide), a crRNA and tracrRNA (Type V crRNA:tracrRNA guide), or a crRNA and scoutRNA (Type V crRNA:scoutRNA guide). The guide RNA can be either a single RNA molecule or a complex of multiple RNA molecules.

Thus, in embodiments where the sequence targeting component of the present invention comprises a Type V Cas protein, it is preferable that the RNA-ligand binding complex comprises a crRNA guide RNA, a crRNA:tracrRNA guide RNA or a crRNA:scoutRNA guide RNA.

**Type V CRISPR-Cas System: crRNA-only Guide**

Type V CRISPR-Cas systems wherein the sequence-targeting component is a Cas protein such as Cas12a from *Acidaminococcus sp.* BV3L6 (AsCas12a), require a guide RNA comprising of only a crRNA molecule (Type V crRNA-only guide).

The crRNA provides targeting specificity, and comprises a spacer that has a nucleotide sequence that is complementary and capable of hybridisation to a pre-selected target site of interest. In various embodiments, the spacer can comprise from about 10 nucleotides to more than about 25 nucleotides. For example, the region of base pairing between the spacer and the corresponding target site sequence can be about 10, 11, 12, 13, 14, 15, 16, 17, 18, 19, 20, 22, 23, 24, 25, or more than 25 nucleotides in length. In an exemplary embodiment, the targeting sequence is about 18-25 nucleotides in length, such as 24 nucleotides. The spacer is variable and may be selected based on where one wishes for the Cas protein and/or effector to cause base editing.

One requirement for selecting a suitable target nucleic acid for some Type V CRISPR-Cas systems (as detailed in Table 2) is that it has a PAM site/sequence. Some Type V CRISPR-Cas systems, for example Cas12g1, do not require a PAM site/sequence for target recognition. Each target sequence and (if required) its corresponding PAM site/sequence are referred herein as a Cas-targeted site.

The target nucleic acid strand can be either of the two strands on a genomic DNA in a host cell. Examples of such genomic dsDNA include, but are not necessarily limited to, a host cell chromosome, mitochondrial DNA and a stably maintained plasmid. However, it is to be understood that the present method can be practiced on other dsDNA present in a host cell, such as non-stable plasmid DNA, viral DNA, and phagemid DNA, as long as there is Cas-targeted site regardless of the nature of the host cell dsDNA. The present method can be practiced on RNAs too.

In addition to a spacer, the crRNA in Type V crRNA-only guide comprises a direct repeat that forms a pseudoknot-type hairpin secondary structure. The secondary structure forms critical contacts with the Cas enzyme.

Shown below is a non-limiting example of Type V crRNA-only guides.

**AsCas12a crRNA targets *PPIB* (target-specific spacer underlined) (SEQ ID No. 3)**

5' UAAUUUCUACUCUUGUAGAU<u>ACCTACGAATTGGAGATGAAGATG</u> 3'

Thus, in embodiments wherein the sequence targeting component comprises a Type V Cas protein such as AsCas12a, it is preferable that the RNA-ligand binding complex comprises Type V crRNA-only guide RNA.

**Type V CRISPR-Cas System: crRNA:tracrRNA Guide**

Type V CRISPR-Cas systems wherein the sequence-targeting component is a Cas protein such as Cas12b from *Alicylobacillus acidiphilus* (AaCas12b), require a guide RNA comprising a crRNA and a tracrRNA (Type V crRNA:tracrRNA guide).

The crRNA provides targeting specificity, and comprises a programmable spacer that has a nucleotide sequence that is complementary and capable of hybridisation to a pre-selected target site of interest. In various embodiments, this spacer can comprise from about 10 nucleotides to more than about 25 nucleotides. For example, the region of base pairing between the spacer and the corresponding target site sequence can be about 10, 11, 12, 13, 14, 15, 16, 17, 18, 19, 20, 22, 23, 24, 25, or more than 25 nucleotides in length. In an exemplary embodiment, the spacer is about 19-22 nucleotides in length, such as 20 nucleotides. The spacer is variable and may be selected based on the target sequence of interest for the Cas protein and/or effector to cause base editing. The spacer does not hybridise with tracrRNA.

One requirement for selecting a suitable target nucleic acid for some Type V CRISPR-Cas systems (as detailed in Table 2) is that it has a PAM site/sequence. Some Type V CRISPR-Cas systems, for example Cas12g1, do not require a PAM site/sequence for target recognition. Each target sequence and (if required) its corresponding PAM site/sequence are referred herein as a Cas-targeted site.

The target nucleic acid strand can be either of the two strands on a genomic DNA in a host cell. Examples of such genomic dsDNA include, but are not necessarily limited to, a host cell chromosome, mitochondrial DNA and a stably maintained plasmid. However, it is to be understood that the present method can be practiced on other dsDNA present in a host cell, such as non-stable plasmid DNA, viral DNA, and phagemid DNA, as long as there is Cas-targeted site regardless of the nature of the host cell dsDNA. The present method can be practiced on RNAs too.

In addition to the spacer, the crRNA also comprises a repeat region. The repeat region hybridises with the anti-repeat region of the tracrRNA, described below, to form a repeat:antirepeat duplex. In various embodiments, the repeat region can comprise from about 20 nucleotides to more than about 35 nucleotides. In an exemplary embodiment, the repeat region is about 28-33 nucleotides in length, such as 31 nucleotides.

Besides the above-described crRNA, the Type V crRNA:tracrRNA guide comprises a trans-activating CRISPR RNA (tracrRNA). The tracrRNA sequence may comprise from about 40 nucleotides to more than about 100 nucleotides. For example, the tracrRNA can be about 40, 50, 60, 70 80, 90 or more than 100 nucleotides in length. In an exemplary embodiment, the tracrRNA is about 98-100 nucleotides in length, such as 100 nucleotides. The tracrRNA sequence comprises an anti-repeat region and stem loops. Various tracrRNA sequences are known in the art. As used herein, an active portion of a tracrRNA retains the ability to form a complex with a Type V Cas protein, such as Cas12b or dCas12b or nCas12b.

In some embodiments the tracrRNA is from *A. acidoterrestris.*

In some embodiments, the tracrRNA activity and crRNA activity are part of a single continuous strand of nucleotides, known as single guide RNA (or sgRNA). In some embodiments, the crRNA may be immediately downstream of the tracrRNA or it may be downstream of the tracrRNA with an intervening sequence or moiety between the tracrRNA and crRNA. If the tracrRNA and crRNA are part of a contiguous strand of nucleotides (sgRNA), there may be a loop region between the tracrRNA and the crRNA of for example 3 to 6 nucleotides, herein referred to as a tetraloop. Methods for generating Type V crRNA:tracrRNA guide RNAs and sgRNAs are known in the art. A non-limiting exemplary Type V crRNA:tracrRNA sgRNA is shown below (SEQ ID No. 4).

**AaCas12b sgRNA - Targets *PPIB* (target-specific spacer underlined) (SEQ ID No. 4)**

5´GGTCTAAAGGACAGAATTTTTCAACGGGTGTGCCAATGGCCACTTTCCAGGTGGCAAAGCCCGTT

GAACTTCTCAAAAAGAACGCTCGCTCAGTGTTCTGACGTCGGATCACTGAGCGAGCGATCTGAGAAGTGGCA

CACCTACGAATTGGAGATGAA 3´

In some embodiments, the tracrRNA activity and the crRNA comprising the guide RNA are two separate RNA molecules, which together form the functional guide RNA and part of the RNA-ligand binding complex (or RNA scaffold). In this case, the molecule with the tracrRNA activity should be able to interact with (usually by base pairing) the molecule with the crRNA activity, to form a two-part Type V crRNA:tracrRNA guide.

Thus, in embodiments wherein the sequence targeting component comprises a Type V Cas protein such as Cas12b from *Alicylobacillus acidiphilus* (AaCas12b), it is preferable that the RNA-ligand binding complex comprises a Type V crRNA:tracrRNA guide.

**Type V CRISPR-Cas System: crRNA:scoutRNA Guide**

Type V CRISPR-Cas systems wherein the sequence-targeting component is a Cas protein such as Cas12d15, require a guide RNA comprising a crRNA and a scoutRNA (crRNA:scoutRNA guide RNA).

The crRNA provides targeting specificity, and comprises a programmable spacer that has a nucleotide sequence that is complementary and capable of hybridisation to a pre-selected target site of interest. In various embodiments, this spacer can comprise from about 10 nucleotides to more than about 25 nucleotides. For example, the region of base pairing between the spacer and the corresponding target site sequence can be about 10, 11, 12, 13, 14, 15, 16, 17, 18, 19, 20, 22, 23, 24, 25, or more than 25 nucleotides in length. In an exemplary embodiment, the spacer is about 19-22 nucleotides in length, such as 20 nucleotides. The spacer is variable and may be selected based on the target sequence of interest for the Cas protein and/or effector to cause base editing. The spacer does not hybridise with scoutRNA

One requirement for selecting a suitable target nucleic acid for some Type V CRISPR-Cas systems (as detailed in Table 2) is that it has a PAM site/sequence. Some Type V CRISPR-Cas systems, for example Cas12g1, do not require a PAM site/sequence for target recognition. Each target sequence and (if required) its corresponding PAM site/sequence are referred herein as a Cas-targeted site.

The target nucleic acid strand can be either of the two strands on a genomic DNA in a host cell. Examples of such genomic dsDNA include, but are not necessarily limited to, a host cell chromosome, mitochondrial DNA and a stably maintained plasmid. However, it is to be understood that the present method can be practiced on other dsDNA present in a host cell, such as non-stable plasmid DNA, viral DNA, and phagemid DNA, as long as there is Cas-targeted site regardless of the nature of the host cell dsDNA. The present method can be practiced on RNAs too.

In addition to the spacer, the crRNA also comprises a 5' direct-repeat region. This region comprises a conserved 5'nt sequence that hybridises to a complementary sequence of the scoutRNA, described below. In various embodiments, the repeat region can comprise from about 20 nucleotides to more than about 35 nucleotides. In an exemplary embodiment, the repeat region is about 28-33 nucleotides in length, such as 31 nucleotides.

Besides the above-described crRNA, the Type V crRNA:scoutRNA guide of this invention comprises a short-complementary untranslated RNA (scoutRNA). ScoutRNA together with crRNA, is required for the activity of certain Type V CRISPR systems, for example Cas12d-catalysed activity. The scoutRNA differs in secondary structure from previously described tracrRNAs used by CRISPR-Cas9 and some Cas12 enzymes, and in Cas12d-containing systems, scoutRNA includes a conserved five-nucleotide sequence that is essential for hybridisation to the crRNA and subsequent enzymatic activity. In addition to supporting crRNA-directed DNA recognition, biochemical and cell-based experiments establish scoutRNA as an essential cofactor for Cas12c-catalyzed pre-crRNA maturation.

The scoutRNA may be 40 to 100 nucleotides long. The scoutRNA sequence comprises a crRNA complementary region, an upstream region that is upstream of the crRNA complementary region, and a downstream region that is downstream of the crRNA complementary region. Preferably, the crRNA complementary region is 5 nucleotides long. The crRNA complementary region may be located at or near the 5' end of the scoutRNA or at or near the 3' end of the scoutRNA or between consecutive nucleotides within the scoutRNA that are neither at or nor the 5' or 3' end of the scoutRNA.

In some native scoutRNAs, self-complementary regions allow for one or more self-hybridisation regions, loops, and bulges, as well as optionally 5' ssRNA overhangs and or no overhangs. In some embodiments, any bulge or bulges of naturally occurring scoutRNAs are preserved even when the ligand binding moiety is attached.

In some embodiments, the anti-repeat region is at least 80%, at least 85%, at least 90%, at least 95%, or 100% complementary to the Cas association region over at least 5 consecutive nucleotides. Thus, the Cas association region of the above-described programmable crRNA and the anti-repeat region of the scoutRNA are capable of hybridising to form a hybridisation region. If the scoutRNA self-hybridises to form one or more hairpin regions, in some embodiments, its anti-repeat region may form a bulge.

When the anti-repeat region of the scoutRNA and the Cas association region of the crRNA hybridise to form the hybridisation region, the RNA that contains both the scoutRNA and the crRNA is capable of retaining association with a Cas RNA binding domain of a Type V Cas protein.

In some embodiments, the scoutRNA activity and crRNA activity are part of a single continuous strand of nucleotides. In some embodiments, the crRNA may be immediately downstream of the scoutRNA or it may be downstream of the scoutRNA with an intervening sequence or moiety between the scoutRNA and crRNA. The intervening sequence or moiety may be the ligand binding moiety, or a nucleotide or non-nucleotide loop region or ethylene glycol spacers such as 18S, 9S or C3. In a 5' to 3' direction, the crRNA:scoutRNA guide RNA may comprise, consist essentially of or consist of a first region of the scoutRNA, the anti-repeat region of the scout RNA, a second region of the scoutRNA, the loop between the scoutRNA, a Cas association region and the targeting region.

In some embodiments, the scoutRNA activity and the crRNA comprising the guide RNA are two separate RNA molecules, which together form the functional guide RNA and part of the RNA-ligand binding complex (or RNA scaffold). In this case, the molecule with the scoutRNA activity should be able to interact with (usually by base pairing) the molecule (crRNA) having the targeting sequence to form a two part guide crRNA:scoutRNA.

Non-limiting examples of scoutRNAs and crRNAs that may be used in a Type V CRISPR-Cas system connection to the present invention appear below.

**Cas12d15 scoutRNA (SEQ ID No. 5)**

       5'CUUAGUUAAGGAUGUUCCAGGUUCUUUCGGGAGCCUUGGCCUUCUCCCUUAACCUAUGCCA

CUAAUGAUU 3'

**Cas12d15 crRNA - Targets *PPIB* (targeting sequence underlined) (SEQ ID No. 6)**
5' ACCCGUAAAGCAGAGCGAUGAAGGC<u>ACCTACGAATTGGAGATGAA</u> 3'

Thus, in embodiments wherein the sequence targeting component comprises a Type V Cas protein such as Cas12d15 (Fu, B.X.H., Smith, J.D., Fuchs, R.T. et al. Target-dependent nickase activities of the CRISPR-Cas nucleases Cpf1 and Cas9. Nat Microbiol 4, 888-897 (2019). https://doi.org/10.1038/s41564-019-0382-0), it is preferable that the RNA-ligand binding complex comprises a Type V crRNA:scoutRNA guide.

**(b) Ligand Binding Moiety**

In any embodiment, the RNA-ligand binding complex further comprises a ligand binding moiety capable of associating with a cognate ligand which, in effect, recruits the two or more effector proteins to the target DNA. As disclosed herein, the guide RNA and the Cas protein together form a CRISPR/Cas-based module for sequence targeting and recognition, while the ligand binding moiety recruits one or more effector proteins, such as a base editing enzyme, which carries out one or more genetic modifications. This linkage is critical for the base editing systems and methods disclosed herein.

In some embodiments, the ligand binding moiety is selected from the group consisting of moieties that associate with the following ligands: MS2 coat protein (MCP), Ku, PP7 coat protein (PCP), Com RNA binding protein or the binding domain thereof, SfMu, Sm7, Tat, CSY4, Qbeta, COM, pumilio, lambda N22, A non-exhaustive list of examples of ligand binding moieties/ligands that could be used in the methods and systems provided herein is summarised in Table 3.

**Table 3 - Non-exhaustive examples of ligand binding moiety-ligand pairs that may be used in various embodiments of the present invention. Both unmodified and chemically modified versions of the ligand binding moieties and ligands may be used.**

| Ligand Binding Moieties | Ligands |
| --- | --- |
| Telomerase Ku binding motif | Ku |
| Telomerase Sm7 binding motif | Sm7 |
| MS2 phage operator stem-loop | MS2 Coat Protein (MCP) |
| PP7 phage operator stem-loop | PP7 coat protein (PCP) |
| SfMu phage Com stem-loop | Com RNA binding protein |
| Non-natural RNA aptamer | Corresponding aptamer ligand |
| Tat binding motif | BIV Tat protein |
| Tat binding motif | HIV Tat protein |
| Pumilio binding motif | PUM-HD domain |
| BoxB binding motif | Lambda bacteriophage protein N (LambdaN-(1-22)) |
| Csy4 binding motif | Csy4[H29A] |
| Qbeta phage operator stem-loop | Qbeta coat protein [Q65H] |

In a preferred embodiment, the ligand binding moiety is an RNA motif (also can be referred to as an RNA aptamer) capable of associating with a cognate RNA binding domain (also can be referred to as an RNA aptamer ligand) which, in effect, recruits the effector proteins (base editing enzymes) to the target DNA. In embodiments where the ligand binding moiety is an RNA motif, the RNA-ligand binding complex may be referred to as an RNA scaffold. As disclosed herein, the guide RNA and the Cas protein together form a CRISPR/Cas-based module for sequence targeting and recognition, while the RNA motif recruits, via an RNA binding domain, more than one effector protein, such as a base editing enzyme, which carries out one or more genetic modifications. Accordingly, the RNA scaffold reversibly connects the effector module (e.g. base editing enzyme) to the sequence recognition module (e.g. Type II Cas protein or Type V Cas protein). To this end, an RNA scaffold is designed such that an RNA motif (e.g., MS2 operator motif), which specifically binds to an RNA binding protein (e.g., MS2 coat protein, MCP), is linked to the guide RNA.

As a result, this RNA scaffold component of the platform disclosed herein is a designed RNA molecule, which contains not only the guide RNA for specific DNA/RNA sequence recognition and Cas protein binding, but also the RNA motif (also known as the RNA aptamer) for effector recruitment. In this way, effector proteins can be recruited to the target site through their ability to bind to the RNA motif through an associated or fused RNA binding domain. Due to the flexibility of RNA scaffold mediated recruitment, a functional monomer, as well as dimer, trimer, tetramer, or oligomer could be relatively easy to form near the target DNA or RNA sequence. Preferably, dimeric RNA binding domains are recruited to a single RNA motif. These pairs of RNA motif/binding protein could be derived from naturally occurring sources (e.g., RNA phages, or yeast telomerase) or could be artificially designed (e.g., RNA aptamers and their corresponding binding protein ligands). A non-exhaustive list of examples of recruiting RNA motif/RNA binding protein pairs that could be used in the methods and systems provided herein is summarised in Table 4. As will be apparent to the skilled person, chemically modified versions and/or or sequence variants of the aptamers and their binding partners may also be utilised.

**Table 4. Examples of recruiting RNA motifs that can be used in this invention,** as **well** as **their pairing RNA binding proteins/protein domains.**

| RNA motif | Pairing interacting protein (RNA binding domain)* | Organism |
|---|---|---|
| Telomerase Ku binding motif | Ku | Yeast |
| Telomerase Sm7 binding motif | Sm7 | Yeast |
| MS2 phage operator stem-loop | MS2 Coat Protein (MCP) | Phage |
| PP7 phage operator stem-loop | PP7 coat protein (PCP) | Phage |
| Qbeta phage operator stem-loop | Qbeta coat protein [Q65H] | Phage |
| BoxB | Lambda bacteriophage protein N (LambdaN-(1-22) | Phage |
| Csy4 binding motif | Csy4[H29A] | Bacteria |
| SfMu phage Com stem-loop | Com RNA binding protein | Phage |
| Non-natural RNA aptamer | Corresponding aptamer ligand | Artificially designed |

*In the present invention, RNA binding domains are fused to effector proteins, for examples see **Table 5.**

The sequences for the above binding pairs are listed below.

**1. Telomerase Ku binding motif / Ku heterodimer**

**a. Ku binding hairpin (SEQ ID No. 7)**

5'-

UUCUUGUCGUACUUAUAGAUCGCUACGUUAUUUCAAUUUUGAAAAUCUGAGUCCUGGGAGUGCGGA-

3'

**b. Ku heterodimer** (SEQ ID No. 8)

MSGWESYYKTEGDEEAEEEQEENLEASGDYKYSGRDSLIFLVDASKAMFESQSEDELTPFDMSIQCIQSVYISKIISS

DRDLLAVVFYGTEKDKNSVNFKNIYVLQELDNPGAKRILELDQFKGQQGQKRFQDMMGHGSDYSLSEVLWVCAN

LFSDVQFKMSHKRIMLFTNEDNPHGNDSAKASRARTKAGDLRDTGIFLDLMHLKKPGGFDISLFYRDIISIAEDEDLR

VHFEESSKLEDLLRKVRAKETRKRALSRLKLKLNKDIVISVGIYNLVQKALKPPPIKLYRETNEPVKTKTRTFNTSTGGLL

LPSDTKRSQIYGSRQIILEKEETEELKRFDDPGLMLMGFKPLVLLKKHHYLRPSLFVYPEESLVIGSSTLFSALLIKCLEKE

VAALCRYTPRRNIPPYFVALVPQEEELDDQKIQVTPPGFQLVFLPFADDKRKMPFTEKIMATPEQVGKMKAIVEKLR

FTYRSDSFENPVLQQHFRNLEALALDLMEPEQAVDLTLPKVEAMNKRLGSLVDEFKELVYPPDYNPEGKVTKRKHD

NEGSGSKRPKVEYSEEELKTHISKGTLGKFTVPMLKEACRAYGLKSGLKKQELLEALTKHFQD


MVRSGNKAAVVLCMDVGFTMSNSIPGIESPFEQAKKVITMFVQRQVFAENKDEIALVLFGTDGTDNPLSGGDQY

QNITVHRHLMLPDFDLLEDIESKIQPGSQQADFLDALIVSMDVIQHETIGKKFEKRHIEIFTDLSSRFSKSQLDIIIHSLK

KCDISERHSIHWPCRLTIGSNLSIRIAAYKSILQERVKKTWTVVDAKTLKKEDIQKETVYCLNDDDETEVLKEDIIQGFR

YGSDIVPFSKVDEEQMKYKSEGKCFSVLGFCKSSQVQRRFFMGNQVLKVFAARDDEAAAVALSSLIHALDDLDMV

AIVRYAYDKRANPQVGVAFPHIKHNYECLVYVQLPFMEDLRQYMFSSLKNSKKYAPTEAQLNAVDALIDSMSLAKK

DEKTDTLEDLFPTTKIPNPRFQRLFQCLLHRALHPREPLPPIQQHIWNMLNPPAEVTTKSQIPLSKIKTLFPLIEAKKKD

QVTAQEIFQDNHEDGPTAK

### 2. Telomerase Sm7 biding motif / Sm7 homoheptamer

**a. Sm consensus site (single stranded)** (SEQ ID No. 9)
5'-AAUUUUUGGA-3'
**b. Monomeric Sm - like protein (archaea)** (SEQ ID No. 10)


GSVIDVSSQRVNVQRPLDALGNSLNSPVIIKLKGDREFRGVLKSFDLHMNLVLNDAEELEDGEVTRRLGTVLIRGDN

IVYISP

### 3. MS2 phage operator stem loop / MS2 coat protein

**a. MS2 phage operator stem loop** (SEQ ID No. 11)
5'- GCGCACAUGAGGAUCACCCAUGUGC -3'
**b. MS2 coat protein** (SEQ ID No. 12)


MASNFTQFVLVDNGGTGDVTVAPSNFANGIAEWISSNSRSQAYKVTCSVRQSSAQNRKYTIKVEVPKGAWRSYL

NMELTIPIFATNSDCELIVKAMQGLLKDGNPIPSAIAANSGIY

### 4. PP7 phage operator stem loop / PP7 coat protein

**a. PP7 phage operator stem loop**
5'-aUAAGGAGUUUAUAUGGAAACCCUUA -3' (SEQ ID No. 13)
**b. PP7 coat protein (PCP)** (SEQ ID No. 14)


MSKTIVLSVGEATRTLTEIQSTADRQIFEEKVGPLVGRLRLTASLRQNGAKTAYRVNLKLDQADVVDCSTSVCGELPK

VRYTQVWSHDVTIVANSTEASRKSLYDLTKSLVATSQVEDLVVNLVPLGR.

### 5. SfMu Com stem loop / SfMu Com binding protein

**a. SfMu Com stem loop** (SEQ ID No. 15)
5'-CUGAAUGCCUGCGAGCAUC-3'
**b. SfMu Com binding protein** (SEQ ID No. 16)
MKSIRCKNCNKLLFKADSFDHIEIRCPRCKRHIIMLNACEHPTEKHCGKREKITHSDETVRY

Further recruiting RNA motifs and their RNA binding proteins/protein domains are Qbeta phage operator stem loop (SEQ ID No. 68) and Qbeta coat protein [Q65H] (SEQ ID No. 69), BoxB (SEQ ID No. 70) and Lambda bacteriophage protein N (SEQ ID No. 71), Csy4 binding motif (SEQ ID No. 72) and Csy4[H29A] (SEQ ID No. 73).

The RNA-ligand binding complex may comprise one or more ligand binding moieties located at the 3' end of, 5' end of or within the guide RNA of the RNA-ligand binding complex. In a preferred embodiment, the RNA-ligand binding complex may comprise one or more RNA motifs enabling recruitment of one or more effectors. In any embodiment where the RNA-ligand binding complex comprises more than one RNA motif, the RNA motifs may be the same or different. In an embodiment where the RNA motifs are different, each motif may recruit either a single or multiple effector proteins. In an embodiment where multiple effector proteins are recruited, these proteins may have the same or different functions. In all embodiments, at least one of the RNA motifs will be used to recruit two or more effector proteins.

In a preferred embodiment, at least one RNA motif may be an MS2 aptamer, optionally wherein the MS2 aptamer has an extended stem. The extended stem may comprise 2 -24 nucleotides. In an embodiment, at least one RNA motif may be a PP7 aptamer. The MS2 aptamer and PP7 aptamer may recruit a single effector protein or multiple effector proteins each. The MS2 aptamer and PP7 aptamer may recruit the same or different effector proteins.

The RNA ligand binding complex (or RNA scaffold) can be either a single RNA molecule or a complex of multiple RNA molecules. For example, the guide RNA (comprising either crRNA, crRNA and tracrRNA, or crRNA and scoutRNA) and ligand binding moiety (or recruiting RNA motif) can be three segments of one, long single RNA molecule. Alternatively, the various components can be on separate molecules. In the latter case, the two or more components can be linked together to form the scaffold via covalent or non-covalent linkage or binding, including e.g., Watson-Crick base-pairing.

In one example, the RNA scaffold can comprise two separate RNA molecules. The first RNA molecule can comprise the programmable crRNA and a region that can form a stem duplex structure with a complementary region. The second RNA molecule can comprise the complementary region in addition to the tracrRNA and one or more RNA motif(s). Via this stem duplex structure, the first and second RNA molecules form an RNA scaffold of this invention. In one embodiment, the first and second RNA molecules each comprise a sequence (of about 6 to about 30 nucleotides) that base pairs to the other sequence. By the same token, the tracrRNA and the RNA motif can also be on different RNA molecules and be brought together with another stem duplex structure.

In another example, the RNA scaffold can comprise two separate RNA molecules. The first RNA molecule can comprise the programmable crRNA and a complementary region. The second RNA molecule can comprise the cognate complementary region in addition to the scoutRNA and one or more RNA motif(s). Through hybridisation of the complementary regions, the first and second RNA molecules form an RNA scaffold of this invention. In one embodiment, the first and second RNA molecules each comprise a sequence (of about 4 to 10 nucleotides, preferably 5 nucleotides) that base pairs to the other sequence. By the same token, the scout RNA and the RNA motif can also be on different RNA molecule and be brought together with another stem duplex structure.

The RNAs and related scaffold of this invention can be made by various methods known in the art including cell-based expression, in vitro transcription, and chemical synthesis. The ability to chemically synthesise relatively long RNAs (as long as 200 mers or more) using TC-RNA chemistry (see, e.g., US Patent 8,202,983) allows one to produce RNAs with special features that outperform those enabled by the basic four ribonucleotides (A, C, G and U).

The Cas protein-RNA ligand binding complexes can be made with recombinant technology using a host cell system or an in vitro translation-transcription system known in the art. Details of such systems and technology can be found in e.g., WO2014144761 WO2014144592, WO2013176772, US20140273226, and US20140273233. The complexes can be isolated or purified, at least to some extent, from cellular material of a cell or an in vitro translation-transcription system in which they are produced.

### Positioning of the Ligand Binding Moiety

The ligand binding moiety may be positioned at various positions within the RNA-ligand binding complex. The ligand binding moiety may be bound to the guide RNA directly (e.g. through a covalent bond), or through a linker that is associated with each of the guide RNA and the ligand binding moiety through one or more covalent bonds. The association of the ligand binding moiety with the guide RNA, regardless of whether directly through a covalent bond or through a linker may be at any of a number of locations.

By way of non-limiting examples, the ligand binding moiety may be bound directly (e.g. through a covalent bond) or through a linker to the 3' end of the guide RNA or to the 5' end of the guide RNA if the guide RNA is a single strand. Alternatively, the ligand binding moiety may be bound directly (e.g. through a covalent bond) or through a linker to the 3' end or 5' end of the components that make up the guide RNA, namely the crRNA, tracrRNA or scoutRNA.

When the ligand binding moiety is a nucleotide sequence e.g. an RNA motif it may be inserted in to the guide RNA.

More than one ligand binding moiety may be incorporated in to the RNA-ligand binding complex, in any one of the positions outlined in the non-limiting examples below. Where multiple ligand binding moieties are incorporated they may be adjacent to one another, or attached at distinct locations.

Wherein the RNA-ligand binding complex is an RNA scaffold, the RNA motif may be positioned at various positions of the RNA scaffold as described in Example 1. The RNA motif e.g. the MS2 aptamer can be positioned at the 3' end of the crRNA, at the tetra loop of the sgRNA, at stem loop 2 of the tracrRNA or at the stem loop 3 of the tracrRNA. The positioning of the MS2 aptamer is crucial due to the steric hindrance that can result from the bulky loops. In a preferred embodiment, the MS2 aptamer is at the 3'end of the tracrRNA. In an alternative embodiment, the MS2 aptamer is at the 5'end of the scoutRNA. In an alternative embodiment, the MS2 aptamer is at the 5'end of the tracrRNA. Advantageously, the positioning of the MS2 aptamer at the 3'end of the tracrRNA or 5' end of the scoutRNA is in space therefore reducing steric hindrance with other bulky loops of the RNA scaffold.

**Positioning of the Ligand Binding Moiety: Type II crRNA:tracrRNA Guide**

Wherein the guide RNA is a type II crRNA:tracrRNA guide, the ligand binding moiety may be bound directly or through a linker to the 3' end of the guide RNA or to the 5' end of the guide RNA if the guide RNA is a single strand (which correspond to the 3' end of the tracrRNA and the 5' end of the crRNA in the sgRNA) or to the 3' end of the crRNA, to the 3' end of the tracrRNA, to the 5' end of the tracrRNA, or to the 5' end of the crRNA if they are separate strands. Thus the ligand binding moiety may be bound to the first or last nucleotide in sgRNA, the crRNA or the tracrRNA. Alternatively, the ligand binding moiety may be bound to a nucleotide that is not the first or last nucleotide in the sgRNA, the tracrRNA or the crRNA. Alternatively, the association of the ligand binding moiety with the guide RNA may be at the tracrRNA in either the anti-repeat region or the distal region, or at the crRNA adjacent to the spacer or in the repeat region, or a tetraloop between the tracrRNA and the crRNA, if present.

**Positioning of the Ligand Binding Moiety: Type V crRNA:tracrRNA Guide**

Wherein the guide RNA is a type V crRNA:tracrRNA guide, the ligand binding moiety may be bound directly or through a linker to the 3' end of the guide RNA or to the 5' end of the guide RNA if the guide RNA is a single strand (which correspond to the 3' end of the crRNA and the 5' end of the tracrRNA in the sgRNA) or to the 3' end of the crRNA, to the 3' end of the tracrRNA, to the 5' end of the tracrRNA, or to the 5' end of the crRNA if they are separate strands. Thus the ligand binding moiety may be bound to the first or last nucleotide in sgRNA, the crRNA or the tracrRNA. Alternatively, the ligand binding moiety may be bound to a nucleotide that is not the first or last nucleotide in the sgRNA, the tracrRNA or the crRNA. Alternatively, the association of the ligand binding moiety with the guide RNA may be at the tracrRNA in either the anti-repeat region or the distal region, or at the crRNA adjacent to the spacer or in the repeat region, or a tetraloop between the tracrRNA and the crRNA, if present. Non-limiting exemplary positions for the ligand binding moiety can be seen in Fig. 2, Fig. 3 and Fig. 4.

**Positioning of the Ligand Binding Moiety: Type V crRNA:scoutRNA Guide**

Wherein the guide RNA is a crRNA:scoutRNA guide the ligand binding moiety may be bound directly or through a linker to the 3' end of the crRNA, to the 3' end of the scoutRNA, to the 5' end of the scoutRNA or to the 5' end of the crRNA. Thus the ligand binding moiety may be bound to the first or last nucleotide in the guide RNA, the crRNA or the scoutRNA. Alternatively, the ligand binding moiety may be bound to a nucleotide that is not the first or last nucleotide in the guide RNA, scoutRNA or crRNA. Alternatively, the association of the ligand binding moiety with the guide RNA may be at the scoutRNA in either the anti-repeat region or the upstream region or the downstream region, or at the crRNA adjacent to the spacer or in the repeat region. Non-limiting exemplary positions for the ligand binding moiety can be seen in Fig. 6.

**Positioning of the Ligand Binding Moiety: Type V crRNA-only Guide**

Wherein the guide RNA is a Type V crRNA-only guide, the ligand binding moiety may be bound directly or through a linker to the 3' end of the guide RNA or to the 5' end of the guide RNA. Thus the ligand binding moiety may be bound to the first or last nucleotide in the guide RNA. Alternatively, the ligand binding moiety may also be attached to the gRNA at a position other than the 5' end or the 3' end. Non-limiting exemplary positions for the ligand binding moiety can be seen in Fig. 5.

**Modifications**

The RNA-ligand binding complex (or RNA scaffold) as disclosed herein may include one or more modifications. Such modifications may include inclusion of at least one non-naturally occurring nucleotide, or a modified nucleotide, or analogues thereof. Examples of such modifications include, but are not limited to the addition of nucleotides to extend sequences, substitution of nucleotides, addition of linker sequences and modifying the positioning of various components of the RNA-ligand binding complex (or RNA scaffold).

Nucleotides may be modified at the ribose, phosphate linkage, and/or base moiety. Modified nucleotides may include 2'-O-methyl analogs, 2'-fluoro analogs or 2'-deoxy analogs or 2'-ribose analogs. The nucleic acid backbone may be modified, for example, a phosphorothioate backbone may be used. The use of locked nucleic acids (LNA) or bridged nucleic acids (BNA) may also be possible. Further examples of modified bases include, but are not limited to, 2-aminopurine, 5-bromo-uridine, 5-methylcytidine, 5-methoxyuridine, pseudouridine, inosine, 7-methylguanosine. These modifications may apply to any component of the RNA-ligand binding complex (or RNA scaffold). These modifications may apply to any component of the CRISPR system. In a preferred embodiment these modifications are made to the RNA components, e.g., the guide RNA sequence.

In some embodiments, the RNA-ligand binding complex (or RNA scaffold) described above or a subsection thereof can comprise one or more modifications, e.g., a base modification, a backbone modification, etc. to provide the nucleic acid with a new or enhanced feature (e.g., improved stability).

**Modified Backbones and Modified Inter-nucleoside Linkages**

Examples of suitable nucleic acids containing modifications include nucleic acids containing modified backbones, bases, sugars, or non-natural internucleoside linkages. Nucleic acids (having modified backbones include those that retain a phosphorus atom in the backbone and those that do not have a phosphorus atom in the backbone).

Suitable modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, phosphorodiamidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Suitable oligonucleotides having inverted polarity comprise a single 3' to 3' linkage at the 3'-most internucleotide linkage i.e. a single inverted nucleoside residue which may be a basic (the nucleobase is missing or has a hydroxyl group in place thereof). Various salts (such as, for example, potassium or sodium), mixed salts and free acid forms are also included.

In some embodiments, a subject nucleic acid comprises one or more phosphorothioate and/or heteroatom internucleoside linkages, in particular -CH2-NH-O-CH2-, -CH2-N(CH3)-O-CH2-(known as a methylene (methylimino) or MMI backbone), -CH2-O-N(CH3)-CH2-, -CH2-N(CH3)-N(CH3)-CH2- and -O-N(CH3)-CH2-CH2- (wherein the native phosphodiester internucleotide linkage is represented as -O-P(=O)(OH)-O-CH2-). MMI type internucleoside linkages are disclosed in the above referenced U.S. Pat. No. 5,489,677. Suitable amide internucleoside linkages are disclosed in t U.S. Pat. No. 5,602,240.

Also suitable are nucleic acids having morpholino backbone structures as described in, e.g., U.S. Pat. No. 5,034,506. For example, in some embodiments, a subject nucleic acid comprises a 6-membered morpholino ring in place of a ribose ring. In some of these embodiments, a phosphorodiamidate or other non-phosphodiester internucleoside linkage replaces a phosphodiester linkage.

Suitable modified polynucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH2 component parts.

**Modified Sugar Moieties**

A subject nucleic acid can also include one or more substituted sugar moieties. Suitable polynucleotides comprise a sugar substituent group selected from: OH; H; F; O-, S-, or N-alkyl; O-, S-, or N-alkenyl; O-, S- or N-alkynyl; or O-alkyl-Co-alkyl, wherein the alkyl, alkenyl and alkynyl may be substituted or unsubstituted C1 to C10 alkyl or C2 to C10 alkenyl and alkynyl. Particularly suitable are O((CH2)nO)mCH3, O(CH2)nOCH3, O(CH2)nNH2, O(CH2)nCH3, O(CH2)nONH2, and O(CH2)nON((CH2)nCH3)2, where n and m are from 1 to about 10. Other suitable polynucleotides comprise a sugar substituent group selected from: C1 to C10 lower alkyl, substituted lower alkyl, alkenyl, alkynyl, alkaryl, aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A suitable modification includes 2'-methoxyethoxy (2'-O-CH2 CH2OCH3, also known as 2'-O-(2-methoxyethyl) or 2'-MOE) (Martin et al., Helv. Chim. Acta, 1995, 78, 486-504) i.e., an alkoxyalkoxy group. A further suitable modification includes 2'-dimethylaminooxyethoxy, i.e., a O(CH2)2ON(CH3)2 group, also known as 2'-DMAOE, as described in examples hereinbelow, and 2'-dimethylaminoethoxyethoxy (also known in the art as 2'-O-dimethyl-amino-ethoxy-ethyl or 2'-DMAEOE), i.e., 2'-O-CH2-O-CH2-N(CH3)2.

Other suitable sugar substituent groups include methoxy (-O-CH3), aminopropoxy (-O CH2 CH2 CH2NH2), allyl (-CH2-CH=CH2), -O-allyl CH2-CH=CH2) and fluoro (F). 2'-sugar substituent groups may be in the arabino (up) position or ribo (down) position. A suitable 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar.

**Base Modifications and Substitutions**

A subject nucleic acid may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine (1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g. 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo(2,3-d)pyrimidin-2-one).

Heterocyclic base moieties may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., Angewandte Chemie, International Edition, 1991, 30, 613, and those disclosed by Sanghvi, Y. S., Chapter 15, Antisense Research and Applications, pages 289-302, Crooke, S. T. and Lebleu, B., ed., CRC Press, 1993. Certain of these nucleobases are useful for increasing the binding affinity of an oligomeric compound. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C. (Sanghvi et al., eds., Antisense Research and Applications, CRC Press, Boca Raton, 1993, pp. 276-278) and are suitable base substitutions, e.g., when combined with 2'-O-methoxyethyl sugar modifications.

Modifications as disclosed herein can be incorporated at various positions of the RNA-ligand binding complex (or RNA scaffold) such as at the tetra loop of a sgRNA, at the 3' end of the tracrRNA or scoutRNA and at the RNA motif, in stem loop 1, 2 or 3. Modifications disclosed herein include an extension of the repeat anti-repeat of a sgRNA, positioning of the RNA motif at the 3' end of the tracrRNA or scoutRNA, linker sequence linking the RNA motif to the tracrRNA or scoutRNA, modifying the RNA motif's nucleotides and extending the RNA motif.

**Modifying the RNA Motif's Nucleotides**

Wherein the RNA ligand binding complex is an RNA scaffold, modifications may be made to the RNA motif e.g. aptamer sequence. For example a suitable modification is to the C-5 and F-5 aptamer mutant. In a preferred embodiment, the modification to the aptamer is a substitution of the Adenine to 2-aminopurine (2-AP) at position 10. Advantageously, the substitution induces conformational changes resulting in greater affinity. The affinity for the MS2 coat protein by the F-5 mutant was approximately 3-fold higher than the parental F5 sequence. Whilst not wishing to be bound by any theory, it is believed that the conformational change induced by 2-AP results in hydrogen bond formation between the exocyclic amino group of the 2-AP nucleotide at position 10 and the carbonyl the B59 at the backbone. It is thought that replacing the MS2 hairpin sequence with the higher affinity MS2 sequences will result in increased based editing efficiencies because substituting amino acids helps to order the RNA stem loop into a conformation that is better recognised by the coat protein.

Suitable modifications to the RNA motif are listed above, such as 2' deoxy-2-aminopurine, 2'ribose-2-aminopurine, phosphorothioate mods, 2'-Omethyl mods, 2'-Fluro mods and LNA mods. Advantageously, the modifications help to increase stability and promote stronger bonds/folding structure of the desired hairpin.

**Extension of RNA Motif**

Wherein the RNA ligand binding complex is an RNA scaffold, the RNA motif can be extended. The length of the RNA motif extension can be variable. The extension to the RNA motif can range from 2-24 nucleotides. The extension to the RNA motif can be more than 24 nucleotides. Advantageously, the extension of the RNA motif increases flexibility of the motif. The extension to the RNA motif may be a double-stranded or a single-stranded extension. Double-stranded extension provides greater stabilisation of the RNA scaffold.

**Extension of the Repeat:AntiRepeat Upper Stem**

The guide RNA comprising a crRNA and tracrRNA can be provided as a sgRNA. The two components are linked via a repeat:anti-repeat. The repeat:antirepeat upper stem can be extended to increase the flexibility, proper folding and stability of the loop. The repeat:antirepeat can be extended by 2, 3, 4, 5, 6, 7 bp or more than 7 bp at either side of the tetra loop.

**Combination of Modifications**

The RNA scaffold may have one or more of the above mentioned modifications. The one or more modification can be on the different components of the RNA scaffold e.g. extension of repeat:antirepeat of the sgRNA and extension of the RNA motif, or can be on the same component of the RNA scaffold, e.g. extension of the RNA motif and substitution of the RNA motif's nucleotides. The modifications may be two or more, three or more, four or more, or five or more. In one embodiment, the modification may be the extension of the RNA motif and/or may the substitution of one or more nucleotides

An example of an RNA motif as used herein is the MS2 aptamer. The MS2 motif specifically binds to the MS2 bacteriophage coat protein (MCP). In vitro selection process was repeated yielding a series of aptamer families. Two of the aptamer family members include MS2 C-5 mutant and MS2 F-5 mutant. One of the significant differences between the wild-type MS2 and the C-5 and F-5 mutants is the substitution of the Uracil nucleotide to Cytosine at position 5 of the aptamer loop. The F-5 mutant has been reported to have higher affinity for the coat protein compared to the wild-type and other members of the aptamer family. Suitably, both C-5 mutants and F-5 mutants are used as aptamers in the present invention. In one embodiment, the MS2 aptamer is a wild-type MS2, a mutant MS2 or variants thereof. In another embodiment, the MS2 aptamer comprises a C-5 and/or F-5 mutation. The MS2 protein linked to the tracrRNA can be a single-copy (i.e. one MS2 loop) or a double-copy (i.e. two MS2 loops). In a preferred embodiment, the RNA motif is a single-copy. In other embodiments, the RNA motif is more than one copy.

*Linker Sequence*

A linker, when present, may be a species that connects the ligand binding moiety to the guide RNA. In an embodiment where the ligand binding moiety is an RNA motif, a linker may be a species that connects the RNA motif to the guide RNA. The linker may be attached to each of the RNA motif and the guide RNA at one location or it may be attached to either or both of the RNA motif and the guide RNA at a plurality of locations. Attachments at a plurality of locations may allow for greater control in the three dimensional space of the RNA motif and in turn the effector to be used. The RNA motif may be linked to the tracrRNA via a linker sequence. The linker sequence may be 2, 3, 4, 5, 6, 7 or more than 7 nucleotides. Advantageously, the linker sequence provides flexibility to the RNA-ligand binding complex (or RNA scaffold). The linker sequence may be a GC rich sequence.

In some embodiments, the linker comprises, consists essentially of, or consists of an oligonucleotide sequence and optionally the linker comprises at least one or a plurality of 2' modifications, e.g., all nucleotides are 2' modified nucleotides within the linker. The nucleotide sequence may be random or intentionally designed not to be undesirably complementary to sequence within the ligand binding moiety, the [RNA] or the target site of the DNA.

In some embodiments, the linker comprises, consists essentially of, or consists of at least one phosphorothioate linkage.

In some embodiments, the linker comprises, consists essentially of, or consists of a levulinyl moiety.

In some embodiments, the linker comprises, consists essentially of, or consists of an ethylene glycol moiety.

In some embodiments, the linker comprises or is selected from the group consisting of 18S, 9S or C3.

In some embodiments, the linker, or if there are more than one linker, each linker, is a nucleotide sequence that is one to sixty or one to twenty-four or two to twenty or five to fifteen nucleotides long. Additionally, in some embodiments, the linker is GC rich, e.g., having at least 50%, at least 60%, at least 70%, at least 80% or at least 90% GC nucleotides. When a linker comprises nucleotides, it may, for example, be single stranded or double stranded or partially single stranded and partially double stranded. Additionally, when a linker is an oligonucleotide, the linker may be exclusively RNA, exclusively DNA or a combination thereof.

### iii) Effector Protein

The third component of the platform disclosed in this invention is a non-nuclease effector attached to a ligand. The effector protein may also be referred to as the 'effector component'. In embodiments where an RNA scaffold is used, the ligand is an RNA binding domain. The effector is not a nuclease and does not have any nuclease activity but can have the activity of other types of DNA modifying enzymes, for example base editing. Examples of the enzymatic activity include, but are not limited to, deamination activity, methyltransferase activity, demethylase activity, DNA repair activity, DNA damage activity, dismutase activity, nickase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity. In some embodiments, the effector has the activity of cytidine deaminases (e.g., AID, APOBEC3G), adenosine deaminases (e.g., ADA), DNA methyltransferases, and DNA demethylases. In some embodiments, the effectors are from different vertebrate animal species and have distinct properties, or could be novel chimeric proteins assembled in silico to bring together the desirable attributes and activities.

In preferred embodiments, this third component is a conjugate or a fusion protein that has an RNA-binding domain and an effector domain. These two domains can be joined via a linker.

In some embodiments, no effector is needed in some cell types (e.g., cancer lines over-expressing deaminases). In that case, endogenous effector (e.g. APOBEC, AID, etc) can be gene-edited to include the recruitment module, so no exogenous editor is needed. This is applicable to cell types that express the editor of interest - e.g., lymphoid (B + T cells) and certain cancer cells.

### (c) Ligand

In any embodiment, the ligand of the present invention is selected such that it is capable of associating with the ligand binding moiety of the RNA ligand binding complex, thus recruiting the effector to the base-editing complex. Appropriate ligands and their cognate ligand binding moieties are shown in Table 3. Alternative ligands and their cognate ligand binding moieties are well known in the art.

Wherein the RNA ligand binding complex is an RNA scaffold, the ligand will be an RNA binding domain. Although various RNA-binding domains can be used as the ligand in this invention, the RNA-binding domain of Cas protein (such as Cas9) or its variant (such as dCas9) should not be used. As mentioned above, the direct fusion to dCas9, which anchors to DNA in a defined conformation, would hinder the formation of a functional oligomeric enzyme complex at the right location. Instead, the present invention takes advantages of various other RNA motif-RNA binding protein binding pairs. Examples include those listed in Table 4.

In this way, the effector protein can be recruited to the target site through RNA-binding domain's ability to bind to the recruiting RNA motif. Due to the flexibility of RNA scaffold mediated recruitment, a functional monomer, as well as dimer, tetramer, or oligomer could be formed relatively easily near the target DNA or RNA sequence. In each case, a monomer, dimer, tetramer or oligomer may be recruited to a single RNA motif. In a preferred embodiment, an effector protein comprising a dimeric RNA binding protein may be recruited to a single RNA motif. In such an embodiment, the two RNA binding domain monomers are each associated with an individual effector domain. The effector domains are the same or different. In an alternative embodiment, only one of the RNA binding domains constituting the dimer is associated with an effector domain.

**(d) Effector Domain**

The effector component comprises an activity portion, i.e., an effector domain. The effector domain may also be referred to as an 'effector'. In some embodiments, the effector domain comprises the naturally occurring activity portion of a non-nuclease protein (e.g., deaminases). In other embodiments, the effector domain comprises a modified amino acid sequence (e.g., substitution, deletion, insertion) of a naturally occurring activity portion of a non-nuclease protein. The effector domain has an enzymatic activity. Examples of this activity include deamination activity, methyltransferase activity, demethylase activity, DNA repair activity, DNA damage activity, dismutase activity, alkylation activity, depurination activity, oxidation activity, pyrimidine dimer forming activity, integrase activity, transposase activity, recombinase activity, polymerase activity, ligase activity, helicase activity, photolyase activity, glycosylase activity, DNA methylation, histone acetylation activity, or histone methylation activity. Some modifications in non-nuclease protein (e.g., deaminases) can help reduce off-target effect. For example, as described below, one can reduce the recruitment of AID to off-target sites by mutating Ser38 in AID to Ala (SEQ ID No. 26). A non-exhaustive list of examples of DNA/RNA modifying enzymes that can be used as effector domains is detailed in Table 5.

**Table 5. Examples of Effectors (or Effector Domains) that can be used in this invention**

| Enzyme type | Genetic change | Effector (Effector domain) abbreviated |
|---|---|---|
| Cytidine deaminase | C→U/T | AID<br>APOBEC1<br>APOBEC3A<br>APOBEC3B<br>APOBEC3C<br>APOBEC3D<br>APOBEC3F<br>APOBEC3G<br>APOBEC3H<br>CDA |
| Adenosine deaminase | A→I/G | ADA<br>ADAR1<br>ADAR2<br>ADAR3 |
| DNA Methyl transferase | C→Met-C | Dnmt1<br>Dnmt3a<br>Dnmt3b |
| Demethylase | Met-C→C | Tet1<br>Tet2<br>TDG |

**Effector (effector domain) full names:**

**AID:** activation induced cytidine deaminase, a.k.a AICDA
**APOBEC1:** apolipoprotein B mRNA editing enzyme, catalytic polypeptide-like 1.
**APOBEC3A:** apolipoprotein B mRNA editing enzyme, catalytic polypeptide-like 3A
**APOBEC3B:** apolipoprotein B mRNA editing enzyme, catalytic polypeptide-like 3B
**APOBEC3C:** apolipoprotein B mRNA editing enzyme, catalytic polypeptide-like 3C
**APOBEC3D:** apolipoprotein B mRNA editing enzyme, catalytic polypeptide-like 3D
**APOBEC3F:** apolipoprotein B mRNA editing enzyme, catalytic polypeptide-like 3F
**APOBEC3G:** apolipoprotein B mRNA editing enzyme, catalytic polypeptide-like 3G
**APOBEC3H:** apolipoprotein B mRNA editing enzyme, catalytic polypeptide-like 3H
**ADA:** adenosine deaminase
**ADAR1:** adenosine deaminase acting on RNA 1

**ADAR2:** adenosine deaminase acting on RNA 2
**ADAR3:** adenosine deaminase acting on RNA 3
**CDA:** Cytidine deaminase
**Dnmt1:** DNA (cytosine-5-)-methyltransferase 1
**Dnmt3a:** DNA (cytosine-5-)-methyltransferase 3 alpha
**Dnmt3b:** DNA (cytosine-5-)-methyltransferase 3 beta
**Tet1:ten-eleven translocation 1**
**Tet2:** ten-eleven translocation 2
**Tdg:** thymine DNA glycosylase

In alternative embodiments, the effector domain may be a reporter, a fluorescent tag, an active agent or an HDR enhancing factor.

**Other Domains**

The effector protein can comprise other domains. In certain embodiments, the effector protein can comprise at least one nuclear localisation signal (NLS). In general, an NLS comprises a stretch of basic amino acids. Nuclear localisation signals are known in the art (see, e.g., Lange et al., J. Biol. Chem., 2007, 282:5101-5105). The NLS can be located at the N-terminus, the C-terminal, or in an internal location of the fusion protein. The NLSs help to optimise on-target base editing, resulting in more efficient base editing.

In some embodiments, the fusion protein can comprise at least one cell-penetrating domain to facilitate delivery of the protein into a target cell. In one embodiment, the cell-penetrating domain can be a cell-penetrating peptide sequence. Various cell-penetrating peptide sequences are known in the art and examples include that of the HIV-1 TAT protein, TLM of the human HBV, Pep-1, VP22, and a polyarginine peptide sequence.

In one embodiment, AID was used as an example to illustrate how the system works. AID is a cytidine deaminase that can catalyse the reaction of deamination of cytidine in the context of DNA or RNA. When brought to the targeted site, AID changes a C base to U base. In dividing cells, this could lead to a C to T point mutation. Alternatively, the change of C to U could trigger cellular DNA repair pathways, mainly excision repair pathway, which will remove the mismatching U-G base-pair, and replace with a T-A, A-T, C-G, or G-C pair. As a result, a point mutation would be generated at the target C-G site. As excision repair pathway is present in most, if not all, somatic cells, recruitment of AID to the target site can correct a C-G base pair to others. In that case, if a C-G base pair is an underlying disease-causing genetic mutation in somatic tissues/cells, the above-described approach can be used to correct the mutation and thereby treat the disease.

By the same token, if an underlying disease causing genetic mutation is an A-T base pair at a specific site, one can use the same approach to recruit an adenosine deaminase to the specific site, where adenosine deaminase can correct the A-T base pair to others, see for example David Liu - US10113163. Other effector enzymes are expected to generate other types of changes in base-pairing.

*Linker*

The above-mentioned two domains as well as others as disclosed herein can be joined by means of linkers, such as, but not limited to chemical modification, peptide linkers, chemical linkers, covalent or non-covalent bonds, or protein fusion or by any means known to one skilled in the art. The joining can be permanent or reversible. See for example U.S. Pat. Nos. 4625014, 5057301 and 5514363, US Application Nos. 20150182596 and 20100063258, and WO2012142515. In some embodiments, several linkers can be included in order to take advantage of desired properties of each linker and each protein domain in the conjugate. For example, flexible linkers and linkers that increase the solubility of the conjugates are contemplated for use alone or with other linkers. Peptide linkers can be linked by expressing DNA encoding the linker to one or more protein domains in the conjugate. Linkers can be acid cleavable, photocleavable and heat sensitive linkers. Methods for conjugation are well known by persons skilled in the art and are encompassed for use in the present invention.

In some embodiments, the RNA-binding domain and the effector domain can be joined by a peptide linker. Peptide linkers can be linked by expressing nucleic acid encoding in frame the two domains and the linker. Optionally the linker peptide can be joined at either or both of the amino terminus and carboxy terminus of the domains. In some examples, a linker is an immunoglobulin hinge region linker as disclosed in U.S. Pat. Nos. 6,165,476, 5,856,456, US Application Nos. 20150182596 and 2010/0063258 and International Application WO2012/142515.

The above-described three specific components ((i) sequence targeting component (or sequence targeting protein), (ii) RNA-ligand binding complex and (iii) effector protein) constitute the technological platform. Each component could be chosen from the Tables 1 to 5 to achieve a specific therapeutic/utility goal. It will be understood by the skilled person that the guide RNA must be suited for the sequence targeting protein selected. It will also be understood by the skilled person that the ligand binding moiety must be suitable for the ligand selected and vice versa.

A non-limiting example of an RNA scaffold mediated recruitment system constructed using (i) dCas9 from S. pyogenes as the sequence targeting protein, (ii) an RNA scaffold comprising a crRNA, tracrRNA, and a MS2 operator motif, and (iii) an effector fusion comprising a human AID fusing to MS2 operator binding protein MCP and an effector fusion containing PmCDA1 fused to MCP is shown below. The sequences for the components are listed below.

**Amino acid sequence Streptococcus pyogenes dCas9 (D10A, H840A active site mutants in bold and underlined) (SEQ ID No. 17)**

MDKKYSIGLAIGTNSVGWAVITDEYKVPSKKFKVLGNTDRHSIKKNLIGALLFDSGETAEATRLKRTARRRYTRRKNR

ICYLQEIFSNEMAKVDDSFFHRLEESFLVEEDKKHERHPIFGNIVDEVAYHEKYPTIYHLRKKLVDSTDKADLRLIYLAL

AHMIKFRGHFLIEGDLNPDNSDVDKLFIQLVQTYNQLFEENPINASGVDAKAILSARLSKSRRLENLIAQLPGEKKNG

LFGNLIALSLGLTPNFKSNFDLAEDAKLQLSKDTYDDDLDNLLAQIGDQYADLFLAAKNLSDAILLSDILRVNTEITKAP

LSASMIKRYDEHHQDLTLLKALVRQQLPEKYKEIFFDQSKNGYAGYIDGGASQEEFYKFIKPILEKMDGTEELLVKLN

REDLLRKQRTFDNGSIPHQIHLGELHAILRRQEDFYPFLKDNREKIEKILTFRIPYYVGPLARGNSRFAWMTRKSEETI

TPWNFEEVVDKGASAQSFIERMTNFDKNLPNEKVLPKHSLLYEYFTVYNELTKVKYVTEGMRKPAFLSGEQKKAIV

DLLFKTNRKVTVKQLKEDYFKKIECFDSVEISGVEDRFNASLGTYHDLLKIIKDKDFLDNEENEDILEDIVLTLTLFEDRE

MIEERLKTYAHLFDDKVMKQLKRRRYTGWGRLSRKLINGIRDKQSGKTILDFLKSDGFANRNFMQLIHDDSLTFKE

DIQKAQVSGQGDSLHEHIANLAGSPAIKKGILQTVKVVDELVKVMGRHKPENIVIEMARENQTTQKGQKNSRERM

KRIEEGIKELGSQILKEHPVENTQLQNEKLYLYYLQNGRDMYVDQELDINRLSDYDVDAIVPQSFLKDDSIDNKVLTR

SDKNRGKSDNVPSEEVVKKMKNYWRQLLNAKLITQRKFDNLTKAERGGLSELDKAGFIKRQLVETRQITKHVAQIL

DSRMNTKYDENDKLIREVKVITLKSKLVSDFRKDFQFYKVREINNYHHAHDAYLNAVVGTALIKKYPKLESEFVYGDY

KVYDVRKMIAKSEQEIGKATAKYFFYSNIMNFFKTEITLANGEIRKRPLIETNGETGEIVWDKGRDFATVRKVLSMP

QVNIVKKTEVQTGGFSKESILPKRNSDKLIARKKDWDPKKYGGFDSPTVAYSVLVVAKVEKGKSKKLKSVKELLGITI

MERSSFEKNPIDFLEAKGYKEVKKDLIIKLPKYSLFELENGRKRMLASAGELQKGNELALPSKYVNFLYLASHYEKLKG

SPEDNEQKQLFVEQHKHYLDEIIEQISEFSKRVILADANLDKVLSAYNKHRDKPIREQAENIIHLFTLTNLGAPAAFKYF

DTTIDRKRYTSTKEVLDATLIHQSITGLYETRIDLSQLGGD

(Residues underlined: D10A, H840A active site mutants)

**Cas9 D10A Amino Acid Sequence (mutated residues underlined: D10A, SEQ ID No. 18)**

DKKYSIGL**A**IGTNSVGWAVITDEYKVPSKKFKVLGNTDRHSIKKNLIGALLFDSGETAEATRLKRTARRRYTRRKNRIC

YLQEIFSNEMAKVDDSFFHRLEESFLVEEDKKHERHPIFGNIVDEVAYHEKYPTIYHLRKKLVDSTDKADLRLIYLALA

HMIKFRGHFLIEGDLNPDNSDVDKLFIQLVQTYNQLFEENPINASGVDAKAILSARLSKSRRLENLIAQLPGEKKNGL

FGNLIALSLGLTPNFKSNFDLAEDAKLQLSKDTYDDDLDNLLAQIGDQYADLFLAAKNLSDAILLSDILRVNTEITKAPL

SASMIKRYDEHHQDLTLLKALVRQQLPEKYKEIFFDQSKNGYAGYIDGGASQEEFYKFIKPILEKMDGTEELLVKLNR

EDLLRKQRTFDNGSIPHQIHLGELHAILRRQEDFYPFLKDNREKIEKILTFRIPYYVGPLARGNSRFAWMTRKSEETIT

PWNFEEVVDKGASAQSFIERMTNFDKNLPNEKVLPKHSLLYEYFTVYNELTKVKYVTEGMRKPAFLSGEQKKAIVD

LLFKTNRKVTVKQLKEDYFKKIECFDSVEISGVEDRFNASLGTYHDLLKIIKDKDFLDNEENEDILEDIVLTLTLFEDREM

IEERLKTYAHLFDDKVMKQLKRRRYTGWGRLSRKLINGIRDKQSGKTILDFLKSDGFANRNFMQLIHDDSLTFKEDI

QKAQVSGQGDSLHEHIANLAGSPAIKKGILQTVKVVDELVKVMGRHKPENIVIEMARENQTTQKGQKNSRERMK

RIEEGIKELGSQILKEHPVENTQLQNEKLYLYYLQNGRDMYVDQELDINRLSDYDVDHIVPQSFLKDDSIDNKVLTRS

DKNRGKSDNVPSEEVVKKMKNYWRQLLNAKLITQRKFDNLTKAERGGLSELDKAGFIKRQLVETRQITKHVAQILD

SRMNTKYDENDKLIREVKVITLKSKLVSDFRKDFQFYKVREINNYHHAHDAYLNAVVGTALIKKYPKLESEFVYGDYK

VYDVRKMIAKSEQEIGKATAKYFFYSNIMNFFKTEITLANGEIRKRPLIETNGETGEIVWDKGRDFATVRKVLSMPQ

VNIVKKTEVQTGGFSKESILPKRNSDKLIARKKDWDPKKYGGFDSPTVAYSVLVVAKVEKGKSKKLKSVKELLGITIM

ERSSFEKNPIDFLEAKGYKEVKKDLIIKLPKYSLFELENGRKRMLASAGELQKGNELALPSKYVNFLYLASHYEKLKGSP

EDNEQKQLFVEQHKHYLDEIIEQISEFSKRVILADANLDKVLSAYNKHRDKPIREQAENIIHLFTLTNLGAPAAFKYFD

TTIDRKRYTSTKEVLDATLIHQSITGLYETRIDLSQLGGD

**Humanised Cas9 D10A Protein Nucleotide Sequence (SEQ ID No. 19)**

GATAAAAAGTATTCTATTGGTTTAGCCATCGGCACTAATTCCGTTGGATGGGCTGTCATAACCGATGAATACA

AAGTACCTTCAAAGAAATTTAAGGTGTTGGGGAACACAGACCGTCATTCGATTAAAAAGAATCTTATCGGTGC

CCTCCTATTCGATAGTGGCGAAACGGCAGAGGCGACTCGCCTGAAACGAACCGCTCGGAGAAGGTATACACG

TCGCAAGAACCGAATATGTTACTTACAAGAAATTTTTAGCAATGAGATGGCCAAAGTTGACGATTCTTTCTTTC

ACCGTTTGGAAGAGTCCTTCCTTGTCGAAGAGGACAAGAAACATGAACGGCACCCCATCTTTGGAAACATAGT

AGATGAGGTGGCATATCATGAAAGTACCCAACGATTTATCACCTCAGAAAAAAGCTAGTTGACTCAACTGAT

AAAGCGGACCTGAGGTTAATCTACTTGGCTCTTGCCCATATGATAAAGTTCCGTGGGCACTTTCTCATTGAGG

GTGATCTAAATCCGGACAACTCGGATGTCGACAAACTGTTCATCCAGTTAGTACAAACCTATAATCAGTTGTTT

GAAGAGAACCCTATAAATGCAAGTGGCGTGGATGCGAAGGCTATTCTTAGCGCCCGCCTCTCTAAATCCCGAC

GGCTAGAAAACCTGATCGCACAATTACCCGGAGAGAAGAAAATGGGTTGTTCGGTAACCTTATAGCGCTCTC

ACTAGGCCTGACACCAAATTTTAAGTCGAACTTCGACTTAGCTGAAGATGCCAAATTGCAGCTTAGTAAGGAC

ACGTACGATGACGATCTCGACAATCTACTGGCACAAATTGGAGATCAGTATGCGGACTTATTTTTGGCTGCCA

AAAACCTTAGCGATGCAATCCTCCTATCTGACATACTGAGAGTTAATACTGAGATTACCAAGGCGCCGTTATCC

GCTTCAATGATCAAAAGGTACGATGAACATCACCAAGACTTGACACTTCTCAAGGCCCTAGTCCGTCAGCAAC

TGCCTGAGAAATATAAGGAAATATTCTTTGATCAGTCGAAAAACGGGTACGCAGGTTATATTGACGGCGGAG

CGAGTCAAGAGGAATTCTACAAGTTTATCAAACCCATATTAGAGAAGATGGATGGGACGGAAGAGTTGCTTG

TAAAACTCAATCGCGAAGATCTACTGCGAAAGCAGCGGACTTTCGACAACGGTAGCATTCCACATCAAATCCA

CTTAGGCGAATTGCATGCTATACTTAGAAGGCAGGAGGATTTTTATCCGTTCCTCAAAGACAATCGTGAAAAG

ATTGAGAAAATCCTAACCTTTCGCATACCTTACTATGTGGGACCCCTGGCCCGAGGGAACTCTCGGTTCGCATG

GATGACAAGAAAGTCCGAAGAAACGATTACTCCATGGAATTTTGAGGAAGTTGTCGATAAAGGTGCGTCAGC

TCAATCGTTCATCGAGAGGATGACCAACTTTGACAAGAATTTACCGAACGAAAAGTATTGCCTAAGCACAGT

TTACTTTACGAGTATTTCACAGTGTACAATGAACTCACGAAAGTTAAGTATGTCACTGAGGGCATGCGTAAAC

CCGCCTTTCTAAGCGGAGAACAGAAGAAAGCAATAGTAGATCTGTTATTCAAGACCAACCGCAAAGTGACAG

TTAAGCAATTGAAAGAGGACTACTTTAAGAAAATTGAATGCTTCGATTCTGTCGAGATCTCCGGGGTAGAAGA

TCGATTTAATGCGTCACTTGGTACGTATCATGACCTCCTAAAGATAATTAAAGATAAGGACTTCCTGGATAACG

AAGAGAATGAAGATATCTTAGAAGATATAGTGTTGACTCTTACCCTCTTTGAAGATCGGGAAATGATTGAGGA

AAGACTAAAAACATACGCTCACCTGTTCGACGATAAGGTTATGAAACAGTTAAAGAGGCGTCGCTATACGGG

CTGGGGACGATTGTCGCGGAAACTTATCAACGGGATAAGAGACAAGCAAAGTGGTAAAACTATTCTCGATTT

TCTAAAGAGCGACGGCTTCGCCAATAGGAACTTTATGCAGCTGATCCATGATGACTCTTTAACCTTCAAAGAG

GATATACAAAAGGCACAGGTTTCCGGACAAGGGGACTCATTGCACGAACATATTGCGAATCTTGCTGGTTCGC

CAGCCATCAAAAAGGGCATACTCCAGACAGTCAAAGTAGTGGATGAGCTAGTTAAGGTCATGGGACGTCACA

AACCGGAAAACATTGTAATCGAGATGGCACGCGAAAATCAAACGACTCAGAAGGGGCAAAAAAACAGTCGA

GAGCGGATGAAGAGAATAGAAGAGGGTATTAAAGAACTGGGCAGCCAGATCTTAAAGGAGCATCCTGTGGA

AAATACCCAATTGCAGAACGAGAAACTTTACCTCTATTACCTACAAAATGGAAGGGACATGTATGTTGATCAG

GAACTGGACATAAACCGTTTATCTGATTACGACGTCGATCACATTGTACCCCAATCCTTTTTGAAGGACGATTC

AATCGACAATAAAGTGCTTACACGCTCGGATAAGAACCGAGGGAAAAGTGACAATGTTCCAAGCGAGGAAGT

CGTAAAGAAAATGAAGAACTATTGGCGGCAGCTCCTAAATGCGAAACTGATAACGCAAAGAAAGTTCGATAA

CTTAACTAAAGCTGAGAGGGGTGGCTTGTCTGAACTTGACAAGGCCGGATTTATTAAACGTCAGCTCGTGGAA

ACCCGCCAAATCACAAAGCATGTTGCACAGATACTAGATTCCCGAATGAATACGAAATACGACGAGAACGATA

AGCTGATTCGGGAAGTCAAAGTAATCACTTTAAAGTCAAAATTGGTGTCGGACTTCAGAAAGGATTTTCAATT

CTATAAAGTTAGGGAGATAAATAACTACCACCATGCGCACGACGCTTATCTTAATGCCGTCGTAGGGACCGCA

CTCATTAAGAAATACCCGAAGCTAGAAAGTGAGTTTGTGTATGGTGATTACAAAGTTTATGACGTCCGTAAGA

TGATCGCGAAAGCGAACAGGAGATAGGCAAGGCTACAGCCAAATACTTCTTTTATTCTAACATTATGAATTT

CTTTAAGACGGAAATCACTCTGGCAAACGGAGAGATACGCAAACGACCTTTAATTGAAACCAATGGGGAGAC

AGGTGAAATCGTATGGGATAAGGGCCGGGACTTCGCGACGGTGAGAAAAGTTTTGTCCATGCCCCAAGTCAA

CATAGTAAAGAAAACTGAGGTGCAGACCGGAGGGTTTTCAAAGGAATCGATTCTTCCAAAAAGGAATAGTGA

TAAGCTCATCGCTCGTAAAAAGGACTGGGACCCGAAAAAGTACGGTGGCTTCGATAGCCCTACAGTTGCCTAT

TCTGTCCTAGTAGTGGCAAAAGTTGAGAAGGGAAAATCCAAGAAACTGAAGTCAGTCAAAGAATTATTGGGG

ATAACGATTATGGAGCGCTCGTCTTTTGAAAAGAACCCCATCGACTTCCTTGAGGCGAAAGGTTACAAGGAAG

TAAAAAAGGATCTCATAATTAAACTACCAAAGTATAGTCTGTTTGAGTTAGAAAATGGCCGAAAACGGATGTT

GGCTAGCGCCGGAGAGCTTCAAAAGGGGAACGAACTCGCACTACCGTCTAAATACGTGAATTTCCTGTATTTA

GCGTCCCATTACGAGAAGTTGAAAGGTTCACCTGAAGATAACGAACAGAAGCAACTTTTTGTTGAGCAGCACA

AACATTATCTCGACGAAATCATAGAGCAAATTTCGGAATTCAGTAAGAGAGTCATCCTAGCTGATGCCAATCT

GGACAAAGTATTAAGCGCATACAACAAGCACAGGGATAAACCCATACGTGAGCAGGCGGAAAATATTATCCA

TTTGTTTACTCTTACCAACCTCGGCGCTCCAGCCGCATTCAAGTATTTTGACACAACGATAGATCGCAAACGAT

ACACTTCTACCAAGGAGGTGCTAGACGCGACACTGATTCACCAATCCATCACGGGATTATATGAAACTCGGAT

AGATTTGTCACAGCTTGGGGGTGAC

**RNA scaffold expression cassette (*S. pyogenes*), containing a 20-nucleotide programmable sequence, a CRISPR RNA motif, and an MS2 aptamer motif (SEQ ID No. 20):**

$N_{20}$<u>GTTTTAGAGCTAGAAATAGCAAGTTAAAATAAGGCTAGTCCGTTATCAACTTGAAAAAGTGGCACCGAGT CGGTGC</u>**GCGCACATGAGGATCACCCATGTGC***TTTTTTT*

($N_{20}$: programmable sequence; Underlined: CRISPR RNA motif; Bold: MS2 motif; Italic: terminator)

The above RNA scaffold containing one MS2 loop (1xMS2). Shown below is an RNA scaffold containing two MS2 loops (2xMS2), where MS2 scaffolds are underlined (SEQ ID No. 21):

$N_{20}$GTTTTAGAGCTAGAAATAGCAAGTTAAAATAAGGCTAGTCCGTTATCAACTTGAAAAAGTGGCACCGAGT CGGTGCgggagc<u>ACATGAGGATCACCCATGT</u>gccacgagcg<u>ACATGAGGATCACCCATGT</u>cgctcgtgttcccTTTTTTT

**Effector AID-MCP fusion (SEQ ID No. 22):**

MDSLLMNRRKFLYQFKNVRWAKGRRETYLCYVVKRRDSATSFSLDFGYLRNKNGCHVELLFLRYISDWDLDPGRC

YRVTWFTSWSPCYDCARHVADFLRGNPNLSLRIFTARLYFCEDRKAEPEGLRRLHRAGVQIAIMTFKDYFYCWNTF

VENHERTFKAWEGLHENSVRLSRQLRRILLPLYEVDDLRDAFRTLGL<u>ELKTPLGDTTHTSPPCPAPELLGGPMASNF</u>

<u>TQFVLVDNGGTGDVTVAPSNFANGIAEWISSNSRSQAYKVTCSVRQSSAQNRKYTIKVEVPKGAWRSYLNMELTI</u>

<u>PIFATNSDCELIVKAMQGLLKDGNPIPSAIAANSGIY</u>

**($NH_2$)-AID-linker-MCP-(COOH)**

Like the Cas protein described above, the non-nuclease effector can also be obtained as a recombinant polypeptide. Techniques for making recombinant polypeptides are known in the art. See *e.g.*, Creighton, "Proteins: Structures and Molecular Principles," W.H. Freeman & Co., NY, 1983); Ausubel et al., Current Protocols in Molecular Biology, John Wiley & Sons, 2003; and Sambrook et al., Molecular Cloning, A Laboratory Manual," Cold Spring Harbor Press, Cold Spring Harbor, NY, 2001).

As described herein, by mutating Ser38 to Ala in AID one can reduce the recruitment of AID to off-target sites. Listed below are the DNA and protein sequences of both wild type AID as well as AID_S38A (phosphorylation null, pnAID):

**wtAID cDNA (Ser38 codon in bold and underlined, SEQ ID No. 23):**

ATGGACAGCCTCTTGATGAACCGGAGGAAGTTTCTTTACCAATTCAAAAATGTCCGCTGGGCTAAGGGTCGGC
GTGAGACCTACCTGTGCTACGTAGTGAAGAGGCGTGAC**<u>AGT</u>**GCTACATCCTTTTCACTGGACTTTGGTTATCTT
CGCAATAAGAACGGCTGCCACGTGGAATTGCTCTTCCTCCGCTACATCTCGGACTGGGACCTAGACCCTGGCC
GCTGCTACCGCGTCACCTGGTTCACCTCCTGGAGCCCCTGCTACGACTGTGCCCGACATGTGGCCGACTTTCTG
CGAGGGAACCCCAACCTCAGTCTGAGGATCTTCACCGCGCGCCTCTACTTCTGTGAGGACCGCAAGGCTGAGC
CCGAGGGGCTGCGGCGGCTGCACCGCGCCGGGGTGCAAATAGCCATCATGACCTTCAAAGATTATTTTTACTG
CTGGAATACTTTTGTAGAAAACCATGAAAGAACTTTCAAAGCCTGGGAAGGGCTGCATGAAAATTCAGTTCGT
CTCTCCAGACAGCTTCGGCGCATCCTTTTGCCCCTGTATGAGGTTGATGACTTACGAGACGCATTTCGTACTTT
GGGACTT

**wtAID protein (Ser38 in bold and underlined, SEQ ID No. 24):**

MDSLLMNRRKFLYQFKNVRWAKGRRETYLCYVVKRRD**<u>S</u>**ATSFSLDFGYLRNKNGCHVELLFLRYISDWDLDPGRC
YRVTWFTSWSPCYDCARHVADFLRGNPNLSLRIFTARLYFCEDRKAEPEGLRRLHRAGVQIAIMTFKDYFYCWNTF
VENHERTFKAWEGLHENSVRLSRQLRRILLPLYEVDDLRDAFRTLGL

**AID_S38A cDNA (S38A mutation in bold and underlined, SEQ ID No. 25)**

ATGGACAGCCTCTTGATGAACCGGAGGAAGTTTCTTTACCAATTCAAAAATGTCCGCTGGGCTAAGGGTCGGC
GTGAGACCTACCTGTGCTACGTAGTGAAGAGGCGTGAC**<u>GCC</u>**GCTACATCCTTTTCACTGGACTTTGGTTATCTT
CGCAATAAGAACGGCTGCCACGTGGAATTGCTCTTCCTCCGCTACATCTCGGACTGGGACCTAGACCCTGGCC
GCTGCTACCGCGTCACCTGGTTCACCTCCTGGAGCCCCTGCTACGACTGTGCCCGACATGTGGCCGACTTTCTG
CGAGGGAACCCCAACCTCAGTCTGAGGATCTTCACCGCGCGCCTCTACTTCTGTGAGGACCGCAAGGCTGAGC
CCGAGGGGCTGCGGCGGCTGCACCGCGCCGGGGTGCAAATAGCCATCATGACCTTCAAAGATTATTTTTACTG
CTGGAATACTTTTGTAGAAAACCATGAAAGAACTTTCAAAGCCTGGGAAGGGCTGCATGAAAATTCAGTTCGT
CTCTCCAGACAGCTTCGGCGCATCCTTTTGCCCCTGTATGAGGTTGATGACTTACGAGACGCATTTCGTACTTT
GGGACTT

**AID_S38A protein (S38A mutation in bold and underlined, SEQ ID No. 26)**

MDSLLMNRRKFLYQFKNVRWAKGRRETYLCYVVKRRD**<u>A</u>**ATSFSLDFGYLRNKNGCHVELLFLRYISDWDLDPGRC
YRVTWFTSWSPCYDCARHVADFLRGNPNLSLRIFTARLYFCEDRKAEPEGLRRLHRAGVQIAIMTFKDYFYCWNTF
VENHERTFKAWEGLHENSVRLSRQLRRILLPLYEVDDLRDAFRTLGL

**Humanised nucleotide sequence PmCDA1 *(Petromyzon marinus)* (SEQ ID No. 27)**

ATGACCGACGCTGAATACGTCAGGATACACGAAAAGCTCGATATTTACACCTTTAAGAAACAGTTCTTCAATA ATAAGAAGAGTGTTTCACATCGATGCTATGTGCTTTTCGAGCTCAAACGGCGAGGAGAAAGGAGAGCATGTT TTTGGGGCTACGCTGTAAACAAACCCCAATCTGGCACAGAGCGGGGCATTCACGCCGAAATTTTTTCCATCAG GAAAGTCGAGGAATACTTGCGCGATAACCCCGGTCAATTTACAATCAACTGGTATTCTTCATGGTCCCCCTGCG CTGACTGCGCCGAAAAGATCTTGGAATGGTATAACCAGGAACTTAGAGGTAACGGTCACACTCTCAAAATATG GGCCTGCAAACTTTACTACGAGAAGAATGCTCGAAACCAAATAGGCCTGTGGAATCTTCGCGATAACGGGGT GGGACTTAATGTCATGGTAAGCGAACACTACCAATGCTGCCGCAAGATTTTTATACAGTCTAGTCATAACCAG CTTAACGAGAACCGCTGGCTTGAAAAGACGCTCAAACGGGCGGAAAAAAGGAGGAGTGAGCTCAGTATAAT GATTCAAGTGAAAATACTGCACGACTAAGTCACCAGCAGTA

**Amino acid sequence PmCDA1 (SEQ ID No. 28)**

MTDAEYVRIHEKLDIYFKKQFFNNKKSVSHRCYVLFELKRRGERRACFWGYAVNKPQSGTERGIHAEIFSIRKVEEY LRDNPGQFTINWYSSWSPCADCAEKILEWYNQELRGNGHTLKIWACKLYYEKNARNQIGLWNLRDNGVGLNVM VSEHYQCCRKIFIQSSHNQLNENRWLEKTLKRAEKRRSELSIMIQVKILHTTKSPAV

**PmCDA1-linker-MCP (SEQ ID No. 29)**

MTDAEYVRIHEKLDIYFKKQFFNNKKSVSHRCYVLFELKRRGERRACFWGYAVNKPQSGTERGIHAEIFSIRKVEEY LRDNPGQFTINWYSSWSPCADCAEKILEWYNQELRGNGHTLKIWACKLYYEKNARNQIGLWNLRDNGVGLNVM VSEHYQCCRKIFIQSSHNQLNENRWLEKTLKRAEKRRSELSIMIQVKILHTTKSPAV<u>ELKTPLGDTTHTSPPCPAPELL</u> <u>GGPMASNFTQFVLVDNGGTGDVTVAPSNFANGIAEWISSNSRSQAYKVTCSVRQSSAQNRKYTIKVEVPKGAWR</u> <u>SYLNMELTIPIFATNSDCELIVKAMQGLLKDGNPIPSAIAANSGIY</u>

Key: PmCDA1-<u>linker</u>-<u>MCP</u>

Shown below are a number of non-limiting exemplary RNA sequence of guide RNA constructs that can be used with a Type II Cas protein as outlined above. Each contains, from the 3' end to the 5' end, a customisable target, a gRNA scaffold, and one or two copies of a MS2 aptamer.

**Sequence of gRNA_MS2 construct (SEQ ID No. 30):**

<u>NNNNNNNNNNNNNNNNNNNNN</u>GUUUUAGAGCUAUGCUGUUGAAAAACAGCAUAGCAAGUUAAAAUAAGGCUAGU CCGUUAUCAACUUGAAAAAGUGGCACCGAGUCGGUGCgcgc<u>ACAUGAGGAUCACCCAUGU</u>gc

Key: <u>Customizable target</u>-gRNA scaffold-<u>MS2 aptamer</u>

**Sequence of gRNA_2xMS2 construct (SEQ ID No. 31):**

<u>NNNNNNNNNNNNNNNNNNNNN</u>GUUUUAGAGCUAGAAAUAGCAAGUUAAAAUAAGGCUAGUCCGUUAUC AACUUGAAAAAGUGGCACCGAGUCGGUGCgggagc<u>ACAUGAGGAUCACCCAUGU</u>gccacgagcg<u>ACAUGAGG</u> <u>AUCACCCAUGU</u>

Key: <u>Customizable target-gRNA scaffold-MS2 aptamers</u>

The above three components of the platform/system disclosed herein can be expressed using one, two or three expression vectors. The system can be programmed to target virtually any DNA or RNA sequence. In addition to the second generation base editors described above, similar second generation base editors could be generated by varying the modular components of the system, including any suitable Cas orthologs, deaminase orthologs, and other DNA modification enzymes.

## BINDING AFFINITY

The most widely used value in the literature for RNA-protein binding affinity is the dissociation constant at equilibrium ($K_d$). It is a ratio defined as:

$$K_d = \frac{k_{off}}{k_{on}}$$

$K_d$ is the equilibrium dissociation constant. This constant relflects the stability of the RNA-protein complex (e.g. RNA.Protein). The stability of a temporary complex can be measured using $K_d$.

$$k_{on}$$

$$RNA + Protein \rightleftharpoons RNA.\,Protein\ Complex$$

$$k_{off}$$

$k_{off}$ is the dissociation rate constant. This constant allows the calculation of time during which the temporary RNA-protein complex remains undissociated. Complex dissociation is a first-order process, and so the half-life time of dissociation ($t_{1/2}$) depends only on $k_{off}$:

$$t_{\frac{1}{2}} = \frac{0.693}{k_{off}}$$

$k_{on}$ is the association rate constant. This constant allows the calculation of the delay time of inhibition i.e. the time required for complex formation.

In an embodiment, a ligand binding moiety and/or ligand with a binding affinity with a dissociation constant ($K_D$) of less than 100 nM will be selected. In a preferred embodiment an RNA motif and/or RNA binding domain with a dissociation constant of between 10-100 nM will be selected. In a preferred embodiment, a ligand binding moiety and/or ligand with a dissociation constant of between 1-10 nM will be selected. In the most preferred embodiment, a ligand binding moiety and/or ligand with a dissociation constant of less than 1 nM will be selected. Low binding affinity of the a ligand binding moiety and/or ligand will have a dissociation constant of more than 100 nM, and is not suited for this system

In an embodiment, an RNA motif and/or the RNA binding domain with a binding affinity with a dissociation constant ($K_D$) of less than 150 nM, less than 140 nM, less than 130 nM, less than 120 nM, less than 110 nM or less than 100 nM will be selected. In a preferred embodiment an RNA motif and/or RNA binding domain with a dissociation constant of between 10-150 nM will be selected. In a preferred embodiment, an RNA motif and/or the RNA binding domain with a dissociation constant of between 1-10 nM will be selected. In one embodiment, an RNA motif and/or the RNA binding domain with a dissociation constant of less than 1 nM will be selected. Low binding affinity of the RNA motif and/or the RNA binding domain will have a dissociation constant of more than 150 nM, and is not suited for this system.

Methods to measure the dissociation constant at equilibrium are well known to a person skilled in the art. Preferentially, surface plasmon resonance should be used, details of such methods are available in Jing, M. & Bowser, M. T. (2011) Methods for measuring aptamer-protein equilibria: A review. Analytica Chimica Acta. 686(1-2), 9-18.

## CELL TYPES AND THERAPEUTIC USES

Immune cells, particularly primary immune cells, either naturally occurring within a host animal or patient, or derived from an induced pluripotent stem cell (iPSC) may be genetically modified using the methods and system provided herein. Immune cells include T cells, Natural Killer (NK) cells, B cells, myeloblasts, erythroblasts and pluripotent cells that are immune cell precursors, such as haematopoietic stem cells (HSCs) which can differentiate into all blood and immune cells. Haemopoietic stem cells (HSCs) arise from hemangioblasts, which can give rise to HSCs, vascular smooth muscle cells and angioblasts, which differentiate into vascular endothelial cells. HSCs can give rise to common myeloid and common lymphoid progenitors from which arise T cells, Natural Killer (NK) cells, B cells, myeloblasts, erythroblasts and other cells involved in the production of cells of blood, bone marrow, spleen, lymph nodes, and thymus.

Provided herein are also methods for genome engineering (e.g., for altering or manipulating the expression of one or more genes or one or more gene products) in prokaryotic or eukaryotic cells, in vitro, in vivo, or ex vivo. In particular, the methods provided herein are useful for targeted base editing disruption in mammalian cells including primary human T cells, natural killer (NK) cells, CD34+ hematopoietic stem and progenitor cells (HSPCs), such as HSPCs isolated from umbilical cord blood or bone marrow and cells differentiated from them.

Also provided herein are genetically engineered cells arising from haematopoietic stem cells, such as T cells, that have been modified according to the methods described herein.

In some cases, the methods are configured to produce genetically engineered T cells arising from HSCs or iPSCs that are suitable as "universally acceptable" cells for therapeutic application. Such methods can also be applied to natural killer (NK) cells, CD34+ hematopoietic stem and progenitor cells (HSPCs), such as HSPCs isolated from umbilical cord blood or bone marrow and cells differentiated from them.

In another aspect, provided herein are methods for targeting diseases for base editing correction. The target sequence can be any disease-associated polynucleotide or gene, as have been established in the art. Examples of useful applications of mutation or 'correction' of an endogenous gene sequence include alterations of disease-associated gene mutations, alterations in sequences encoding splice sites, alterations in regulatory sequences, alterations in sequences to cause a gain-of-function mutation, and/or alterations in sequences to cause a loss-of-function mutation, and targeted alterations of sequences encoding structural characteristics of a protein.

In some cases, it will be advantageous to genetically modify a cell using the methods described herein such that cell expresses a chimeric antigen receptor (CAR) and/or T cell receptor (TCR). The "chimeric antigen receptor (CAR)" is sometimes called a "chimeric receptor", a "T-body", or a "chimeric immune receptor (CIR)." As used herein, the term "chimeric antigen receptor (CAR)" refers to an artificially constructed hybrid protein or polypeptide comprising an extracellular antigen binding domains of an antibody (e.g., single chain variable fragment (scFv)) operably linked to a transmembrane domain and at least one intracellular domain. Generally, the antigen binding domain of a CAR has specificity for a particular antigen expressed on the surface of a target cell of interest. For example, T cells can be engineered to express CAR specific for CD19 on B-cell lymphoma. For allogenic antitumor cell therapeutics not limited by donor-matching, cells can be engineered to knock-in nucleic acids encoding a CAR but also knocking out genes responsible for donor matching (TCR and HLA markers).

As used herein, the terms "genetically modified" and "genetically engineered" are used interchangeably and refer to a prokaryotic or eukaryotic cell that includes an exogenous polynucleotide, regardless of the method used for insertion. In some cases, the effector cell has been modified to comprise a non-naturally occurring nucleic acid molecule that has been created or modified by the hand of man (e.g., using recombinant DNA technology) or is derived from such a molecule (e.g., by transcription, translation, etc.). An effector cell that contains an exogenous, recombinant, synthetic, and/or otherwise modified polynucleotide is considered to be an engineered cell.

In another aspect, this disclosure provides a host cell or cell line or progeny thereof comprising the system described above. In another aspect, a cell line can be derived from at least one cell prepared according to the methods provided herein. Methods for preparing a cell line are well known in the art.

### Cell Therapies and Ex Vivo Therapies

Various embodiments of the present invention also provide cells that are produced or used in accordance with any of the other embodiments of the present invention for use in therapy. In one embodiment, the present invention is directed to methods for generating therapeutic cells such as T cells engineered to express a Chimeric Antigen Receptor (CAR-T) or T Cell Receptor (TCR-T). The CAR-T/TCR-T cells may be derived from primary T cells or differentiated from stem cells. Suitable stem cells include, but are not limited to, mammalian stem cells such as human stem cells, including, but not limited to, hematopoietic, neural, embryonic, induced pluripotent stem cells (iPSC), mesenchymal, mesodermal, liver, pancreatic, muscle, and retinal stem cells. Other stems cells include, but are not limited to, mammalian stem cells such as mouse stem cells, e.g., mouse embryonic stem cells.

In various embodiments, the present invention may be used to knockout, modify or increase the expression of a single gene or multiple genes in various types of cells or cell lines, including but not limited to cells from mammals. The present systems and methods may be applicable to multiplex genetic modification, which, as in known in the art, involves genetically modifying multiple genes or multiple targets within the same gene. The technology may be used for many applications, including but not limited to knock out of genes to prevent graft versus host disease by making non-host cells non-immunogenic to the host or prevent host vs graft disease by making non-host cells resistant to attack by the host. These approaches are also relevant to generating allogenic (off-the-shelf) or autologous (patient specific) cell-based therapeutics. Such genes include, but are not limited to, the T Cell Receptor (TRAC, TRBC1. TRBC2, TRDC, TRGC1, TRGC2), the major histocompatibility complex (MHC class I and class II) genes, including B2M, co-receptors (HLA-F, HLA-G), genes involved in the innate immune response (MICA, MICB, HCPS, STING, DDX41 and Toll-like-receptors (TLRs)), inflammation (NKBBiL, LTA, TNF, LTB, LST1, NCR3, AIF1), heat shock proteins (HSPA1L, HSPA1A, HSPA1B), complement cascade, regulatory receptors (NOTCH family members), antigen processing (TAP, HLA-DM, HLA-DO), increased potency or persistence (such as PD-1, CTLA-4 and other members of the B7 family of checkpoint proteins), genes involved in immunosuppressive immune cells (such as FOXP3 and Interleukin (IL)-10), genes involved in T cell interaction with the tumour microenvironment (including but not limited to receptors of cytokines such as TGFB, IL-4, IL-7, IL-2, IL-15, IL-12, IL-18, IFNgamma), genes involved in contributing to cytokine release syndrome (including but not limited to IL-6, IFNgamma, IL-8 (CXCL8), IL-10, GM-CSF, MIP-1$\alpha$/$\beta$, MCP-1 (CCL2), CXCL9, and CXCL10 (IP-10), genes that code for the antigen targeted by a CAR/TCR (for example endogenous CS1 where the CAR is designed against CS1) or other genes found to be beneficial to CAR-T/TCR-T (such as TET2) or other cell based therapeutics including but not limited to CAR-NK, CAR-B etc. See, e.g., DeRenzo et al., Genetic Modification Strategies to Enhance CAR T Cell Persistence for Patients With Solid Tumors. Front. Immunol., 15 February 2019.

The technology may also be used to knock down or modify genes that are involved in fratricide of immune cells, such as T cells and NK cells, or genes that alert the immune system of a patient or animal that a foreign cell, particle or molecule has entered a patient or animal, or genes encoding proteins that are current therapeutic targets used to compromise or boost an immune response, for example, CD52 and PD1, respectively.

One application of the method and system provided herein is to engineer HLA alleles of bone marrow cells or bone marrow cells differentiated from iPS cells to increase haplotype match. The engineered cells can be used for bone marrow transplantation for treating leukemia. Another application is to engineer the negative regulatory element of fetal hemoglobin gene in hematopoietic stem cells for treating sickle cell anemia and beta-thalassemia. The negative regulatory element will be mutated and the expression of fetal hemoglobin gene is re-activated in hematopoietic stem cells, compensating the functional loss due to mutations in adult alpha or beta hemoglobin genes. A further application is to engineer iPS cells for generating allogenic therapeutic cells for various degenerative diseases including Parkinson's disease (neuronal cell loss), Type 1 diabetes (pancreatic beta cell loss). Other exemplary applications include engineering HIV infection resistant T- Cells by inactivating CCRS gene and other genes encoding receptors required for HIV entering cells; removing a premature stop codon in the DMD gene to re-establish expression of dystrophin; and the correction of cancer driver mutations, such as p53 Y163C.

## TYPES OF GENETIC MODIFICATIONS

Accordingly, provided herein are methods for targeted disruption of transcription or translation of a target gene. In particular, the methods comprise targeted disruption of transcription or translation of a target gene via disruption of a start codon, introduction of a premature stop codon, and/or targeted disruption of intron/exon splice sites.

Using the methods described herein, one may knock-in and/or knock-out one or more genes of interest in primary cells with improved efficiency and a reduced rate of off-target indel formation. In preferred embodiments, the methods are used for multiplexed base editing comprising gene knock-in, gene knock-out, and missense mutation.

As described in the paragraphs and Examples that follow, the inventors' streamlined approach to genome engineering employs base editors for applications including targeted gene disruption by knock-out and missense mutation and gene correction. The methods described herein are well-suited for studying immune cell biology and gene function, modeling diseases such as primary immunodeficiencies, as well as correcting disease-causing point mutations, and generating novel cell products (e.g., T cell products) for therapeutic applications.

Disruptions at splice acceptor-splice donor (SA-SD) sites are also useful because one may knock out coding sequence and non-coding RNAs (ncRNAs) without stop codon read through.

## DELIVERY OF COMPONENTS INTO CELLS

Suitable methods for delivering the base editing components to immune cells such as haemopoietic cells are provided in the examples herein below.

In embodiments provided herein the guide RNA molecule can be delivered to the target cell via various methods, without limitation, listed below. Firstly, direct introduction of synthetic RNA molecules (whether sgRNA, crRNA, or tracrRNA and modifications thereof) to the cell of interest by electroporation, nucleofection, transfection, via nanoparticles, via viral mediated RNA delivery, via non-viral mediated delivery, via extracellular vesicles (for example exosome and microvesicles), via eukaryotic cell transfer (for example by recombinant yeast ) and other methods that can package the RNA molecules and can be delivered to the target viable cell without changes to the genomic landscape. Other methods for the introduction of guide RNA molecules include non-integrative transient transfer of DNA polynucleotides that includes the relevant sequence for the protein recruitment so that the molecule can be transcribed into the target guide RNA molecule, this includes, without limitation, DNA-only vehicles (for example, plasmids, MiniCircles, MiniVectors, MiniStrings, protelomerase generated DNA molecules (for example Doggybones), artificial chromosome (for example HAC), cosmids), via DNA vehicles by nanoparticles, extracellular vesicles (for example exosome and microvesicles), via eukaryotic cell transfer (for example by recombinant yeast), transient viral transfer by AAV, non-integrating viral particles (for example lentivirus and retrovirus based systems), cell penetrating peptides and other technology that can mediate the introduction of DNA into a cell without direct integration into the genomic landscape. Another method for the introduction of the guide RNA include the use of integrative gene transfer technology for stable introduction of the machinery for guide RNA transcription into the genome of the target cells, this can be controls via constitutive or promoter inducible systems to attenuate the guide RNA expression and this can also be designed so that the system can be removed after the utility has been met (for example, introducing a Cre-Lox recombination system), such technology for stable gene transfer includes, but not limited to, integrating viral particles (for example lentivirus, adenovirus and retrovirus based systems), transposase mediate transfer (for example Sleeping Beauty and Piggybac), exploitation of the non-homologous repair pathways introduced by DNA breaks (for example utilising CRISPR and TALEN) technology and a surrogate DNA molecule, and other technology that encourages integration of the target DNA into a cell of interest.

The method for delivering deaminase effector fusion protein and the CRISPR sequencing targeting components are often mediated by the same technology but in some situations, there are advantages to mediate the delivery via different methods. The applicable methods, and not limited to, are listed below. Firstly, the direct introduction of mRNA and Protein molecules directly to the cell of interest by electroporation, nucleofection, transfection, via nanoparticles, via viral mediated packaged delivery, extracellular vesicles (for example exosome and microvesicles), via eukaryotic cell transfer (for example by recombinant yeast), and other methods that can package the macromolecules and can be delivered to the target viable cell without integration into genomic landscape. Other methods for the introduction of molecules include non-integrative transient transfer of DNA polynucleotides that includes the relevant sequence for the protein recruitment so that the molecule or molecules can be transcribed and translated into the target protein molecule, this includes, without limitation, DNA-only vehicles (for example, plasmids, MiniCircles, MiniVectors, MiniStrings, protelomerase generated DNA molecules (for example Doggybones), artificial chromosome (for example HAC), cosmids), via DNA vehicles by nanoparticles, extracellular vesicles (for example exosome and microvesicles), via eukaryotic cell transfer (for example by recombinant yeast), transient viral transfer by AAV, non-integrating viral particles (for example lentivirus and retrovirus based systems), and other technology that can mediate the introduction of DNA into a cell without direct integration into the genomic landscape. Another method for the introduction of the deaminase effector fusion protein and the CRISPR sequencing targeting components include the use of integrative gene transfer technology for stable introduction of the machinery for transcription and translation into the genome of the target cells, this can be controlled via constitutive or inducible promoter systems to attenuate the molecule, or molecules expression, and this can also be designed so that the system can be removed after the utility has been met (for example, introducing a Cre-Lox recombination system), such technology for stable gene transfer includes, but not limited to, integrating viral particles (for example lentivirus, adenovirus and retrovirus based systems), transposase mediate transfer (for example Sleeping Beauty and Piggybac), exploitation of the non-homologous repair pathways introduced by DNA breaks (for example utilising CRISPR and TALEN) technology and a surrogate DNA molecule, and other technology that encourages integration of the target DNA into a cell of interest.

**EXPRESSION SYSTEM**

To use the platform described above, it may be desirable to express one or more of the protein and RNA components from nucleic acids that encode them. This can be performed in a variety of ways. For example, the nucleic acids encoding the RNA scaffold or proteins can be cloned into one or more intermediate vectors for introducing into prokaryotic or eukaryotic cells for replication and/or transcription. Intermediate vectors are typically prokaryotic vectors, e.g., plasmids, or shuttle vectors, or insect vectors, for storage or manipulation of the nucleic acid encoding the RNA scaffold or protein for production of the RNA scaffold or protein. The nucleic acids can also be cloned into one or more expression vectors, for administration to a plant cell, animal cell, preferably a mammalian cell or a human cell, fungal cell, bacterial cell, or protozoan cell. Accordingly, the present invention provides nucleic acids that encode any of the RNA scaffold or proteins mentioned above. Preferably, the nucleic acids are isolated and/or purified.

The present invention also provides recombinant constructs or vectors having sequences encoding one or more of the RNA scaffold or proteins described above. Examples of the constructs include a vector, such as a plasmid or viral vector, into which a nucleic acid sequence of the invention has been inserted, in a forward or reverse orientation. In a preferred embodiment, the construct further includes regulatory sequences, including a promoter, operably linked to the sequence. Large numbers of suitable vectors and promoters are known to those of skill in the art, and are commercially available. Appropriate cloning and expression vectors for use with prokaryotic and eukaryotic hosts are also described in e.g., Sambrook et al. (2001, Molecular Cloning: A Laboratory Manual, Cold Spring Harbor Press).

A vector refers to a nucleic acid molecule capable of transporting another nucleic acid to which it has been linked. The vector can be capable of autonomous replication or integration into a host DNA. Examples of the vector include a plasmid, cosmid, or viral vector. The vector of this invention includes a nucleic acid in a form suitable for expression of the nucleic acid in a host cell. Preferably, the vector includes one or more regulatory sequences operatively linked to the nucleic acid sequence to be expressed. A "regulatory sequence" includes promoters, enhancers, and other expression control elements (e.g., polyadenylation signals). Regulatory sequences include those that direct constitutive expression of a nucleotide sequence, as well as inducible regulatory sequences. The design of the expression vector can depend on such factors as the choice of the host cell to be transformed, transfected, or transduced, the level of expression of RNAs or proteins desired, and the like.

Examples of expression vectors include chromosomal, non-chromosomal and synthetic DNA sequences, bacterial plasmids, phage DNA, baculovirus, yeast plasmids, vectors derived from combinations of plasmids and phage DNA, viral DNA such as vaccinia, adenovirus, fowl pox virus, and pseudorabies. However, any other vector may be used provided it is replicable and viable in the host. The appropriate nucleic acid sequence may be inserted into the vector by a variety of procedures. In general, a nucleic acid sequence encoding one of the RNAs or proteins described above can be inserted into an appropriate restriction endonuclease site(s) by procedures known in the art. Such procedures and related sub-cloning procedures are within the scope of those skilled in the art.

The vector may include appropriate sequences for amplifying expression. In addition, the expression vector preferably contains one or more selectable marker genes to provide a phenotypic trait for selection of transformed host cells such as dihydrofolate reductase or neomycin resistance for eukaryotic cell cultures, or such as tetracycline or ampicillin resistance in E. coli.

The vectors for expressing the RNAs can include RNA Pol III promoters to drive expression of the RNAs, e.g., the HI, U6 or 7SK promoters. These human promoters allow for expression of RNAs in mammalian cells following plasmid transfection. Alternatively, a T7 promoter may be used, e.g., for in vitro transcription, and the RNA can be transcribed in vitro and purified.

The vector containing the appropriate nucleic acid sequences as described above, as well as an appropriate promoter or control sequence, can be employed to transform, transfect, or infect an appropriate host to permit the host to express the RNAs or proteins described above. Examples of suitable expression hosts include bacterial cells (e.g., *E. coli, Streptomyces, Salmonella typhimurium*), fungal cells (yeast), insect cells (e.g., *Drosophila* and *Spodoptera frugiperda* (Sf9)), animal cells (e.g., CHO, COS, and HEK 293), adenoviruses, and plant cells. The selection of an appropriate host is within the scope of those skilled in the art. In some embodiments, the present invention provides methods for producing the above mentioned RNAs or proteins by transforming, transfecting, or infecting a host cell with an expression vector having a nucleotide sequence that encodes one of the RNAs, or polypeptides, or proteins. The host cells are then cultured under a suitable condition, which allows for the expression of the RNAs or proteins.

Any of the procedures known in the art for introducing foreign nucleotide sequences into host cells may be used. Examples include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell.

## CULTURING THE CELLS

The method further comprises maintaining the cell under appropriate conditions such that the guide RNA guides the effector protein to the targeted site in the target sequence, and the effector domain modifies the target sequence.

In general, the cell can be maintained under conditions appropriate for cell growth and/or maintenance. Suitable cell culture conditions are well known in the art and are described, for example, in Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, N.Y., 3rd edition, 2001), Santiago et al. (2008) PNAS 105:5809-5814; Moehle et al. (2007) PNAS 104:3055-3060; Urnov et al. (2005) Nature 435:646-651; and Lombardo et al. (2007) Nat. Biotechnology 25:1298-1306. Those of skill in the art appreciate that methods for culturing cells are known in the art and can and will vary depending on the cell type. Routine optimization may be used, in all cases, to determine the best techniques for a particular cell type.

Cells useful for the methods provided herein can be freshly isolated primary cells or obtained from a frozen aliquot of a primary cell culture. In some cases, cells are electroporated for uptake of gRNAs and the base editing fusion protein. As described in the Examples that follow, electroporation conditions for some assays (e.g., for T cells) can comprise 1600 volts, pulse width of 10 milliseconds, 3 pulses. Following electroporation, electroporated T cells are allowed to recover in a cell culture medium and then cultured in a T cell expansion medium. In some cases, electroporated cells are allowed to recover in the cell culture medium for about 5 to about 30 minutes (e.g., about 5, 10, 15, 20, 25, 30 minutes). Preferably, the recovery cell culture medium is free of an antibiotic or other selection agent. In some cases, the T cell expansion medium is Immunocult XT T-cell Expansion medium.

### KITS

Another aspect of the technology described herein relates to kits for use to generate genetically modified cells. Described herein are kit components that can be included in one or more of the kits.

In some embodiments, the components described herein can be provided singularly or in any combination as a kit. The kit includes the components described herein, e.g., a sequence targeting protein, an RNA-ligand binding complex (or RNA scaffold), and two or more effector proteins.

In some embodiments the components of the kit can be provided as mRNA, plasmids, ribonucleoproteins (RNPs) or a combination of two or more of these formats. For example, in one embodiment the sequence targeting component and effector proteins may be provided as separate mRNA molecules, while the one-part synthetic sgRNA and associated RNA motif (RNA scaffold) is provided as RNA. Alternatively, in another embodiment the sequence targeting component and effector proteins may be provided as separate mRNA molecules, while the RNA scaffold is provided as one or more separate components, able to hybridise to form a single RNA-ligand binding complex. In another embodiment the sequence targeting component and effector proteins may be provided as separate RNPs, while the one-part synthetic sgRNA and associated RNA motif (RNA scaffold) is provided as RNA. Alternatively, in another embodiment the sequence targeting component and effector proteins may be provided as separate RNPs, while the RNA scaffold is provided as one or more separate components, able to hybridise to form a single RNA-ligand binding complex.

In some embodiment, the Cas protein may be provided using a stably expressing cell line.

In some embodiments, the compound in the kit can be provided in a watertight or gas tight container which in some embodiments is substantially free of other components of the kit. In some embodiments, the components can be provided in a single container. It is preferred that the component(s) described herein are substantially pure and/or sterile.

The kit will typically be provided with its various elements included in one package, e.g., a fiber-based, e.g., a cardboard, or polymeric, e.g., a Styrofoam box. The enclosure can be configured so as to maintain a temperature differential between the interior and the exterior, e.g., it can provide insulating properties to keep the reagents at a preselected temperature for a preselected time.

### DEFINITIONS

A nucleic acid or polynucleotide refers to a DNA molecule (for example, but not limited to, a cDNA or genomic DNA) or an RNA molecule (for example, but not limited to, an mRNA), and includes DNA or RNA analogs. A DNA or RNA analog can be synthesized from nucleotide analogs. The DNA or RNA molecules may include portions that are not naturally occurring, such as modified bases, modified backbone, deoxyribonucleotides in an RNA, etc. The nucleic acid molecule can be single-stranded or double-stranded.

The term "isolated" when referring to nucleic acid molecules or polypeptides means that the nucleic acid molecule or the polypeptide is substantially free from at least one other component with which it is associated or found together in nature.

As used herein, the term "guide RNA" generally refers to an RNA molecule (or a group of RNA molecules collectively) that can bind to a CRISPR protein and target the CRISPR protein to a specific location within a target DNA. A guide RNA can comprise two segments: a DNA-targeting guide segment and a protein-binding segment. The DNA-targeting segment comprises a nucleotide sequence that is complementary to (or at least can hybridise to under stringent conditions) a target sequence. The protein-binding segment interacts with a CRISPR protein, such as a Cas9 or Cas9 related polypeptide, or Cas12 or Cas12 related polypeptide. These two segments can be located in the same RNA molecule or in two or more separate RNA molecules. The components comprising the guide RNA will be suitably selected and/or designed based on the sequence targeting protein with which the guide RNA is intended to be used. For example, if the sequence targeting protein is a Cas9 or Cas9 related polypeptide, the guide RNA will comprise a crRNA and a tracrRNA. When the sequence targeting protein is from a Type V CRISPR-Cas system, the guide RNA may be a cr-only guide RNA, crRNA:tracrRNA guide RNA or crRNA:scoutRNA guide RNA.

As used herein, the term "target nucleic acid" or "target" refers to a nucleic acid containing a target nucleic acid sequence. A target nucleic acid may be single-stranded or double-stranded, and often is double-stranded DNA. A "target nucleic acid sequence," "target sequence" or "target region," as used herein, means a specific sequence or the complement thereof that one wishes to bind to or modify using a CRISPR system. A target sequence may be within a nucleic acid in vitro or in vivo within the genome of a cell, which may be any form of single-stranded or double-stranded nucleic acid.

A "target nucleic acid strand" refers to a strand of a target nucleic acid that is subject to base-pairing with a guide RNA as disclosed herein. That is, the strand of a target nucleic acid that hybridizes with the crRNA and targeting sequence is referred to as the "target nucleic acid strand." The other strand of the target nucleic acid, which is not complementary to the targeting sequence, is referred to as the "non-complementary strand." In the case of double-stranded target nucleic acid (e.g., DNA), each strand can be a "target nucleic acid strand" to design crRNA and guide RNAs and used to practice the method of this invention as long as there is a suitable PAM site. It should be noted, some Type V Cas proteins are capable of base-pairing with a target nucleic acid strand, without requiring a PAM site.

As used herein, the term "RNA scaffold" refers to an RNA ligand binding complex, wherein the ligand binding moiety is an RNA motif (or RNA aptamer).

As used herein, the term "derived from" refers to a process whereby a first component (e.g., a first molecule), or information from that first component, is used to isolate, derive or make a different second component (e.g., a second molecule that is different from the first). For example, the mammalian codon-optimized Cas9 polynucleotides are derived from the wild type Cas9 protein amino acid sequence. Also, the variant mammalian codon-optimized Cas9 polynucleotides, including the Cas9 single mutant nickase (nCas9, such as nCas9D10A) and Cas9 double mutant null-nuclease (dCas9, such as dCas9 D10A H840A), are derived from the polynucleotide encoding the wild type mammalian codon-optimized Cas9 protein.

As used herein the term "wild type" is a term of the art understood by skilled persons and means the typical form of an organism, strain, gene or characteristic as it occurs in nature as distinguished from mutant or variant forms.

As used herein, the term "variant" refers to a first composition (e.g., a first molecule), that is related to a second composition (e.g., a second molecule, also termed a "parent" molecule). The variant molecule can be derived from, isolated from, based on or homologous to the parent molecule. For example, the mutant forms of mammalian codon-optimized Cas9 (hspCas9), including the Cas9 single mutant nickase and the Cas9 double mutant null-nuclease, are variants of the mammalian codon-optimized wild type Cas9 (hspCas9). The term variant can be used to describe either polynucleotides or polypeptides.

As applied to polynucleotides, a variant molecule can have entire nucleotide sequence identity with the original parent molecule, or alternatively, can have less than 100% nucleotide sequence identity with the parent molecule. For example, a variant of a gene nucleotide sequence can be a second nucleotide sequence that is at least 50%, 60%, 70%, 80%, 90%, 95%, 98%, 99% or more identical in nucleotide sequence compare to the original nucleotide sequence. Polynucleotide variants also include polynucleotides comprising the entire parent polynucleotide, and further comprising additional fused nucleotide sequences. Polynucleotide variants also includes polynucleotides that are portions or subsequences of the parent polynucleotide, for example, unique subsequences (e.g., as determined by standard sequence comparison and alignment techniques) of the polynucleotides disclosed herein are also encompassed by the invention.

In another aspect, polynucleotide variants include nucleotide sequences that contain minor, trivial or inconsequential changes to the parent nucleotide sequence. For example, minor, trivial or inconsequential changes include changes to nucleotide sequence that (i) do not change the amino acid sequence of the corresponding polypeptide, (ii) occur outside the protein-coding open reading frame of a polynucleotide, (iii) result in deletions or insertions that may impact the corresponding amino acid sequence, but have little or no impact on the biological activity of the polypeptide, (iv) the nucleotide changes result in the substitution of an amino acid with a chemically similar amino acid. In the case where a polynucleotide does not encode for a protein (for example, a tRNA or a crRNA or a tracrRNA), variants of that polynucleotide can include nucleotide changes that do not result in loss of function of the polynucleotide. In another aspect, conservative variants of the disclosed nucleotide sequences that yield functionally identical nucleotide sequences are encompassed by the invention. One of skill will appreciate that many variants of the disclosed nucleotide sequences are encompassed by the invention.

As applied to proteins, a variant polypeptide can have entire amino acid sequence identity with the original parent polypeptide, or alternatively, can have less than 100% amino acid identity with the parent protein. For example, a variant of an amino acid sequence can be a second amino acid sequence that is at least 50%, 60%, 70%, 80%, 90%, 95%, 96%, 97%, 98%, 99% or more identical in amino acid sequence compared to the original amino acid sequence.

Polypeptide variants include polypeptides comprising the entire parent polypeptide, and further comprising additional fused amino acid sequences. Polypeptide variants also includes polypeptides that are portions or subsequences of the parent polypeptide, for example, unique subsequences (e.g., as determined by standard sequence comparison and alignment techniques) of the polypeptides disclosed herein are also encompassed by the invention.

In another aspect, polypeptide variants include polypeptides that contain minor, trivial or inconsequential changes to the parent amino acid sequence. For example, minor, trivial or inconsequential changes include amino acid changes (including substitutions, deletions and insertions) that have little or no impact on the biological activity of the polypeptide, and yield functionally identical polypeptides, including additions of non-functional peptide sequence. In other aspects, the variant polypeptides of the invention change the biological activity of the parent molecule, for example, mutant variants of the Cas9 polypeptide that have modified or lost nuclease activity. One of skill will appreciate that many variants of the disclosed polypeptides are encompassed by the invention.

In some aspects, polynucleotide or polypeptide variants of the invention can include variant molecules that alter, add or delete a small percentage of the nucleotide or amino acid positions, for example, typically less than about 10%, less than about 5%, less than 4%, less than 2% or less than 1%.

As used herein, the term "conservative substitutions" in a nucleotide or amino acid sequence refers to changes in the nucleotide sequence that either (i) do not result in any corresponding change in the amino acid sequence due to the redundancy of the triplet codon code, or (ii) result in a substitution of the original parent amino acid with an amino acid having a chemically similar structure. Conservative substitution tables providing functionally similar amino acids are well known in the art, where one amino acid residue is substituted for another amino acid residue having similar chemical properties (e.g., aromatic side chains or positively charged side chains), and therefore does not substantially change the functional properties of the resulting polypeptide molecule.

The following are groupings of natural amino acids that contain similar chemical properties, where a substitution within a group is a "conservative" amino acid substitution. This grouping indicated below is not rigid, as these natural amino acids can be placed in different grouping when different functional properties are considered. Amino acids having nonpolar and/or aliphatic side chains include: glycine, alanine, valine, leucine, isoleucine and proline. Amino acids having polar, uncharged side chains include: serine, threonine, cysteine, methionine, asparagine and glutamine. Amino acids having aromatic side chains include: phenylalanine, tyrosine and tryptophan. Amino acids having positively charged side chains include: lysine, arginine and histidine. Amino acids having negatively charged side chains include: aspartate and glutamate.

A "Cas9 mutant" or "Cas9 variant" refers to a protein or polypeptide derivative of the wild type Cas9 protein such as *S. pyogenes* Cas9 protein (SEQ ID No. 44), *e.g.,* a protein having one or more point mutations, insertions, deletions, truncations, a fusion protein, or a combination thereof. It retains substantially the RNA targeting activity of the Cas9 protein. The protein or polypeptide can comprise, consist of, or consist essentially of a fragment of SEQ ID No. 44. In general, the mutant/variant is at least 50% (e.g., any number between 50% and 100%, inclusive) identical to SEQ ID No. 44. The mutant/variant can bind to an RNA molecule and be targeted to a specific DNA sequence via the RNA molecule, and may additional have a nuclease activity. Examples of these domains include RuvC like motifs (aa. 7-22, 759-766 and 982-989 in SEQ ID No. 34) and HNH motif (aa 837-863). See Gasiunas et al., Proc Natl Acad Sci USA. 2012 September 25; 109(39): E2579-E2586 and WO2013176772.

A "Cas12 mutant" or "Cas12 variant" refers to a protein or polypeptide derivative of the wild type Cas protein from a Type V CRISPR-Cas system such as *Alicylobacillus acidiphilus* Cas12b (SEQ ID No. 49), *e.g.,* a protein having one or more point mutations, insertions, deletions, truncations, a fusion protein, or a combination thereof. A "Cas12 mutant" or "Cas12 variant" retains substantially the RNA targeting activity of the Cas12 protein from which it is derived. By way of non-limiting example, the protein or polypeptide can comprise, consist of, or consist essentially of a fragment of SEQ ID No. 48, 49 or 50. The mutant/variant can bind to an RNA molecule and be targeted to a specific DNA sequence via the RNA molecule, and may additional have a nuclease activity.

"Complementarity" refers to the ability of a nucleic acid to form hydrogen bond(s) with another nucleic acid sequence by either traditional Watson-Crick base-pairing or other non-traditional types. A percent complementarity indicates the percentage of residues in a nucleic acid molecule which can form hydrogen bonds (*e.g.*, Watson-Crick base pairing) with a second nucleic acid sequence (*e.g.*, 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques in Biochemistry and Molecular Biology-Hybridization with Nucleic Acid Probes Part I, Second Chapter "Overview of principles of hybridization and the strategy of nucleic acid probe assay", Elsevier, N.Y.

"Hybridisation" or "hybridising" refers to a process where completely or partially complementary nucleic acid strands come together under specified hybridisation conditions to form a double-stranded structure or region in which the two constituent strands are joined by hydrogen bonds. Although hydrogen bonds typically form between adenine and thymine or uracil (A and T or U) or cytidine and guanine (C and G), other base pairs may form (*e.g.*, Adams et al., The Biochemistry of the Nucleic Acids, 11th ed., 1992).

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, pegylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

The term "fusion polypeptide" or "fusion protein" means a protein created by joining two or more polypeptide sequences together. The fusion polypeptides encompassed in this invention include translation products of a chimeric gene construct that joins the nucleic acid sequences encoding a first polypeptide, *e.g.*, an RNA-binding domain, with the nucleic acid sequence encoding a second polypeptide, *e.g.*, an effector domain, to form a single open-reading frame. In other words, a "fusion polypeptide" or "fusion protein" is a recombinant protein of two or more proteins which are joined by a peptide bond or via several peptides. The fusion protein may also comprise a peptide linker between the two domains.

The term "linker" refers to any means, entity or moiety used to join two or more entities. A linker can be a covalent linker or a non-covalent linker. Examples of covalent linkers include covalent bonds or a linker moiety covalently attached to one or more of the proteins or domains to be linked. The linker can also be a non-covalent bond, *e.g.*, an organometallic bond through a metal center such as platinum atom. For covalent linkages, various functionalities can be used, such as amide groups, including carbonic acid derivatives, ethers, esters, including organic and inorganic esters, amino, urethane, urea and the like. To provide for linking, the domains can be modified by oxidation, hydroxylation, substitution, reduction etc. to provide a site for coupling. Methods for conjugation are well known by persons skilled in the art and are encompassed for use in the present invention. Linker moieties include, but are not limited to, chemical linker moieties, or for example a peptide linker moiety (a linker sequence). It will be appreciated that modification which do not significantly decrease the function of the RNA-binding domain and effector domain are preferred.

As used herein, the term "conjugate" or "conjugation" or "linked" as used herein refers to the attachment of two or more entities to form one entity. A conjugate encompasses both peptide-small molecule conjugates as well as peptide-protein/peptide conjugates.

The terms "subject" and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained *in vivo* or cultured *in vitro* are also encompassed. In some embodiments, a subject may be an invertebrate animal, for example, an insect or a nematode; while in others, a subject may be a plant or a fungus.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

As used herein, the term "contacting," when used in reference to any set of components, includes any process whereby the components to be contacted are mixed into same mixture (for example, are added into the same compartment or solution), and does not necessarily require actual physical contact between the recited components. The recited components can be contacted in any order or any combination (or sub-combination) and can include situations where one or some of the recited components are subsequently removed from the mixture, optionally prior to addition of other recited components. For example, "contacting A with B and C" includes any and all of the following situations: (i) A is mixed with C, then B is added to the mixture; (ii) A and B are mixed into a mixture; B is removed from the mixture, and then C is added to the mixture; and (iii) A is added to a mixture of B and C. "Contacting" a target nucleic acid or a cell with one or more reaction components, such as an Cas protein or guide RNA, includes any or all of the following situations: (i) the target or cell is contacted with a first component of a reaction mixture to create a mixture; then other components of the reaction mixture are added in any order or combination to the mixture; and (ii) the reaction mixture is fully formed prior to mixture with the target or cell.

The term "mixture" as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not spatially distinct. In other words, a mixture is not addressable.

As disclosed herein, a number of ranges of values are provided. It is understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included in the invention. The term "about" generally refers to plus or minus 10% of the indicated number. For example, "about 10%" may indicate a range of 9% to 11%, and "about 20" may mean from 18-22. Other meanings of "about" may be apparent from the context, such as rounding off, so, for example "about 1" may also mean from 0.5 to 1.4.

Various exemplary embodiments of compositions and methods according to this invention are now described by way of the following examples.

## EXAMPLES

In the following examples, eukaryotic cells were used to prove that multiple effectors can be actively recruited to the same genomic locus using multiple strategies to give novel functionality with surprising efficiency and outcomes. The suprising observation is that a single aptamer can transiently recruit effectors when presented with a pool of effectors allowing a bound CRISPR-Cas system to provide multifunctional activity at a single genetic locus.

Examples 1 and 8 are non-limiting examples. Examples 2-7 are prophetic examples.

**Example 1: Locus directed multi-effector via a single aptamer recruitment system for novel base editing applications in human cells**

In this example, primary human lymphocytes were used to prove that multiple enzymes can be actively recruited to the same locus using a single aptamer recruitment system. Multiple deaminases (Apobec1 and AID) are recruited to the site via a single aptamer (RNA motif) on the sgRNA.

The Pan T cells were activated utilising anti-CD3 and anti-CD28 and then cells were electroporated with mRNA components for either Apobec1-MCP, AID-MCP or both deaminases, with nCas9-UGI-UGI components, tracrRNA-MS2 aptamer and the crRNA. The cells were then incubated for a further 24 hours, and then stimulated for 48 hours with anti-CD3 and anti-CD28 and then base editing was checked by targeted PCR amplification and Sanger sequencing.

Material and Methods

Guides were designed for specific base editing windows calculated at set distances from the PAM motif (NGG). The crRNAs and tracrRNA were synthesized by Horizon Discovery (formerly Dharmacon).

**Synthetic crRNA Sequence (SEQ ID No. 32):**
mC*mA*CCTACCTAAGAACCATCCGUUUUAGAGCUAUGCUGUUUUG
2'OMe (m) and phosphorothioate (*) modified residues
**Synthetic tracrRNA-MS2 Aptamer Sequence (SEQ ID No. 33):**

AACAGCAUAGCAAGUUAAAAUAAGGCUAGUCCGUUAUCAACUUGAAAAAGUGGCACCGAGUCG

GUGCGCGCACAUGAGGAUCACCCAUGUGCUUUUmU*mU*U

2'OMe (m) and phosphorothioate (*) modified residues

mRNA Component Generation: Messenger RNA molecules were custom generated by Trilink utilising modified nucleotides: Pseudouridine and 5-Methyl-Cytosine. The mRNA components translated to the following proteins: Deaminase AID-MCP = NLS-hAID-Linker-MCP, Deaminase Apobec1-MCP = NLS-rApobec1-Linker-MCP, and Cas9-UGI-UGI = NLS-nCas9-UGI-UGI-NLS

T Cells Culturing: PBMCs were isolated from fresh blood sources using Lymphoprep (STEMCELL Technologies) gradient centrifugation and then Total T Cells were isolated via negative selection (STEMCELL Technologies). T cells were cultured into Immunocult XT media (STEMCELL Technologies) with 1x Penicillin/Streptomycin (Thermofisher) at 37C and 5% CO2.

T Cell Electroporation: After 48-72 post-activation T cells were electroporated with using the Neon Electroporator (Thermofisher) or 4D Nucleofector (Lonza). Neon Electroporator conditions were 1600v/10ms/3 pulses with a 10ul tip with 250k cells, combined total of mRNA amount of 1-5ug, for both the Deaminase-MCP and nCas9-UGI-UGI, and where applicable 0.2-1.8 umol of complexed crRNA:tracrR or sgRNA. 4D Nucleofector conditions were EO-115 with a 20ul cuvette with 500k combined total of mRNA amount of 1-5ug, for both single or double Deaminase-MCP and nCas9-UGI-UGI (synthesised by Trilink), and 0.2-1.8 umol of complexed crRNA:tracrR or sgRNA (Horizon Discovery). Post-electroporation cells were transferred to Immunocult XT media with 100U IL-2, 100U IL-7 and 100U IL-15 (STEMCELL Technologies) and cultured at 37C and 5% CO2 for 24-72 hours.

CD3+ T Cell Activation: T cells were activated by using 1:1 bead:cell ratio of Dynabeads Human T Activator CD3/CD28 beads (Thermofisher) cultured in Immunocult XT media (STEMCELL Technologies) in the presence of 100U/ml IL-2 (STEMCELL Technologies) and 1x Penicillin/Streptomycin (Thermofisher) at 37C and 5% CO2 for 48 hours. Post-activation, beads were removed by placement on a magnet and the transfer of the cells back into culture.

Genomic DNA Analysis: Genomic DNA was released from lysed cells 48-72 hours post-electroporation. Loci of interest were amplified by PCR and products then sent for Sanger sequencing (Genewiz). Data was analysed by proprietary in-house software.

The data prove that multiple enzymes can be recruited via the same aptamer (RNA motif) on the same guide in addition to the targeted enzymatic activity (Nickase) of the guidance protein. Figure 7 demonstrates that by including both enzymes there is a complementary synergy by the two enzymes, where you get the best of both enzymes at the target locus.

This surprising result indicates that the recruitment strategy is a dynamic process which will allow the on site recruitment of multiple effectors via the same aptamer (RNA motif) to the same locus, which offers many practical therapeutic advantages and opens the opportunity to new technologies being developed in the field of genome editing.

**Example 2: Locus directed multi-protein multi-aptamer recruitment system for novel base editing application in human cells**

In this example, primary human lymphocytes will be used to show that multiple effector proteins can be actively recruited to the same locus using multiple aptamers (RNA motifs) on the same single sgRNA delivering the benefits of protein on the guidance system (e.g. nCas9-UGI-UGI to open and nick the DNA) and multiple deaminases (Rat Apobec1 and Human AID) recruited to the site specifically via different aptamers on the same guide.

The Pan T cells will be activated utilising anti-CD3 and anti-CD28 and then cells will be electroporated with mRNA components for either Apobec1-MCP, AID-PCP or both deaminases, with nCas9-UGI-UGI components, sgRNA-MS2-P7 aptamers. The cells will then be incubated for a further 24 hours, and then stimulated for 48 hours with anti-CD3 and anti-CD28 and then checked for surface KO by flow cytometry and the base editing will be checked by targeted PCR amplification and Sanger sequencing.

The data will show that multiple different enzymes can be recruited to the same locus using multiple different aptamers on the same sgRNA in addition to the targeted enzymatic activity (Nickase) of the guidance protein.

**Example 3: Locus directed system for novel base editing application in human cells via single aptamer recruitment of multiple effectors to the nickase**

In this example, primary human lymphocytes will be used to show that multiple enzymes can be actively recruited to the same locus using the aptamer based recruitment (e.g. via AID-MCP and Apobec1-MCP) where the aptamer is linked to the C-terminus of the nickase guidance system (e.g.MS2-nCas9-UGI-UGI ) to have the benefits of the guidance system (nickase activity) and multiple effectors recruited by the same aptamer in the same genetic locus. This has benefits of single site directed multiple genetic effector action.

The Pan T cells will be activated utilising anti-CD3 and anti-CD28 and then cells will be electroporated with mRNA components for either AID-MCP, with MS2-nCas9-UGI-UGI components, sgRNA. The cells will then be incubated for a further 24 hours, and then stimulated for 48 hours with anti-CD3 and anti-CD28 and then checked for surface KO by flow cytometry and the base editing checked by targeted PCR amplification and Sanger sequencing.

The data will show that multiple base editing technologies can be combined for enhanced functionality, with both the fusion and aptamer recruitment strategies are complementary to have improved functionality. Improved editing efficiency without the requirement to optimise more than one system that delivering an effector to a single locus.

**Example 4: Locus directed system for novel base editing application in human cells via multiple aptamer recruitment of multiple effectors to the nickase**

In this example, primary human lymphocytes will be used to show that multiple enzymes can be actively recruited to the same locus using the aptamer based recruitment (e.g. via AID-MCP and Apobec1-MCP) where multiple aptamers are linked to the nickase guidance system (e.g.MS2-MS2-nCas9-UGI-UGI ) to have the benefits of the guidance system (nickase activity) and multiple effectors recruited by the same aptamer in various orientations at the same genetic locus. This has benefits of single site directed multiple genetic effector action.

The Pan T cells will be activated utilising anti-CD3 and anti-CD28 and then cells will be electroporated with mRNA components for either AID-MCP, with MS2-MS2-nCas9-UGI-UGI components, sgRNA. The cells will then be incubated for a further 24 hours, and then stimulated for 48 hours with anti-CD3 and anti-CD28 and then checked for surface KO by flow cytometry and the base editing will be checked by targeted PCR amplification and Sanger sequencing.

The data will exemplify that multiple base editing technologies can be combined for enhanced functionality, with both the fusion and aptamer recruitment strategies are complementary to have improved functionality.

**Example 5: Locus directed protein aptamer and direct fusion recruitment system for novel base editing application in human cells**

In this example, primary human lymphocytes will be used to show that multiple enzymes can be actively recruited to the same locus using the aptamer based recruitment (e.g. via AID-MCP and sgRNA-MS2 Aptamer) and via direct enzyme fusion to the guidance system (e.g. Apobec1-nCas9-UGI-UGI) to get the benefits of the guidance system (e.g. guidance and nickase activity), the direct fusion partner (e.g Apobec1), and via protein molecules recruited by the aptamer (e.g. AID-MCP) gaining the benefits from multiple base editing technologies and improved editing efficiency.

The Pan T cells will be activated utilising anti-CD3 and anti-CD28 and then cells are electroporated with mRNA components for either AID-MCP, with Apobec1-nCas9-UGI-UGI components, sgRNA. The cells will then be incubated for a further 24 hours, and then stimulated for 48 hours with anti-CD3 and anti-CD28 and then checked for surface KO by flow cytometry and the base editing will be checked by targeted PCR amplification and Sanger sequencing.

The data will exemplify that multiple base editing technologies can be combined for enhanced functionality, with both the fusion and aptamer recruitment strategies are complementary to have improved functionality.

**Example 6: Locus directed multi-protein via a single aptamer and direct fusion recruitment system for novel base editing application in human cells**

In this example, primary human lymphocytes will be used to prove that multiple enzymes can be actively recruited to the same locus using the aptamer based recruitment (e.g. Apobec-MCP, AID-MCP, and sgRNA-MS2 Aptamer), of multiple proteins with the same aptamer, and simultaneously via direct enzyme fusion to the guidance system (e.g. TadA-TadA*-nCas9 ). To get the benefits of the guidance system (e.g. guidance and nickase activity), the direct fusion partner (e.g Apobec1), and via multiple protein molecules recruited by the aptamer (e.g. AID-MCP and Apobec1-MCP) simultaneously gaining the benefits from multiple base editing technologies be combined for an enhanced version.

The Pan T cells will ne activated utilising anti-CD3 and anti-CD28 and then cells will be electroporated with mRNA components for Apobec1-MCP, AID-MCP, Apobec1-MCP, TadA-TadA*-nCas9-UGI-UGI components, and sgRNA. The cells will then be incubated for a further 24 hours, and then stimulated for 48 hours with anti-CD3 and anti-CD28 and then checked for surface KO by flow cytometry and the base editing will be checked by targeted PCR amplification and Sanger sequencing.

The data will exemplify that multiple base editing technologies can be combined for enhanced functionality, with both the fusion and aptamer recruitment strategies are complementary to have improved functionality.

**Example 7: Locus directed multi-protein via a multiple aptamers and direct fusion recruitment system for novel base editing application in human cells**

In this example, primary human lymphocytes will be used to exemplify that multiple enzymes can be actively recruited to the same locus using the aptamer based recruitment (e.g. Apobec-MCP, AID-PCP, and sgRNA-MS2-P7 Aptamers), of multiple proteins with multiple different aptamers, and via direct enzyme fusion to the guidance system (e.g. TadA-TadA*-nCas9 ) to get the benefits of the guidance system (e.g. guidance and nickase activity), the direct fusion partner (e.g TadA-TadA*), and via multiple protein molecules recruited by aptamers (e.g. AID-MCP and Apobec1-PCP) gaining the benefits from multiple base editing technologies be combined for an enhanced version.

The Pan T cells will be activated utilising anti-CD3 and anti-CD28 and then cells will be electroporated with mRNA components for Apobec1-MCP, AID-MCP, Apobec1-MCP, TadA-TadA*-nCas9-UGI-UGI components, and sgRNA. The cells will then be incubated for a further 24 hours, and then stimulated for 48 hours with anti-CD3 and anti-CD28 and then checked for surface KO by flow cytometry and the base editing will be checked by targeted PCR amplification and Sanger sequencing.

The data will exemplify that multiple base editing technologies can be combined for enhanced functionality, with both the fusion and aptamer recruitment strategies are complementary to have improved functionality.

**Example 8: Locus directed multi-protein via a single aptamer recruitment system for novel multiplex base editing applications in human cells**In this example, primary human lymphocytes were used to demonstrate that multiple different effector proteins can be actively recruited to the same locus using a single aptamer recruitment system (RNA-ligand binding complex). In this example, each of the three effector proteins comprised an effector domain with deaminase activity, and a ligand, MCP. Through sharing a common ligand binding moiety, the three effector proteins were capable of being recruited by the same RNA-ligand binding complex, which comprised a guide RNA and an MS2 RNA motif. Thereby recruiting to a target site and delivering the benefits of both the sequence targeting component on the guidance system (e.g. nCas9-UGI-UGI to open and nick the DNA) and multiple effector proteins (ligand fused or linked to different effector proteins e.g. Lizard Apobec 1 (Anolis), Rat Apobec1(Rattus), and/or Human AID (Homo)) via a single ligand binding moiety on the sgRNA (RNA-ligand binding complex). Moreover, by providing multiple RNA-ligand binding complexes, each comprising distinct guide RNAs but the same ligand binding moiety (the MS2 RNA motif) it was further demonstrated that the system described in the present invention can be applied to target multiple loci in the genome, enabling simultaneous multiplexed base-editing.

The Pan T cells were activated utilising anti-CD3 and anti-CD28 and then cells were electroporated with mRNA components for either Anolis Apobec1-MCP, Rattus Apobec1-MCP, Homo AID-MCP or both deaminases, with nCas9-UGI-UGI components, and sgRNAs. The cells were then incubated for a further 96-120 hours, before creating a stimulated T cell population for flow cytometry and an unstimulated population for gDNA collection, which was then incubated for a further 24-48 hours. Base editing was checked by targeted PCR amplification and Sanger sequencing (Figure 8) and the surface KO by a multi-stain panel including (B2M, CD52, TRAC, and PD-1) by flow cytometry, which was used to ascertain multiplex KO in the population (Figure 9).

The data demonstrate that multiple effector proteins, not just one, can be recruited via the same RNA-ligand binding complex and can be utilised across many loci in the genome at the same time. The data also indicate that different combinations of deaminase can lead to different functional outcomes, with certain mixes leading to preferential editing profiles and functional outcomes compared to using one single deaminase. With Figure 9 displaying a surprising improvement for functional tetraplex editing, compared to a single deaminase, in therapeutically relevant immune cell system.

This supports the other data in that the modular nature of the aptamer recruitment base editing can facilitate novel and practical therapeutic as well as non-therapeutic applications (e.g. screening), opening the possibility for novel methodologies in the field of genome editing.

### Material and Methods

Guides were designed for specific base editing windows calculated at set distances from the PAM motif (NGG). The sgRNAs were synthesized by Agilent Technologies.

**Synthetic sgRNA Sequences:**

B2M sgRNA **(SEQ ID No. 34)**:

mU*mU*CGUAUCUGUAAAACCAAGGUUUUAGAGCUAGAAAUAGCAAGUUAAAAUAAGGCUAGUCCGU UAUCAACUUGAAAAAGUGGCACCGAGUCGGUGCGCGCACAUGAGGAUCACCCAUGUGCUUUUmU*mU* U

CD52 sgRNA **(SEQ ID No. 35)**:

mA*mC*ucacgcuggauagccuccGUUUUAGAGCUAGAAAUAGCAAGUUAAAAUAAGGCUAGUCCGUUAUCA ACUUGAAAAAGUGGCACCGAGUCGGUGCGCGCACAUGAGGAUCACCCAUGUGCUUUUmU*mU*U

TRAC sgRNA **(SEQ ID No. 36)**:

mC*mU*cuuaccuguaccauaaccGUUUUAGAGCUAGAAAUAGCAAGUUAAAAUAAGGCUAGUCCGUUAUC AACUUGAAAAAGUGGCACCGAGUCGGUGCGCGCACAUGAGGAUCACCCAUGUGCUUUUmU*mU*U

PDCD-1 sgRNA **(SEQ ID No. 37)**:

mC\*mA\*ccuaccuaagaaccauccGUUUUAGAGCUAGAAAUAGCAAGUUAAAAUAAGGCUAGUCCGUUAUCA
ACUUGAAAAAGUGGCACCGAGUCGGUGCGCGCACAUGAGGAUCACCCAUGUGCUUUUmU\*mU\*U

Scramble sgRNA **(SEQ ID No. 38)**:


mG\*mC\*ACUACCAGAGCUAACUCAGUUUUAGAGCUAGAAAUAGCAAGUUAAAAUAAGGCUAGUCCGUU
AUCAACUUGAAAAAGUGGCACCGAGUCGGUGCGCGCACAUGAGGAUCACCCAUGUGCUUUUmU\*mU\*U

2'OMe (m) and phosphorothioate (\*) modified residues

**mRNA Component Generation:** Most of the messenger RNA molecules were custom generated by Trilink utilising modified nucleotides: Pseudouridine and 5-Methyl-Cytosine. The mRNA components translated to the following proteins: Deaminase AID-MCP = NLS-Homo-AID-Linker-MCP **(SEQ ID No. 41)**, Deaminase Apobec1-MCP = NLS-Rattus-Apobec1-Linker-MCP **(SEQ ID No. 40)**, and Cas9-UGI-UGI = NLS-nCas9-UGI-UGI-NLS **(SEQ ID No. 42)**. One mRNA component was synthesised in-house utilised unmodified ribonucleotides. The mRNA components translated to the following protein: NLS-Anolis(P16A-E17A)-Apobec1-Linker-MCP **(SEQ ID No. 39)**.

NLS-Anolis(P16A-E17A)-Apobec1-Linker-MCP **(SEQ ID No. 39)**:


MAPKKKRKVMEPEAFQRNFDPREFAACTLLLYEIHWDNNTSRNWCTNKPGLHAEENFLQIFNEKIDIKQDTPCSIT
WFLSWSPCYPCSQAIIKFLEAHPNVSLEIKAARLYMHQIDCNKEGLRNLGRNRVSIMNLPDYRHCWTTFVVPRGA
NEDYWPQDFLPAITNYSRELDSILQDELKTPLGDTTHTSPPCPAPELLGGPMASNFTQFVLVDNGGTGDVTVAPSN
FANGIAEWISSNSRSQAYKVTCSVRQSSAQNRKYTIKVEVPKGAWRSYLNMELTIPIFATNSDCELIVKAMQGLLKD
GNPIPSAIAANSGIY

NLS-Rattus-Apobec1-Linker-MCP **(SEQ ID No. 40)**:


MAPKKKRKVSSETGPVAVDPTLRRRIEPHEFEVFFDPRELRKETCLLYEINWGGRHSIWRHTSQNTNKHVEVNFIEK
FTTERYFCPNTRCSITWFLSWSPCGECSRAITEFLSRYPHVTLFIYIARLYHHADPRNRQGLRDLISSGVTIQIMTEQES
GYCWRNFVNYSPSNEAHWPRYPHLWVRLYVLELYCIILGLPPCLNILRRKQPQLTFFTIALQSCHYQRLPPHILWAT
GLKELKTPLGDTTHTSPPCPAPELLGGPMASNFTQFVLVDNGGTGDVTVAPSNFANGIAEWISSNSRSQAYKVTCS
VRQSSAQNRKYTIKVEVPKGAWRSYLNMELTIPIFATNSDCELIVKAMQGLLKDGNPIPSAIAANSGIY

NLS-Homo-AID-L25-MCP **(SEQ ID No. 41)**:


MAPKKKRKVMDSLLMNRRKFLYQFKNVRWAKGRRETYLCYVVKRRDSATSFSLDFGYLRNKNGCHVELLFLRYISD
WDLDPGRCYRVTWFTSWSPCYDCARHVADFLRGNPNLSLRIFTARLYFCEDRKAEPEGLRRLHRAGVQIAIMTFK
DYFYCWNTFVENHERTFKAWEGLHENSVRLSRQLRRILLPLYEVDDLRDAFRTLGLELKTPLGDTTHTSPPCPAPELL
GGPMASNFTQFVLVDNGGTGDVTVAPSNFANGIAEWISSNSRSQAYKVTCSVRQSSAQNRKYTIKVEVPKGAWR
SYLNMELTIPIFATNSDCELIVKAMQGLLKDGNPIPSAIAANSGIY

NLS-nCas9(D10A)-UGI-UGI-NLS **(SEQ ID No. 42)**:

MPKKKRKVDKKYSIGLAIGTNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHEKYPTIYHLRKKLVDSTDKAD
LRLIYLALAHMIKFRGHFLIEGDLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFRIPYYVGPLARGNSRFAWM
TRKSEETITPWNFEEVVDKGASAQSFIERMTNFDKNLPNEKVLPKHSLLYEYFTVYNELTKVKYVTEGMRKPAFLSG
EQKKAIVDLLFKTNRKVTVKQLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPENIVIEMARENQTTQKGQK
NSRERMKRIEEGIKELGSQILKEHPVENTQLQNEKLYLYYLQNGRDMYVDQELDINRLSDYDVDHIVPQSFLKDDSI
DNKVLTRSDKNRGKSDNVPSEEVVKKMKNYWRQLLNAKLITQRKFDNLTKAERGGLSELDKAGFIKRQLVETRQIT
KHVAQILDSRMNTKYDENDKLIREVKVITLKSKLVSDFRKDFQFYKVREINNYHHAHDAYLNAVVGTALIKKYPKLES
EFVYGDYKVYDVRKMIAKSEQEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYSVLVVAKVEKGKSKKLKSVK
ELLGITIMERSSFEKNPIDFLEAKGYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDSGGSGGSGGSTNLSDIIEKETGKQLVIQESILM
LPEEVEEVIGNKPESDILVHTAYDESTDENVMLLTSDAPEYKPWALVIQDSNGENKIKMLSGGSGGSGGSTNLSDIIE
KETGKQLVIQESILMLPEEVEEVIGNKPESDILVHTAYDESTDENVMLLTSDAPEYKPWALVIQDSNGENKIKMLSG
GSKRTADGSEFEPKKKRKV

**T Cells Culturing:** PBMCs were isolated from fresh blood sources using Lymphoprep (STEMCELL Technologies) gradient centrifugation and then Total T Cells were isolated via negative selection (STEMCELL Technologies). T cells were cultured into Immunocult XT media (STEMCELL Technologies) with 1x Penicillin/Streptomycin (Thermofisher) at 37C and 5% CO2.

**CD3+ T Cell Activation:** T cells were activated by using 1:1 bead:cell ratio of Dynabeads Human T Activator CD3/CD28 beads (Thermofisher) cultured in Immunocult XT media (STEMCELL Technologies) in the presence of 100U/ml IL-2 (STEMCELL Technologies) and 1x Penicillin/Streptomycin (Thermofisher) at 37C and 5% $CO_2$ for 48 hours. Post-activation, beads were removed by placement on a magnet and the transfer of the cells back into culture.

**T Cell Electroporation:** After 48-72 post-activation T cells were electroporated with using the Neon Electroporator (Thermofisher) or 4D Nucleofector (Lonza). Neon Electroporator conditions were 1600v/10ms/3 pulses with a 10ul tip with 250k cells, combined total of mRNA amount of 1-2ug, for both the Deaminase-MCP and nCas9-UGI-UGI, and 0.2-1.8 umol of each sgRNA. Post-electroporation cells were transferred to Immunocult XT media with 100U IL-2, 100U IL-7 and 100U IL-15 (STEMCELL Technologies) and cultured at 37C and 5% CO2 for 96-120 hours. The cell culture wells were then divided into two, one half left unstimulated and another stimulated with 50 ng/ml phorbol myristate acetate and 250 ng/ml Ionomycin, which was incubated for an additional 24-48 hours.

**Genomic DNA Analysis:** Genomic DNA was released from lysed cells 48-72 hours post-electroporation. Loci of interest were amplified by PCR and products then sent for Sanger sequencing (Genewiz). Data was analysed by proprietary in-house software.

## SEQUENCES

**Humanised nucleotide sequence WT Streptococcus pyogenes Cas9 (SEQ ID No. 43)**

ATGGACAAGAAGTACAGCATCGGCCTGGACATCGGCACCAACTCTGTGGGCTGGGCCGTGATCACCGACGAG
TACAAGGTGCCCAGCAAGAAATTCAAGGTGCTGGGCAACACCGACCGGCACAGCATCAAGAAGAACCTGATC
GGAGCCCTGCTGTTCGACAGCGGCGAAACAGCCGAGGCCACCCGGCTGAAGAGAACCGCCAGAAGAAGATA
CACCAGACGGAAGAACCGGATCTGCTATCTGCAAGAGATCTTCAGCAACGAGATGGCCAAGGTGGACGACAG
CTTCTTCCACAGACTGGAAGAGTCCTTCCTGGTGGAAGAGGATAAGAAGCACGAGCGGCACCCCATCTTCGG
CAACATCGTGGACGAGGTGGCCTACCACGAGAAGTACCCCACCATCTACCACCTGAGAAAGAAACTGGTGGA
CAGCACCGACAAGGCCGACCTGCGGCTGATCTATCTGGCCCTGGCCCACATGATCAAGTTCCGGGGCCACTTC
CTGATCGAGGGCGACCTGAACCCCGACAACAGCGACGTGGACAAGCTGTTCATCCAGCTGGTGCAGACCTAC
AACCAGCTGTTCGAGGAAAACCCCATCAACGCCAGCGGCGTGGACGCCAAGGCCATCCTGTCTGCCAGACTG
AGCAAGAGCAGACGGCTGGAAAATCTGATCGCCCAGCTGCCCGGCGAGAAGAAGAATGGCCTGTTCGGAAA
CCTGATTGCCCTGAGCCTGGGCCTGACCCCCAACTTCAAGAGCAACTTCGACCTGGCCGAGGATGCCAAACTG
CAGCTGAGCAAGGACACCTACGACGACGACCTGGACAACCTGCTGGCCCAGATCGGCGACCAGTACGCCGAC
CTGTTTCTGGCCGCCAAGAACCTGTCCGACGCCATCCTGCTGAGCGACATCCTGAGAGTGAACACCGAGATCA
CCAAGGCCCCCCTGAGCGCCTCTATGATCAAGAGATACGACGAGCACCACCAGGACCTGACCCTGCTGAAAG
CTCTCGTGCGGCAGCAGCTGCCTGAGAAGTACAAAGAGATTTTCTTCGACCAGAGCAAGAACGGCTACGCCG
GCTACATTGACGGCGGAGCCAGCCAGGAAGAGTTCTACAAGTTCATCAAGCCCATCCTGGAAAAGATGGACG
GCACCGAGGAACTGCTCGTGAAGCTGAACAGAGAGGACCTGCTGCGGAAGCAGCGGACCTTCGACAACGGC
AGCATCCCCCACCAGATCCACCTGGGAGAGCTGCACGCCATTCTGCGGCGGCAGGAAGATTTTTACCCATTCC
TGAAGGACAACCGGGAAAAGATCGAGAAGATCCTGACCTTCCGCATCCCCTACTACGTGGGCCCTCTGGCCA
GGGGAAACAGCAGATTCGCCTGGATGACCAGAAAGAGCGAGGAAACCATCACCCCCTGGAACTTCGAGGAA
GTGGTGGACAAGGGCGCTTCCGCCCAGAGCTTCATCGAGCGGATGACCAACTTCGATAAGAACCTGCCCAAC
GAGAAGGTGCTGCCCAAGCACAGCCTGCTGTACGAGTACTTCACCGTGTATAACGAGCTGACCAAAGTGAAA
TACGTGACCGAGGGAATGAGAAAGCCCGCCTTCCTGAGCGGCGAGCAGAAAAAGGCCATCGTGGACCTGCT
GTTCAAGACCAACCGGAAAGTGACCGTGAAGCAGCTGAAAGAGGACTACTTCAAGAAAATCGAGTGCTTCGA
CTCCGTGGAAATCTCCGGCGTGGAAGATCGGTTCAACGCCTCCCTGGGCACATACCACGATCTGCTGAAAATT
ATCAAGGACAAGGACTTCCTGGACAATGAGGAAAACGAGGACATTCTGGAAGATATCGTGCTGACCCTGACA
CTGTTTGAGGACAGAGAGATGATCGAGGAACGGCTGAAAACCTATGCCCACCTGTTCGACGACAAAGTGATG
AAGCAGCTGAAGCGGCGGAGATACACCGGCTGGGGCAGGCTGAGCCGGAAGCTGATCAACGGCATCCGGG
ACAAGCAGTCCGGCAAGACAATCCTGGATTTCCTGAAGTCCGACGGCTTCGCCAACAGAAACTTCATGCAGCT
GATCCACGACGACAGCCTGACCTTTAAAGAGGACATCCAGAAAGCCCAGGTGTCCGGCCAGGGCGATAGCCT
GCACGAGCACATTGCCAATCTGGCCGGCAGCCCGCCATTAAGAAGGGCATCCTGCAGACAGTGAAGGTGGT
GGACGAGCTCGTGAAAGTGATGGGCCGGCACAAGCCCGAGAACATCGTGATCGAAATGGCCAGAGAGAACC
AGACCACCCAGAAGGGACAGAAGAACAGCCGCGAGAGAATGAAGCGGATCGAAGAGGGCATCAAAGAGCT
GGGCAGCCAGATCCTGAAAGAACACCCCGTGGAAAACACCCAGCTGCAGAACGAGAAGCTGTACCTGTACTA
CCTGCAGAATGGGCGGGATATGTACGTGGACCAGGAACTGGACATCAACCGGCTGTCCGACTACGATGTGGA
CCATATCGTGCCTCAGAGCTTTCTGAAGGACGACTCCATCGACAACAAGGTGCTGACCAGAAGCGACAAGAA
CCGGGGCAAGAGCGACAACGTGCCCTCCGAAGAGGTCGTGAAGAAGATGAAGAACTACTGGCGGCAGCTGC
TGAACGCCAAGCTGATTACCCAGAGAAAGTTCGACAATCTGACCAAGGCCGAGAGAGGCGGCCTGAGCGAA
CTGGATAAGGCCGGCTTCATCAAGAGACAGCTGGTGGAAACCCGGCAGATCACAAAGCACGTGGCACAGATC
CTGGACTCCCGGATGAACACTAAGTACGACGAGAATGACAAGCTGATCCGGGAAGTGAAAGTGATCACCCTG
AAGTCCAAGCTGGTGTCCGATTTCCGGAAGGATTTCCAGTTTTACAAAGTGCGCGAGATCAACAACTACCACC
ACGCCCACGACGCCTACCTGAACGCCGTCGTGGGAACCGCCCTGATCAAAAAGTACCCTAAGCTGGAAAGCG

AGTTCGTGTACGGCGACTACAAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGACCGAGATTACCCTGGCCAACGG
CGAGATCCGGAAGCGGCCTCTGATCGAGACAAACGGCGAAACCGGGGAGATCGTGTGGGATAAGGGCCGG
GATTTTGCCACCGTGCGGAAAGTGCTGAGCATGCCCCAAGTGAATATCGTGAAAAAGACCGAGGTGCAGACA
GGCGGCTTCAGCAAAGAGTCTATCCTGCCCAAGAGGAACAGCGATAAGCTGATCGCCAGAAAGAAGGACTG
GGACCCTAAGAAGTACGGCGGCTTCGACAGCCCCACCGTGGCCTATTCTGTGCTGGTGGTGGCCAAAGTGGA
AAAGGGCAAGTCCAAGAAACTGAAGAGTGTGAAAGAGCTGCTGGGGATCACCATCATGGAAAGAAGCAGCT
TCGAGAAGAATCCCATCGACTTTCTGGAAGCCAAGGGCTACAAAGAAGTGAAAAAGGACCTGATCATCAAGC
TGCCTAAGTACTCCCTGTTCGAGCTGGAAAACGGCCGGAAGAGAATGCTGGCCTCTGCCGGCGAACTGCAGA
AGGGAAACGAACTGGCCCTGCCCTCCAAATATGTGAACTTCCTGTACCTGGCCAGCCACTATGAGAAGCTGAA
GGGCTCCCCCGAGGATAATGAGCAGAAACAGCTGTTTGTGGAACAGCACAAGCACTACCTGGACGAGATCAT
CGAGCAGATCAGCGAGTTCTCCAAGAGAGTGATCCTGGCCGACGCTAATCTGGACAAAGTGCTGTCCGCCTA
CAACAAGCACCGGGATAAGCCCATCAGAGAGCAGGCCGAGAATATCATCCACCTGTTTACCCTGACCAATCTG
GGAGCCCTGCCGCCTTCAAGTACTTTGACACCACCATCGACCGGAAGAGGTACACCAGCACCAAAGAGGTG
CTGGACGCCACCCTGATCCACCAGAGCATCACCGGCCTGTACGAGACACGGATCGACCTGTCTCAGCTGGGA
GGTGAC

**Humanised amino acid sequence WT *Streptococcus pyogenes* Cas9 (SEQ ID No. 44)**

MDKKYSIGLDIGTNSVGWAVITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLSKSRRLENLIAQLPGEKKNG
LFGNLIALSLGLTPNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKMDGTEELLVKLN
REDLLRKQRTFDNGSIPHQIHLGELHAILRRQEDFYPFLKDNREKIEKILTFRIPYYVGPLARGNSRFAWMTRKSEETI
TPWNFEEVVDKGASAQSFIERMTNFDKNLPNEKVLPKHSLLYEYFTVYNELTKVKYVTEGMRKPAFLSGEQKKAIV
DLLFKTNRKVTVKQLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQLIHDDSLTFKE
DIQKAQVSGQGDSLHEHIANLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDVDHIVPQSFLKDDSIDNKVLTR
SDKNRGKSDNVPSEEVVKKMKNYWRQLLNAKLITQRKFDNLTKAERGGLSELDKAGFIKRQLVETRQITKHVAQIL
DSRMNTKYDENDKLIREVKVITLKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEIVWDKGRDFATVRKVLSMP
QVNIVKKTEVQTGGFSKESILPKRNSDKLIARKKDWDPKKYGGFDSPTVAYSVLVVAKVEKGKSKKLKSVKELLGITI
MERSSFEKNPIDFLEAKGYKEVKKDLIIKLPKYSLFELENGRKRMLASAGELQKGNELALPSKYVNFLYLASHYEKLKG
SPEDNEQKQLFVEQHKHYLDEIIEQISEFSKRVILADANLDKVLSAYNKHRDKPIREQAENIIHLFTLTNLGAPAAFKYF
DTTIDRKRYTSTKEVLDATLIHQSITGLYETRIDLSQLGGD

**Humanised nucleotide sequence *Streptococcus pyogenes* nCas9 (A29C mutation in bold and underlined) (SEQ ID No. 45)**

ATGGACAAGAAGTACAGCATCGGCCTGG**C**CATCGGCACCAACTCTGTGGGCTGGGCCGTGATCACCGACGAG
TACAAGGTGCCCAGCAAGAAATTCAAGGTGCTGGGCAACACCGACCGGCACAGCATCAAGAAGAACCTGATC
GGAGCCCTGCTGTTCGACAGCGGCGAAACAGCCGAGGCCACCGGCTGAAGAGAACCGCCAGAAGAAGATA
CACCAGACGGAAGAACCGGATCTGCTATCTGCAAGAGATC**TT**CAGCAACGAGATGGCCAAGGTGGACGACAG

CTTCTTCCACAGACTGGAAGAGTCCTTCCTGGTGGAAGAGGATAAGAAGCACGAGCGGCACCCCATCTTCGG
CAACATCGTGGACGAGGTGGCCTACCACGAGAAGTACCCCACCATCTACCACCTGAGAAAGAAACTGGTGGA
CAGCACCGACAAGGCCGACCTGCGGCTGATCTATCTGGCCCTGGCCCACATGATCAAGTTCCGGGGCCACTTC
CTGATCGAGGGCGACCTGAACCCCGACAACAGCGACGTGGACAAGCTGTTCATCCAGCTGGTGCAGACCTAC
AACCAGCTGTTCGAGGAAAACCCCATCAACGCCAGCGGCGTGGACGCCAAGGCCATCCTGTCTGCCAGACTG
AGCAAGAGCAGACGGCTGGAAAATCTGATCGCCCAGCTGCCCGGCGAGAAGAAGAATGGCCTGTTCGGAAA
CCTGATTGCCCTGAGCCTGGGCCTGACCCCCAACTTCAAGAGCAACTTCGACCTGGCCGAGGATGCCAAACTG
CAGCTGAGCAAGGACACCTACGACGACGACCTGGACAACCTGCTGGCCCAGATCGGCGACCAGTACGCCGAC
CTGTTTCTGGCCGCCAAGAACCTGTCCGACGCCATCCTGCTGAGCGACATCCTGAGAGTGAACACCGAGATCA
CCAAGGCCCCCCTGAGCGCCTCTATGATCAAGAGATACGACGAGCACCACCAGGACCTGACCCTGCTGAAAG
CTCTCGTGCGGCAGCAGCTGCCTGAGAAGTACAAAGAGATTTTCTTCGACCAGAGCAAGAACGGCTACGCCG
GCTACATTGACGGCGGAGCCAGCCAGGAAGAGTTCTACAAGTTCATCAAGCCCATCCTGGAAAAGATGGACG
GCACCGAGGAACTGCTCGTGAAGCTGAACAGAGAGGACCTGCTGCGGAAGCAGCGGACCTTCGACAACGGC
AGCATCCCCCACCAGATCCACCTGGGAGAGCTGCACGCCATTCTGCGGCGGCAGGAAGATTTTTACCCATTCC
TGAAGGACAACCGGGAAAAGATCGAGAAGATCCTGACCTTCCGCATCCCCTACTACGTGGGCCCTCTGGCCA
GGGGAAACAGCAGATTCGCCTGGATGACCAGAAAGAGCGAGGAAACCATCACCCCCTGGAACTTCGAGGAA
GTGGTGGACAAGGGCGCTTCCGCCCAGAGCTTCATCGAGCGGATGACCAACTTCGATAAGAACCTGCCCAAC
GAGAAGGTGCTGCCCAAGCACAGCCTGCTGTACGAGTACTTCACCGTGTATAACGAGCTGACCAAAGTGAAA
TACGTGACCGAGGGAATGAGAAAGCCCGCCTTCCTGAGCGGCGAGCAGAAAAAGGCCATCGTGGACCTGCT
GTTCAAGACCAACCGGAAAGTGACCGTGAAGCAGCTGAAAGAGGACTACTTCAAGAAAATCGAGTGCTTCGA
CTCCGTGGAAATCTCCGGCGTGGAAGATCGGTTCAACGCCTCCCTGGGCACATACCACGATCTGCTGAAAATT
ATCAAGGACAAGGACTTCCTGGACAATGAGGAAAACGAGGACATTCTGGAAGATATCGTGCTGACCCTGACA
CTGTTTGAGGACAGAGAGATGATCGAGGAACGGCTGAAAACCTATGCCCACCTGTTCGACGACAAAGTGATG
AAGCAGCTGAAGCGGCGGAGATACACCGGCTGGGGCAGGCTGAGCCGGAAGCTGATCAACGGCATCCGGG
ACAAGCAGTCCGGCAAGACAATCCTGGATTTCCTGAAGTCCGACGGCTTCGCCAACAGAAACTTCATGCAGCT
GATCCACGACGACAGCCTGACCTTTAAAGAGGACATCCAGAAAGCCCAGGTGTCCGGCCAGGGCGATAGCCT
GCACGAGCACATTGCCAATCTGGCCGGCAGCCCCGCCATTAAGAAGGGCATCCTGCAGACAGTGAAGGTGGT
GGACGAGCTCGTGAAAGTGATGGGCCGGCACAAGCCCGAGAACATCGTGATCGAAATGGCCAGAGAGAACC
AGACCACCCAGAAGGGACAGAAGAACAGCCGCGAGAGAATGAAGCGGATCGAAGAGGGCATCAAAGAGCT
GGGCAGCCAGATCCTGAAAGAACACCCCGTGGAAAACACCCAGCTGCAGAACGAGAAGCTGTACCTGTACTA
CCTGCAGAATGGGCGGGATATGTACGTGGACCAGGAACTGGACATCAACCGGCTGTCCGACTACGATGTGGA
CCATATCGTGCCTCAGAGCTTTCTGAAGGACGACTCCATCGACAACAAGGTGCTGACCAGAAGCGACAAGAA
CCGGGGCAAGAGCGACAACGTGCCCTCCGAAGAGGTCGTGAAGAAGATGAAGAACTACTGGCGGCAGCTGC
TGAACGCCAAGCTGATTACCCAGAGAAAGTTCGACAATCTGACCAAGGCCGAGAGAGGCGGCCTGAGCGAA
CTGGATAAGGCCGGCTTCATCAAGAGACAGCTGGTGGAAACCCGGCAGATCACAAAGCACGTGGCACAGATC
CTGGACTCCCGGATGAACACTAAGTACGACGAGAATGACAAGCTGATCCGGGAAGTGAAAGTGATCACCCTG
AAGTCCAAGCTGGTGTCCGATTTCCGGAAGGATTTCCAGTTTTACAAAGTGCGCGAGATCAACAACTACCACC
ACGCCCACGACGCCTACCTGAACGCCGTCGTGGGAACCGCCCTGATCAAAAAGTACCCTAAGCTGGAAAGCG
AGTTCGTGTACGGCGACTACAAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGACCGAGATTACCCTGGCCAACGG
CGAGATCCGGAAGCGGCCTCTGATCGAGACAAACGGCGAAACCGGGGAGATCGTGTGGGATAAGGGCCGG
GATTTTGCCACCGTGCGGAAAGTGCTGAGCATGCCCCAAGTGAATATCGTGAAAAAGACCGAGGTGCAGACA
GGCGGCTTCAGCAAAGAGTCTATCCTGCCCAAGAGGAACAGCGATAAGCTGATCGCCAGAAAGAAGGACTG
GGACCCTAAGAAGTACGGCGGCTTCGACAGCCCCACCGTGGCCTATTCTGTGCTGGTGGTGGCCAAAGTGGA
AAAGGGCAAGTCCAAGAAACTGAAGAGTGTGAAAGAGCTGCTGGGGGATCACCATCATGGAAAGAAGCAGCT

TCGAGAAGAATCCCATCGACTTTCTGGAAGCCAAGGGCTACAAAGAAGTGAAAAAGGACCTGATCATCAAGC
TGCCTAAGTACTCCCTGTTCGAGCTGGAAAACGGCCGGAAGAGAATGCTGGCCTCTGCCGGCGAACTGCAGA
AGGGAAACGAACTGGCCCTGCCCTCCAAATATGTGAACTTCCTGTACCTGGCCAGCCACTATGAGAAGCTGAA
GGGCTCCCCCGAGGATAATGAGCAGAAACAGCTGTTTGTGGAACAGCACAAGCACTACCTGGACGAGATCAT
CGAGCAGATCAGCGAGTTCTCCAAGAGAGTGATCCTGGCCGACGCTAATCTGGACAAAGTGCTGTCCGCCTA
CAACAAGCACCGGGATAAGCCCATCAGAGAGCAGGCCGAGAATATCATCCACCTGTTTACCCTGACCAATCTG
GGAGCCCCTGCCGCCTTCAAGTACTTTGACACCACCATCGACCGGAAGAGGTACACCAGCACCAAAGAGGTG
CTGGACGCCACCCTGATCCACCAGAGCATCACCGGCCTGTACGAGACACGGATCGACCTGTCTCAGCTGGGA
GGTGAC

**Humanised amino acid sequence *Streptococcus pyogenes* nCas9 (D10A residue change in bold and underlined) (SEQ ID No. 46)**

MDKKYSIGL**A**IGTNSVGWAVITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLSKSRRLENLIAQLPGEKKNG
LFGNLIALSLGLTPNFKSNFDLAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYKFIKPILEKMDGTEELLVKLN
REDLLRKQRTFDNGSIPHQIHLGELHAILRRQEDFYPFLKDNREKIEKILTFRIPYYVGPLARGNSRFAWMTRKSEETI
TPWNFEEVVDKGASAQSFIERMTNFDKNLPNEKVLPKHSLLYEYFTVYNELTKVKYVTEGMRKPAFLSGEQKKAIV
DLLFKTNRKVTVKQLKEDYFKKIECFDSVEISGVEDRFNASLGTYHDLLKIIKDKDFLDNEENEDILEDIVLTLTLFEDRE
MIEERLKTYAHLFDDKVMKQLKRRRYTGWGRLSRKLINGIRDKQSGKTILDFLKSDGFANRNFMQLIHDDSLTFKE
DIQKAQVSGQGDSLHEHIANLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDVDHIVPQSFLKDDSIDNKVLTR
SDKNRGKSDNVPSEEVVKKMKNYWRQLLNAKLITQRKFDNLTKAERGGLSELDKAGFIKRQLVETRQITKHVAQIL
DSRMNTKYDENDKLIREVKVITLKSKLVSDFRKDFQFYKVREINNYHHAHDAYLNAVVGTALIKKYPKLESEFVYGDY
KVYDVRKMIAKSEQEIGKATAKYFFYSNIMNFFKTEITLANGEIRKRPLIETNGETGEIVWDKGRDFATVRKVLSMP
QVNIVKKTEVQTGGFSKESILPKRNSDKLIARKKDWDPKKYGGFDSPTVAYSVLVVAKVEKGKSKKLKSVKELLGITI
MERSSFEKNPIDFLEAKGYKEVKKDLIIKLPKYSLFELENGRKRMLASAGELQKGNELALPSKYVNFLYLASHYEKLKG
SPEDNEQKQLFVEQHKHYLDEIIEQISEFSKRVILADANLDKVLSAYNKHRDKPIREQAENIIHLFTLTNLGAPAAFKYF
DTTIDRKRYTSTKEVLDATLIHQSITGLYETRIDLSQLGGD

**Humanised nucleotide sequence *Streptococcus pyogenes* dCas9 (A29C, C2518G, and A2519C base changes in bold and underlined) (SEQ ID No. 47)**

ATGGACAAGAAGTACAGCATCGGCCTGG**C**CATCGGCACCAACTCTGTGGGCTGGGCCGTGATCACCGACGAG
TACAAGGTGCCCAGCAAGAAATTCAAGGTGCTGGGCAACACCGACCGGCACAGCATCAAGAAGAACCTGATC
GGAGCCCTGCTGTTCGACAGCGGCGAAACAGCCGAGGCCACCGGCTGAAGAGAACCGCCAGAAGAAGATA
CACCAGACGGAAGAACCGGATCTGCTATCTGCAAGAGATCTTCAGCAACGAGATGGCCAAGGTGGACGACAG
CTTCTTCCACAGACTGGAAGAGTCCTTCCTGGTGGAAGAGGATAAGAAGCACGAGCGGCACCCCATCTTCGG
CAACATCGTGGACGAGGTGGCCTACCACGAGAAGTACCCCACCATCTACCACCTGAGAAAGAAACTGGTGGA
CAGCACCGACAAGGCCGACCTGCGGCTGATCTATCTGGCCCTGGCCCACATGATCAAGTTCCGGGGCCACTTC
CTGATCGAGGGCGACCTGAACCCCGACAACAGCGACGTGGACAAGCTGTTCATCCAGCTGGTGCAGACCTAC
AACCAGCTGTTCGAGGAAAACCCCATCAACGCCAGCGGCGTGGACGCCAAGGCCATCCTGTCTGCCAGACTG
AGCAAGAGCAGACGGCTGGAAAATCTGATCGCCCAGCTGCCCGGCGAGAAGAAGAATGGCCTGTTCGGAAA

CCTGATTGCCCTGAGCCTGGGCCTGACCCCCAACTTCAAGAGCAACTTCGACCTGGCCGAGGATGCCAAACTG
CAGCTGAGCAAGGACACCTACGACGACGACCTGGACAACCTGCTGGCCCAGATCGGCGACCAGTACGCCGAC
CTGTTTCTGGCCGCCAAGAACCTGTCCGACGCCATCCTGCTGAGCGACATCCTGAGAGTGAACACCGAGATCA
CCAAGGCCCCCCTGAGCGCCTCTATGATCAAGAGATACGACGAGCACCACCAGGACCTGACCCTGCTGAAAG
CTCTCGTGCGGCAGCAGCTGCCTGAGAAGTACAAAGAGATTTTCTTCGACCAGAGCAAGAACGGCTACGCCG
GCTACATTGACGGCGGAGCCAGCCAGGAAGAGTTCTACAAGTTCATCAAGCCCATCCTGGAAAAGATGGACG
GCACCGAGGAACTGCTCGTGAAGCTGAACAGAGAGGACCTGCTGCGGAAGCAGCGGACCTTCGACAACGGC
AGCATCCCCCACCAGATCCACCTGGGAGAGCTGCACGCCATTCTGCGGCGGCAGGAAGATTTTTACCCATTCC
TGAAGGACAACCGGGAAAAGATCGAGAAGATCCTGACCTTCCGCATCCCCTACTACGTGGGCCCTCTGGCCA
GGGGAAACAGCAGATTCGCCTGGATGACCAGAAAGAGCGAGGAAACCATCACCCCCTGGAACTTCGAGGAA
GTGGTGGACAAGGGCGCTTCCGCCCAGAGCTTCATCGAGCGGATGACCAACTTCGATAAGAACCTGCCCAAC
GAGAAGGTGCTGCCCAAGCACAGCCTGCTGTACGAGTACTTCACCGTGTATAACGAGCTGACCAAAGTGAAA
TACGTGACCGAGGGAATGAGAAAGCCCGCCTTCCTGAGCGGCGAGCAGAAAAAGGCCATCGTGGACCTGCT
GTTCAAGACCAACCGGAAAGTGACCGTGAAGCAGCTGAAAGAGGACTACTTCAAGAAAATCGAGTGCTTCGA
CTCCGTGGAAATCTCCGGCGTGGAAGATCGGTTCAACGCCTCCCTGGGCACATACCACGATCTGCTGAAAATT
ATCAAGGACAAGGACTTCCTGGACAATGAGGAAAACGAGGACATTCTGGAAGATATCGTGCTGACCCTGACA
CTGTTTGAGGACAGAGAGATGATCGAGGAACGGCTGAAAACCTATGCCCACCTGTTCGACGACAAAGTGATG
AAGCAGCTGAAGCGGCGGAGATACACCGGCTGGGGCAGGCTGAGCCGGAAGCTGATCAACGGCATCCGGG
ACAAGCAGTCCGGCAAGACAATCCTGGATTTCCTGAAGTCCGACGGCTTCGCCAACAGAAACTTCATGCAGCT
GATCCACGACGACAGCCTGACCTTTAAAGAGGACATCCAGAAAGCCCAGGTGTCCGGCCAGGGCGATAGCCT
GCACGAGCACATTGCCAATCTGGCCGGCAGCCCCGCCATTAAGAAGGGCATCCTGCAGACAGTGAAGGTGGT
GGACGAGCTCGTGAAAGTGATGGGCCGGCACAAGCCCGAGAACATCGTGATCGAAATGGCCAGAGAGAACC
AGACCACCCAGAAGGGACAGAAGAACAGCCGCGAGAGAATGAAGCGGATCGAAGAGGGCATCAAAGAGCT
GGGCAGCCAGATCCTGAAAGAACACCCCGTGGAAAACACCCAGCTGCAGAACGAGAAGCTGTACCTGTACTA
CCTGCAGAATGGGCGGGATATGTACGTGGACCAGGAACTGGACATCAACCGGCTGTCCGACTACGATGTGGA
C**GC**TATCGTGCCTCAGAGCTTTCTGAAGGACGACTCCATCGACAACAAGGTGCTGACCAGAAGCGACAAGAA
CCGGGGCAAGAGCGACAACGTGCCCTCCGAAGAGGTCGTGAAGAAGATGAAGAACTACTGGCGGCAGCTGC
TGAACGCCAAGCTGATTACCCAGAGAAAGTTCGACAATCTGACCAAGGCCGAGAGAGGCGGCCTGAGCGAA
CTGGATAAGGCCGGCTTCATCAAGAGACAGCTGGTGGAAACCCGGCAGATCACAAAGCACGTGGCACAGATC
CTGGACTCCCGGATGAACACTAAGTACGACGAGAATGACAAGCTGATCCGGGAAGTGAAAGTGATCACCCTG
AAGTCCAAGCTGGTGTCCGATTTCCGGAAGGATTTCCAGTTTTACAAAGTGCGCGAGATCAACAACTACCACC
ACGCCCACGACGCCTACCTGAACGCCGTCGTGGGAACCGCCCTGATCAAAAAGTACCCTAAGCTGGAAAGCG
AGTTCGTGTACGGCGACTACAAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGACCGAGATTACCCTGGCCAACGG
CGAGATCCGGAAGCGGCCTCTGATCGAGACAAACGGCGAAACCGGGGAGATCGTGTGGGATAAGGGCCGG
GATTTTGCCACCGTGCGGAAAGTGCTGAGCATGCCCCAAGTGAATATCGTGAAAAAGACCGAGGTGCAGACA
GGCGGCTTCAGCAAAGAGTCTATCCTGCCCAAGAGGAACAGCGATAAGCTGATCGCCAGAAAGAAGGACTG
GGACCCTAAGAAGTACGGCGGCTTCGACAGCCCCACCGTGGCCTATTCTGTGCTGGTGGTGGCCAAAGTGGA
AAAGGGCAAGTCCAAGAAACTGAAGAGTGTGAAAGAGCTGCTGGGGATCACCATCATGGAAAGAAGCAGCT
TCGAGAAGAATCCCATCGACTTTCTGGAAGCCAAGGGCTACAAAGAAGTGAAAAAGGACCTGATCATCAAGC
TGCCTAAGTACTCCCTGTTCGAGCTGGAAAACGGCCGGAAGAGAATGCTGGCCTCTGCCGGCGAACTGCAGA
AGGGAAACGAACTGGCCCTGCCCTCCAAATATGTGAACTTCCTGTACCTGGCCAGCCACTATGAGAAGCTGAA
GGGCTCCCCCGAGGATAATGAGCAGAAACAGCTGTTTGTGGAACAGCACAAGCACTACCTGGACGAGATCAT
CGAGCAGATCAGCGAGTTCTCCAAGAGAGTGATCCTGGCCGACGCTAATCTGGACAAAGTGCTGTCCGCCTA
CAACAAGCACCGGGATAAGCCCATCAGAGAGCAGGCCGAGAATATCATCCACCTGTTTACCCTGACCAATCTG

GGAGCCCCTGCCGCCTTCAAGTACTTTGACACCACCATCGACCGGAAGAGGTACACCAGCACCAAAGAGGTG
CTGGACGCCACCCTGATCCACCAGAGCATCACCGGCCTGTACGAGACACGGATCGACCTGTCTCAGCTGGGA
GGTGAC

**Amino acid sequence WT AsCas12a (SEQ ID No. 48)**

MTQFEGFTNLYQVSKTLRFELIPQGKTLKHIQEQGFIEEDKARNDHYKELKPIIDRIYKTYADQCLQLVQLDWENLSA
AIDSYRKEKTEETRNALIEEQATYRNAIHDYFIGRTDNLTDAINKRHAEIYKGLFKAELFNGKVLKQLGTVTTTEHENA
LLRSFDKFTTYFSGFYENRKNVFSAEDISTAIPHRIVQDNFPKFKENCHIFTRLITAVPSLREHFENVKKAIGIFVSTSIEE
VFSFPFYNQLLTQTQIDLYNQLLGGISREAGTEKIKGLNEVLNLAIQKNDETAHIIASLPHRFIPLFKQILSDRNTLSFILE
EFKSDEEVIQSFCKYKTLLRNENVLETAEALFNELNSIDLTHIFISHKKLETISSALCDHWDTLRNALYERRISELTGKITK
SAKEKVQRSLKHEDINLQEIISAAGKELSEAFKQKTSEILSHAHAALDQPLPTTLKKQEEKEILKSQLDSLLGLYHLLDW
FAVDESNEVDPEFSARLTGIKLEMEPSLSFYNKARNYATKKPYSVEKFKLNFQMPTLASGWDVNKEKNNGAILFVK
NGLYYLGIMPKQKGRYKALSFEPTEKTSEGFDKMYYDYFPDAAKMIPKCSTQLKAVTAHFQTHTTPILLSNNFIEPLE
ITKEIYDLNNPEKEPKKFQTAYAKKTGDQKGYREALCKWIDFTRDFLSKYTKTTSIDLSSLRPSSQYKDLGEYYAELNPL
LYHISFQRIAEKEIMDAVETGKLYLFQIYNKDFAKGHHGKPNLHTLYWTGLFSPENLAKTSIKLNGQAELFYRPKSRM
KRMAHRLGEKMLNKKLKDQKTPIPDTLYQELYDYVNHRLSHDLSDEARALLPNVITKEVSHEIIKDRRFTSDKFFFHV
PITLNYQAANSPSKFNQRVNAYLKEHPETPIIGIDRGERNLIYITVIDSTGKILEQRSLNTIQQFDYQKKLDNREKERVA
ARQAWSVVGTIKDLKQGYLSQVIHEIVDLMIHYQAVVVLENLNFGFKSKRTGIAEKAVYQQFEKMLIDKLNCLVLKD
YPAEKVGGVLNPYQLTDQFTSFAKMGTQSGFLFYVPAPYTSKIDPLTGFVDPFVWKTIKNHESRKHFLEGFDFLHYD
VKTGDFILHFKMNRNLSFQRGLPGFMPAWDIVFEKNETQFDAKGTPFIAGKRIVPVIENHRFTGRYRDLYPANELIA
LLEEKGIVFRDGSNILPKLLENDDSHAIDTMVALIRSVLQMRNSNAATGEDYINSPVRDLNGVCFDSRFQNPEWPM
DADANGAYHIALKGQLLLNHLKESKDLKLQNGISNQDWLAYIQELRN

**Amino acid sequence WT AaCas12b (SEQ ID No. 49)**

MAVKSMKVKLRLDNMPEIRAGLWKLHTEVNAGVRYYTEWLSLLRQENLYRRSPNGDGEQECYKTAEECKAELLER
LRARQVENGHCGPAGSDDELLQLARQLYELLVPQAIGAKGDAQQIARKFLSPLADKDAVGGLGIAKAGNKPRWVR
MREAGEPGWEEEKAKAEARKSTDRTADVLRALADFGLKPLMRVYTDSDMSSVQWKPLRKGQAVRTWDRDMF
QQAIERMMSWESWNQRVGEAYAKLVEQKSRFEQKNFVGQEHLVQLVNQLQQDMKEASHGLESKEQTAHYLTG
RALRGSDKVFEKWEKLDPDAPFDLYDTEIKNVQRRNTRRFGSHDLFAKLAEPKYQALWREDASFLTRYAVYNSIVR
KLNHAKMFATFTLPDATAHPIWTRFDKLGGNLHQYTFLFNEFGEGRHAIRFQKLLTVEDGVAKEVDDVTVPISMSA
QLDDLLPRDPHELVALYFQDYGAEQHLAGEFGGAKIQYRRDQLNHLHARRGARDVYLNLSVRVQSQSEARGERRP
PYAAVFRLVGDNHRAFVHFDKLSDYLAEHPDDGKLGSEGLLSGLRVMSVDLGLRTSASISVFRVARKDELKPNSEGR
VPFCFPIEGNENLVAVHERSQLLKLPGETESKDLRAIREERQRTLRQLRTQLAYLRLLVRCGSEDVGRRERSWAKLIE
QPMDANQMTPDWREAFEDELQKLKSLYGICGDREWTEAVYESVRRVWRHMGKQVRDWRKDVRSGERPKIRG
YQKDVVGGNSIEQIEYLERQYKFLKSWSFFGKVSGQVIRAEKGSRFAITLREHIDHAKEDRLKKLADRIIMEALGYVY
ALDDERGKGKWVAKYPPCQLILLEELSEYQFNNDRPPSENNQLMQWSHRGVFQELLNQAQVHDLLVGTMYAAF
SSRFDARTGAPGIRCRRVPARCAREQNPEPFPWWLNKFVAEHKLDGCPLRADDLIPTGEGEFFVSPFSAEEGDFHQ
IHADLNAAQNLQRRLWSDFDISQIRLRCDWGEVDGEPVLIPRTTGKRTADSYGNKVFYTKTGVTYYERERGKKRRK
VFAQEELSEEEAELLVEADEAREKSVVLMRDPSGIINRGDWTRQKEFWSMVNQRIEGYLVKQIRSRVRLQESACEN
TGDI

**Amino acid sequence WT Cas12d15 (SEQ ID No. 50)**

MNEKKTATQRRNARRRRGERARTKSQELRGYRLHDARIEFSGGLGSMRTVKVELLNPDSSREDPQRGQGLQGKV
AKAVFDDYRALYGPMNIEDYLSDPDCPSFLGLWVKAVCLGVIMSRKTATDFGELRGGSKSGQAFDSIPEHLRRQLIK
LKWLDWYDKGIRKSSSKASRLKSLTDVFANPKQPDQGVMAAWEQGEKLAESSRDIAALGRREFKDKLFAIPPPTSS
VVLDDDVKATKVSRDWQWAVDPQFKLPSTDLDITRALEEVDRQWFERLGNNRGMVQQFFAIGDNGNHLNNGL
FGHFFASIRSANLADIVAEMGTAFGFSAEERDIVRQRLETLHEYAQGLPEKPVLASRWAEYRTDMTAKLGSWYSNR
TSKGAASITQVWGTINTETGEVKDDGLVRTLENIQSDLPDSCSIKEGILQETLDFIGDRRSSTDRAFTDELELYLATLRS
DLNTWCQEQSALWEEKQRQVATPASDEKSKKADNPWAGKGSKTDKWLGALHTRIQSSPLFWGVDKLELWKTLA
NLKQAIRDEIDKLNEQVEVFGRSAYDEPVGKDADSGEGDRRVDQLSYLSARLGDQAHEEVRQRLDAIALALGVKFS
ERDDLHRFFVSSRARRRAALLAMPNTITVGKLRELADLTPLWERIKKKPEEPRLLADTVALSKVVNSACASRANPSD
QIELTTIHSRLDGYSKNIGHTEFISRATVQSTNGAQNTVALDSLVSPRLFYYNFPNIVESAEPHVSHLEVATRGNLGSF
EEFAAKEHRTFDRENPQKDSRNRIDSVNPLAVASSRYQIQFFTWWAGLHRSKETALEVGGSFTIAERQVRLDWSQ
EKPQAVVSEELRVFVSQPFTIVPDDKKRPATSGTRYIGVDIGEYGLAWSCWEFAPGYWNGSVVNPSKVTCLDYGFL
AEPGQRRIVERVKKLRESQATKTFTSPDTYIARLRENVVATYQAQLEALMMAYNAQLVFESEISAFETGGNRVKKIY
DAIKRSSVFGRSDAEATDNNQHWGKNGNRSSVKDPDKLRLNEAGQVAARVPWAEPVSAWMTSQTCSACGRVY
VRAYRGKNSNEPDSGATGEVRYFDNKQQKILTKTIGADTVWVTDQERKEFERGVYNAMRPNAFMPDGRWTAA
GEILEAALKSRGTLDGGRGFAGLHLTSKAQVHEYIEGTGKSHRDAHGNSAIFICPYTDCGHIADADLQASYNIALRGF
AYAIVRKKHPELFAGSGSSTDGDEGGGKKPQQKQAFIDEIVRAAGRAS

**Humanised nucleotide sequence WT AsCas12a (SEQ ID No. 51)**

ATGACACAGTTCGAGGGCTTTACCAACCTGTATCAGGTGAGCAAGACACTGCGGTTTGAGCTGATCCCACAGG
GCAAGACCCTGAAGCACATCCAGGAGCAGGGCTTCATCGAGGAGGACAAGGCCCGCAATGATCACTACAAG
GAGCTGAAGCCCATCATCGATCGGATCTACAAGACCTATGCCGACCAGTGCCTGCAGCTGGTGCAGCTGGATT
GGGAGAACCTGAGCGCCGCCATCGACTCCTATAGAAAGGAGAAAACCGAGGAGACAAGGAACGCCCTGATC
GAGGAGCAGGCCACATATCGCAATGCCATCCACGACTACTTCATCGGCCGGACAGACAACCTGACCGATGCC
ATCAATAAGAGACACGCCGAGATCTACAAGGGCCTGTTCAAGGCCGAGCTGTTTAATGGCAAGGTGCTGAAG
CAGCTGGGCACCGTGACCACAACCGAGCACGAGAACGCCCTGCTGCGGAGCTTCGACAAGTTTACAACCTAC
TTCTCCGGCTTTTATGAGAACAGGAAGAACGTGTTCAGCGCCGAGGATATCAGCACAGCCATCCCACACCGCA
TCGTGCAGGACAACTTCCCCAAGTTTAAGGAGAATTGTCACATCTTCACACGCCTGATCACCGCCGTGCCCAGC
CTGCGGGAGCACTTTGAGAACGTGAAGAAGGCCATCGGCATCTTCGTGAGCACCTCCATCGAGGAGGTGTTT
TCCTTCCCTTTTTATAACCAGCTGCTGACACAGACCCAGATCGACCTGTATAACCAGCTGCTGGGAGGAATCTC
TCGGGAGGCAGGCACCGAGAAGATCAAGGGCCTGAACGAGGTGCTGAATCTGGCCATCCAGAAGAATGATG
AGACAGCCCACATCATCGCCTCCCTGCCACACAGATTCATCCCCCTGTTTAAGCAGATCCTGTCCGATAGGAAC
ACCCTGTCTTTCATCCTGGAGGAGTTTAAGAGCGACGAGGAAGTGATCCAGTCCTTCTGCAAGTACAAGACAC
TGCTGAGAAACGAGAACGTGCTGGAGACAGCCGAGGCCCTGTTTAACGAGCTGAACAGCATCGACCTGACAC
ACATCTTCATCAGCCACAAGAAGCTGGAGACAATCAGCAGCGCCCTGTGCGACCACTGGGATACACTGAGGA
ATGCCCTGTATGAGCGGAGAATCTCCGAGCTGACAGGCAAGATCACCAAGTCTGCCAAGGAGAAGGTGCAGC
GCAGCCTGAAGCACGAGGATATCAACCTGCAGGAGATCATCTCTGCCGCAGGCAAGGAGCTGAGCGAGGCC
TTCAAGCAGAAAACCAGCGAGATCCTGTCCCACGCACACGCCGCCCTGGATCAGCCACTGCCTACAACCCTGA
AGAAGCAGGAGGAGAAGGAGATCCTGAAGTCTCAGCTGGACAGCCTGCTGGGCCTGTACCACCTGCTGGACT
GGTTTGCCGTGGATGAGTCCAACGAGGTGGACCCCGAGTTCTCTGCCCGGCTGACCGGCATCAAGCTGGAGA

TGGAGCCTTCTCTGAGCTTCTACAACAAGGCCAGAAATTATGCCACCAAGAAGCCCTACTCCGTGGAGAAGTT
CAAGCTGAACTTTCAGATGCCTACACTGGCCTCTGGCTGGGACGTGAATAAGGAGAAGAACAATGGCGCCAT
CCTGTTTGTGAAGAACGGCCTGTACTATCTGGGCATCATGCCAAAGCAGAAGGGCAGGTATAAGGCCCTGAG
CTTCGAGCCCACAGAGAAACCAGCGAGGGCTTTGATAAGATGTACTATGACTACTTCCCTGATGCCGCCAAG
ATGATCCCAAAGTGCAGCACCCAGCTGAAGGCCGTGACAGCCCACTTTCAGACCCACACAACCCCCATCCTGC
TGTCCAACAATTTCATCGAGCCTCTGGAGATCACAAAGGAGATCTACGACCTGAACAATCCTGAGAAGGAGCC
AAAGAAGTTTCAGACAGCCTACGCCAAGAAAACCGGCGACCAGAAGGGCTACAGAGAGGCCCTGTGCAAGT
GGATCGACTTCACAAGGGATTTTCTGTCCAAGTATACCAAGACAACCTCTATCGATCTGTCTAGCCTGCGGCCA
TCCTCTCAGTATAAGGACCTGGGCGAGTACTATGCCGAGCTGAATCCCCTGCTGTACCACATCAGCTTCCAGA
GAATCGCCGAGAAGGAGATCATGGATGCCGTGGAGACAGGCAAGCTGTACCTGTTCCAGATCTATAACAAGG
ACTTTGCCAAGGGCCACCACGGCAAGCCTAATCTGCACACTGTATTGGACCGGCCTGTTTTCTCCAGAGAA
CCTGGCCAAGACAAGCATCAAGCTGAATGGCCAGGCCGAGCTGTTCTACCGCCCTAAGTCCAGGATGAAGAG
GATGGCACACCGGCTGGGAGAGAAGATGCTGAACAAGAAGCTGAAGGATCAGAAAACCCCAATCCCCGACA
CCCTGTACCAGGAGCTGTACGACTATGTGAATCACAGACTGTCCCACGACCTGTCTGATGAGGCCAGGGCCCT
GCTGCCCAACGTGATCACCAAGGAGGTGTCTCACGAGATCATCAAGGATAGGCGCTTTACCAGCGACAAGTT
CTTTTTCCACGTGCCTATCACACTGAACTATCAGGCCGCCAATTCCCCATCTAAGTTCAACCAGAGGGTGAATG
CCTACCTGAAGGAGCACCCCGAGACACCTATCATCGGCATCGATCGGGGCGAGAGAAACCTGATCTATATCAC
AGTGATCGACTCCACCGGCAAGATCCTGGAGCAGCGGAGCCTGAACACCATCCAGCAGTTTGATTACCAGAA
GAAGCTGGACAACAGGGAGAAGGAGAGGGTGGCAGCAAGGCAGGCCTGGTCTGTGGTGGGCACAATCAAG
GATCTGAAGCAGGGCTATCTGAGCCAGGTCATCCACGAGATCGTGGACCTGATGATCCACTACCAGGCCGTG
GTGGTGCTGGAGAACCTGAATTTCGGCTTTAAGAGCAAGAGGACCGGCATCGCCGAGAAGGCCGTGTACCA
GCAGTTCGAGAAGATGCTGATCGATAAGCTGAATTGCCTGGTGCTGAAGGACTATCCAGCAGAGAAAGTGGG
AGGCGTGCTGAACCCATACCAGCTGACAGACCAGTTCACCTCCTTTGCCAAGATGGGCACCCAGTCTGGCTTC
CTGTTTTACGTGCCTGCCCCATATACATCTAAGATCGATCCCCTGACCGGCTTCGTGGACCCCTTCGTGTGGAA
AACCATCAAGAATCACGAGAGCCGCAAGCACTTCCTGGAGGGCTTCGACTTTCTGCACTACGACGTGAAAACC
GGCGACTTCATCCTGCACTTTAAGATGAACAGAAATCTGTCCTTCCAGAGGGGCCTGCCCGGCTTTATGCCTGC
ATGGGATATCGTGTTCGAGAAGAACGAGACACAGTTTGACGCCAAGGGCACCCCTTTCATCGCCGGCAAGAG
AATCGTGCCAGTGATCGAGAATCACAGATTCACCGGCAGATACCGGGACCTGTATCCTGCCAACGAGCTGATC
GCCCTGCTGGAGGAGAAGGGCATCGTGTTCAGGGATGGCTCCAACATCCTGCCAAAGCTGCTGGAGAATGAC
GATTCTCACGCCATCGACACCATGGTGGCCCTGATCCGCAGCGTGCTGCAGATGCGGAACTCCAATGCCGCCA
CAGGCGAGGACTATATCAACAGCCCCGTGCGCGATCTGAATGGCGTGTGCTTCGACTCCCGGTTTCAGAACCC
AGAGTGGCCCATGGACGCCGATGCCAATGGCGCCTACCACATCGCCCTGAAGGGCCAGCTGCTGCTGAATCA
CCTGAAGGAGAGCAAGGATCTGAAGCTGCAGAACGGCATCTCCAATCAGGACTGGCTGGCCTACATCCAGGA
GCTGCGCAAC

**Humanised nucleotide sequence WT AaCas12b (SEQ ID No. 52)**

ATGGCCGTGAAGAGCATGAAGGTGAAGCTGCGCCTGGACAACATGCCCGAGATCCGCGCCGGCCTGTGGAA
GCTGCACACCGAGGTGAACGCCGGCGTGCGCTACTACACCGAGTGGCTGAGCCTGCTGCGCCAGGAGAACCT
GTACCGCCGCAGCCCCAACGGCGACGGCGAGCAGGAGTGCTACAAGACCGCCGAGGAGTGCAAGGCCGAGC
TGCTGGAGCGCCTGCGCGCCCGCCAGGTGGAGAACGGCCACTGCGGCCCCGCCGGCAGCGACGACGAGCTG
CTGCAGCTGGCCCGCCAGCTGTACGAGCTGCTGGTGCCCCAGGCCATCGGCGCCAAGGGCGACGCCCAGCAG
ATCGCCCGCAAGTTCCTGAGCCCCCTGGCCGACAAGGACGCCGTGGGCGGCCTGGGCATCGCCAAGGCCGGC
AACAAGCCCCGCTGGGTGCGCATGCGCGAGGCCGGCGAGCCCGGCTGGGAGGAGGAGAAGGCCAAGGCCG

AGGCCCGCAAGAGCACCGACCGCACCGCCGACGTGCTGCGCGCCCTGGCCGACTTCGGCCTGAAGCCCCTGA
TGCGCGTGTACACCGACAGCGACATGAGCAGCGTGCAGTGGAAGCCCCTGCGCAAGGGCCAGGCCGTGCGC
ACCTGGGACCGCGACATGTTCCAGCAGGCCATCGAGCGCATGATGAGCTGGGAGAGCTGGAACCAGCGCGT
GGGCGAGGCCTACGCCAAGCTGGTGGAGCAGAAGAGCCGCTTCGAGCAGAAGAACTTCGTGGGCCAGGAGC
ACCTGGTGCAGCTGGTGAACCAGCTGCAGCAGGACATGAAGGAGGCCAGCCACGGCCTGGAGAGCAAGGA
GCAGACCGCCCACTACCTGACCGGCCGCGCCCTGCGCGGCAGCGACAAGGTGTTCGAGAAGTGGGAGAAGC
TGGACCCCGACGCCCCCTTCGACCTGTACGACACCGAGATCAAGAACGTGCAGCGCCGCAACACCCGCCGCTT
CGGCAGCCACGACCTGTTCGCCAAGCTGGCCGAGCCCAAGTACCAGGCCCTGTGGCGCGAGGACGCCAGCTT
CCTGACCCGCTACGCCGTGTACAACAGCATCGTGCGCAAGCTGAACCACGCCAAGATGTTCGCCACCTTCACC
CTGCCCGACGCCACCGCCCACCCCATCTGGACCCGCTTCGACAAGCTGGGCGGCAACCTGCACCAGTACACCT
TCCTGTTCAACGAGTTCGGCGAGGGCCGCCACGCCATCCGCTTCCAGAAGCTGCTGACCGTGGAGGACGGCG
TGGCCAAGGAGGTGGACGACGTGACCGTGCCCATCAGCATGAGCGCCCAGCTGGACGACCTGCTGCCCCGCG
ACCCCCACGAGCTGGTGGCCCTGTACTTCCAGGACTACGGCGCCGAGCAGCACCTGGCCGGCGAGTTCGGCG
GCGCCAAGATCCAGTACCGCCGCGACCAGCTGAACCACCTGCACGCCCGCCGCGGCGCCCGCGACGTGTACC
TGAACCTGAGCGTGCGCGTGCAGAGCCAGAGCGAGGCCCGCGGCGAGCGCCGCCCCCCCTACGCCGCCGTG
TTCCGCCTGGTGGGCGACAACCACCGCGCCTTCGTGCACTTCGACAAGCTGAGCGACTACCTGGCCGAGCACC
CCGACGACGGCAAGCTGGGCAGCGAGGGCCTGCTGAGCGGCCTGCGCGTGATGAGCGTGGACCTGGGCCTG
CGCACCAGCGCCAGCATCAGCGTGTTCCGCGTGGCCCGCAAGGACGAGCTGAAGCCCAACAGCGAGGGCCG
CGTGCCCTTCTGCTTCCCCATCGAGGGCAACGAGAACCTGGTGGCCGTGCACGAGCGCAGCCAGCTGCTGAA
GCTGCCCGGCGAGACCGAGAGCAAGGACCTGCGCGCCATCCGCGAGGAGCGCCAGCGCACCCTGCGCCAGC
TGCGCACCCAGCTGGCCTACCTGCGCCTGCTGGTGCGCTGCGGCAGCGAGGACGTGGGCCGCCGCGAGCGC
AGCTGGGCCAAGCTGATCGAGCAGCCCATGGACGCCAACCAGATGACCCCCGACTGGCGCGAGGCCTTCGAG
GACGAGCTGCAGAAGCTGAAGAGCCTGTACGGCATCTGCGGCGACCGCGAGTGGACCGAGGCCGTGTACGA
GAGCGTGCGCCGCGTGTGGCGCCACATGGGCAAGCAGGTGCGCGACTGGCGCAAGGACGTGCGCAGCGGC
GAGCGCCCCAAGATCCGCGGCTACCAGAAGGACGTGGTGGGCGGCAACAGCATCGAGCAGATCGAGTACCT
GGAGCGCCAGTACAAGTTCCTGAAGAGCTGGAGCTTCTTCGGCAAGGTGAGCGGCCAGGTGATCCGCGCCG
AGAAGGGCAGCCGCTTCGCCATCACCCTGCGCGAGCACATCGACCACGCCAAGGAGGACCGCCTGAAGAAG
CTGGCCGACCGCATCATCATGGAGGCCCTGGGCTACGTGTACGCCCTGGACGACGAGCGCGGCAAGGGCAA
GTGGGTGGCCAAGTACCCCCCCTGCCAGCTGATCCTGCTGGAGGAGCTGAGCGAGTACCAGTTCAACAACGA
CCGCCCCCCCAGCGAGAACAACCAGCTGATGCAGTGGAGCCACCGCGGCGTGTTCCAGGAGCTGCTGAACCA
GGCCCAGGTGCACGACCTGCTGGTGGGCACCATGTACGCCGCCTTCAGCAGCCGCTTCGACGCCCGCACCGG
CGCCCCCGGCATCCGCTGCCGCCGCGTGCCCGCCCGCTGCGCCCGCGAGCAGAACCCCGAGCCCTTCCCCTGG
TGGCTGAACAAGTTCGTGGCCGAGCACAAGCTGGACGGCTGCCCCCTGCGCGCCGACGACCTGATCCCCACC
GGCGAGGGCGAGTTCTTCGTGAGCCCCTTCAGCGCCGAGGAGGGCGACTTCCACCAGATCCACGCCGACCTG
AACGCCGCCCAGAACCTGCAGCGCCGCCTGTGGAGCGACTTCGACATCAGCCAGATCCGCCTGCGCTGCGAC
TGGGGCGAGGTGGACGGCGAGCCCGTGCTGATCCCCCGCACCACCGGCAAGCGCACCGCCGACAGCTACGG
CAACAAGGTGTTCTACACCAAGACCGGCGTGACCTACTACGAGCGCGAGCGCGGCAAGAAGCGCCGCAAGG
TGTTCGCCCAGGAGGAGCTGAGCGAGGAGGAGGCCGAGCTGCTGGTGGAGGCCGACGAGGCCCGCGAGAA
GAGCGTGGTGCTGATGCGCGACCCCAGCGGCATCATCAACCGCGGCGACTGGACCCGCCAGAAGGAGTTCTG
GAGCATGGTGAACCAGCGCATCGAGGGCTACCTGGTGAAGCAGATCCGCAGCCGCGTGCGCCTGCAGGAGA
GCGCCTGCGAGAACACCGGCGACATC

**Humanised nucleotide sequence WT Cas12d15 (SEQ ID No. 53)**

ATGAACGAAAAGAAGACCGCCACACAGAGAAGGAACGCGCGCAGAAGAAGAGGAGAGAGAGCCAGAACCA
AGAGCCAGGAGTTGAGAGGCTACCGGCTGCACGACGCCAGAATCGAGTTCAGCGGCGGACTCGGCAGCATG
AGAACAGTGAAGGTGGAACTGCTGAACCCCGACAGCTCCAGAGAGGATCCACAGAGAGGCCAGGGCCTGCA
GGGTAAAGTGGCCAAGGCCGTGTTCGACGACTACAGAGCCCTGTACGGCCCTATGAACATCGAGGATTATCT
GAGCGACCCTGACTGCCCTTCTTTTCTGGGCCTGTGGGTTAAGGCCGTTTGTCTGGGCGTGATCATGAGCAGA
AAGACCGCCACCGACTTCGGCGAATTACGCGGAGGCTCTAAGAGCGGACAGGCGTTCGATAGCATTCCCGAG
CACCTGAGAAGACAACTGATCAAACTGAAGTGGCTGGACTGGTACGACAAGGGCATCCGGAAGTCCTCCAGC
AAGGCCTCTCGTCTGAAGTCCCTGACCGACGTGTTCGCCAATCCTAAGCAGCCTGACCAGGGAGTTATGGCCG
CGTGGGAGCAGGGCGAGAAGCTTGCTGAAAGCTCCAGAGACATTGCGGCCCTGGGAAGGCGGGAATTTAAG
GACAAGCTGTTTGCTATCCCTCCACCAACCTCTTCCGTGGTGTTGGATGACGACGTGAAGGCTACCAAGGTGA
GCCGCGATTGGCAGTGGGCCGTGGACCCCCAGTTCAAGCTGCCGAGCACCGACCTGGATATCACCAGAGCCC
TGGAGGAAGTGGACCGGCAGTGGTTCGAACGCCTGGGCAATAACAGAGGCATGGTGCAGCAGTTCTTCGCT
ATCGGCGATAACGGCAACCACCTGAACAACGGCCTGTTCGGCCACTTCTTCGCCTCGATCAGAAGTGCCAACC
TGGCCGATATCGTGGCCGAAATGGGCACCGCCTTTGGTTTTAGCGCTGAGGAAAGAGATATCGTGCGGCAGC
GGCTGGAGACCCTGCACGAGTACGCCCAGGGCCTGCCCGAGAAACCTGTCCTGGCTAGCAGATGGGCCGAGT
ACAGAACAGATATGACAGCCAAGCTGGGCAGTTGGTACAGCAACAGAACCTCCAAGGGCGCCGCATCTATCA
CACAGGTGTGGGGCACAATCAACACCGAGACCGGAGAGGTGAAGGATGATGGACTAGTCAGAACCCTGGAA
AACATCCAGAGCGATTTGCCTGACAGCTGTAGCATTAAAGAAGGCATCCTGCAAGAGACCCTGGACTTCATCG
GAGATAGACGGAGCAGCACAGACAGAGCTTTCACAGACGAACTGGAGCTGTACCTGGCTACCCTACGGAGC
GACCTGAACACCTGGTGCCAGGAGCAGAGCGCCTTGTGGGAGGAAAAGCAGCGGCAGGTCGCTACGCCTGC
CTCTGATGAGAAAAGCAAAAAGGCCGACAATCCATGGGCCGGCAAGGGCTCCAAAACAGATAAGTGGCTGG
GCGCCCTGCATACCCGGATCCAAAGCAGCCCCCTGTTCTGGGGCGTGGACAAGCTGGAGCTGTGGAAGACCC
TGGCCAACCTGAAGCAAGCCATCAGAGACGAGATAGACAAGCTGAACGAGCAGGTGGAGGTGTTCGGGAGA
AGCGCCTATGACGAACCCGTAGGCAAAGATGCGGACTCAGGAGAGGGAGATCGGAGAGTGGACCAGCTGA
GCTACCTGAGTGCCAGACTGGGTGATCAGGCTCATGAAGAGGTCAGACAACGGCTGGACGCCATCGCCCTGG
CCCTCGGCGTGAAGTTCTCCGAGCGGGATGATCTTCACCGGTTTTTCGTGTCTAGCAGAGCCAGACGGCGGGC
TGCCCTGCTGGCGATGCCCAATACCATCACAGTGGGCAAGCTGCGGGAGCTGGCTGACCTGACACCACTGTG
GGAACGGATCAAGAAAAAGCCTGAGGAGCCAAGACTGCTGGCCGATACAGTGGCCCTCAGCAAGGTGGTGA
ACAGCGCTTGTGCCAGCCGGGCTAATCCTAGCGACCAGATCGAGCTTACAACCATCCACTCTCGGCTCGACGG
CTACTCCAAGAACATCGGACACACAGAGTTCATCAGCAGAGCTACGGTCCAGAGCACCAACGGAGCCCAGAA
CACCGTCGCCCTGGACAGCCTGGTGAGCCCCAGACTCTTCTACTACAACTTCCCCAATATCGTGGAAAGCGCA
GAACCCCACGTGTCGCACCTGGAAGTGGCCACAAGAGGCAATCTGGGGTCTTTCGAAGAATTCGCCGCCAAG
GAACACCGGACCTTCGACAGAGAAAACCCTCAGAAAGACTCCAGAAACCGGATCGATAGCGTTAACCCTCTG
GCCGTGGCCAGTAGCCGGTACCAGATCCAGTTCTTCACCTGGTGGGCCGGCCTGCACAGGAGCAAGGAGACA
GCCCTGGAGGTCGGCGGATCTTTTACCATCGCCAACGGCAAGTGCGTCTGGATTGGTCCCAGGAGAAACCC
CAGGCCGTGGTGTCTGAAGAACTGAGGGTGTTCGTGTCTCAACCTTTTACCATCGTGCCTGATGACAAGAAGC
GGCCTGCTACCAGCGGCACCCGGTACATCGGCGTGGACATCGGCGAGTACGGCCTCGCCTGGAGCTGCTGGG
AATTCGCCCCTGGCTACTGGAATGGTTCTGTTGTGAATCCCAGCAAGGTGACATGCCTGGACTACGGATTCCT
GGCCGAACCAGGCCAGAGAAGAATCGTGGAGAGTGAAGAAGCTGAGAGAGAGCCAGGCCACCAAGACA
TTCACCAGCCCTGATACCTACATCGCTCGTCTGAGAGAGAATGTGGTGGCTACATACCAAGCCCAGCTGGAAG
CGCTGATGATGGCCTACAACGCCCAACTGGTGTTCGAGTCCGAGATCTCTGCCTTTGAAACAGGAGGCAACCG
GGTCAAGAAAATCTACGACGCAATTAAAAGGTCTAGCGTGTTTGGCCGCAGCGACGCTGAGGCCACCGACAA
TAACCAGCACTGGGGCAAGAACGGCAACAGAAGCTCCGTGAAGGACCCCGACAAGCTGAGGCTGAATGAGG
CCGGCCAGGTGGCCGCCAGAGTGCCTTGGGCCGAGCCTGTGTCCGCATGGATGACCAGCCAAACATGCAGCG
CCTGCGGCCGGGTGTACGTGAGAGCCTACCGAGGCAAGAACTCTAACGAACCTGATAGCGGCGCTACAGGC

GAAGTGAGATATTTCGACAACAAGCAGCAGAAGATCCTGACCAAGACCATCGGCGCCGACACCGTGTGGGTG
ACCGATCAGGAGAGAAAAGAGTTCGAGCGGGGCGTTTATAACGCCATGCGCCCCAACGCCTTCATGCCTGAC
GGCAGATGGACCGCCGCTGGCGAGATTCTGGAAGCCGCCCTCAAAAGCAGAGGAACCCTGGACGGCGGCAG
AGGCTTCGCTGGACTGCACCTGACATCTAAGGCCCAGGTGCACGAGTACATCGAGGGCACAGGCAAGAGCCA
CCGGGACGCCCACGGCAACTCCGCAATCTTCATCTGCCCTTACACCGACTGCGGCCATATCGCCGACGCCGAC
CTGCAGGCCAGCTACAACATCGCCCTGAGAGGCTTCGCATACGCCATCGTACGGAAGAAGCACCCTGAGCTG
TTCGCAGGATCTGGCAGCTCTACTGACGGCGACGAGGGGGGAGGCAAAAAGCCTCAGCAGAAGCAGGCCTT
TATCGACGAGATTGTCAGAGCCGCCGGCAGAGCCTCC

Substituted amino acids are bold + underlined.

**Humanised nucleotide sequence dCas12a (A2723C/T2724C mutations in bold and underlined) (SEQ ID No. 54)**

ATGACACAGTTCGAGGGCTTTACCAACCTGTATCAGGTGAGCAAGACACTGCGGTTTGAGCTGATCCCACAGG
GCAAGACCCTGAAGCACATCCAGGAGCAGGGCTTCATCGAGGAGGACAAGGCCCGCAATGATCACTACAAG
GAGCTGAAGCCCATCATCGATCGGATCTACAAGACCTATGCCGACCAGTGCCTGCAGCTGGTGCAGCTGGATT
GGGAGAACCTGAGCGCCGCCATCGACTCCTATAGAAAGGAGAAAACCGAGGAGACAAGGAACGCCCTGATC
GAGGAGCAGGCCACATATCGCAATGCCATCCACGACTACTTCATCGGCCGGACAGACAACCTGACCGATGCC
ATCAATAAGAGACACGCCGAGATCTACAAGGGCCTGTTCAAGGCCGAGCTGTTTAATGGCAAGGTGCTGAAG
CAGCTGGGCACCGTGACCACAACCGAGCACGAGAACGCCCTGCTGCGGAGCTTCGACAAGTTTACAACCTAC
TTCTCCGGCTTTTATGAGAACAGGAAGAACGTGTTCAGCGCCGAGGATATCAGCACAGCCATCCCACACCGCA
TCGTGCAGGACAACTTCCCCAAGTTTAAGGAGAATTGTCACATCTTCACACGCCTGATCACCGCCGTGCCCAGC
CTGCGGGAGCACTTTGAGAACGTGAAGAAGGCCATCGGCATCTTCGTGAGCACCTCCATCGAGGAGGTGTTT
TCCTTCCCTTTTTATAACCAGCTGCTGACACAGACCCAGATCGACCTGTATAACCAGCTGCTGGGAGGAATCTC
TCGGGAGGCAGGCACCGAGAAGATCAAGGGCCTGAACGAGGTGCTGAATCTGGCCATCCAGAAGAATGATG
AGACAGCCCACATCATCGCCTCCCTGCCACACAGATTCATCCCCCTGTTTAAGCAGATCCTGTCCGATAGGAAC
ACCCTGTCTTTCATCCTGGAGGAGTTTAAGAGCGACGAGGAAGTGATCCAGTCCTTCTGCAAGTACAAGACAC
TGCTGAGAAACGAGAACGTGCTGGAGACAGCCGAGGCCCTGTTTAACGAGCTGAACAGCATCGACCTGACAC
ACATCTTCATCAGCCACAAGAAGCTGGAGACAATCAGCAGCGCCCTGTGCGACCACTGGGATACACTGAGGA
ATGCCCTGTATGAGCGGAGAATCTCCGAGCTGACAGGCAAGATCACCAAGTCTGCCAAGGAGAAGGTGCAGC
GCAGCCTGAAGCACGAGGATATCAACCTGCAGGAGATCATCTCTGCCGCAGGCAAGGAGCTGAGCGAGGCC
TTCAAGCAGAAAACCAGCGAGATCCTGTCCCACGCACACGCCGCCCTGGATCAGCCACTGCCTACAACCCTGA
AGAAGCAGGAGGAGAAGGAGATCCTGAAGTCTCAGCTGGACAGCCTGCTGGGCCTGTACCACCTGCTGGACT
GGTTTGCCGTGGATGAGTCCAACGAGGTGGACCCCGAGTTCTCTGCCCGGCTGACCGGCATCAAGCTGGAGA
TGGAGCCTTCTCTGAGCTTCTACAACAAGGCCAGAAATTATGCCACCAAGAAGCCCTACTCCGTGGAGAAGTT
CAAGCTGAACTTTCAGATGCCTACACTGGCCTCTGGCTGGGACGTGAATAAGGAGAAGAACAATGGCGCCAT
CCTGTTTGTGAAGAACGGCCTGTACTATCTGGGCATCATGCCAAAGCAGAAGGGCAGGTATAAGGCCCTGAG
CTTCGAGCCCACAGAGAAAACCAGCGAGGGCTTTGATAAGATGTACTATGACTACTTCCCTGATGCCGCCAAG
ATGATCCCAAAGTGCAGCACCCAGCTGAAGGCCGTGACAGCCCACTTTCAGACCCACACAACCCCCATCCTGC
TGTCCAACAATTTCATCGAGCCTCTGGAGATCACAAAGGAGATCTACGACCTGAACAATCCTGAGAAGGAGCC
AAAGAAGTTTCAGACAGCCTACGCCAAGAAAACCGGCGACCAGAAGGGCTACAGAGAGGCCCTGTGCAAGT
GGATCGACTTCACAAGGGATTTTCTGTCCAAGTATACCAAGACAACCTCTATCGATCTGTCTAGCCTGCGGCCA
TCCTCTCAGTATAAGGACCTGGGCGAGTACTATGCCGAGCTGAATCCCCTGCTGTACCACATCAGCTTCCAGA
GAATCGCCGAGAAGGAGATCATGGATGCCGTGGAGACAGGCAAGCTGTACCTGTTCCAGATCTATAACAAGG
ACTTTGCCAAGGGCCACCACGGCAAGCCTAATCTGCACACACTGTATTGGACCGGCCTGTTTTCTCCAGAGAA

CCTGGCCAAGACAAGCATCAAGCTGAATGGCCAGGCCGAGCTGTTCTACCGCCCTAAGTCCAGGATGAAGAG
GATGGCACACCGGCTGGGAGAGAAGATGCTGAACAAGAAGCTGAAGGATCAGAAAACCCCAATCCCCGACA
CCCTGTACCAGGAGCTGTACGACTATGTGAATCACAGACTGTCCCACGACCTGTCTGATGAGGCCAGGGCCCT
GCTGCCCAACGTGATCACCAAGGAGGTGTCTCACGAGATCATCAAGGATAGGCGCTTTACCAGCGACAAGTT
CTTTTTCCACGTGCCTATCACACTGAACTATCAGGCCGCCAATTCCCCATCTAAGTTCAACCAGAGGGTGAATG
CCTACCTGAAGGAGCACCCCGAGACACCTATCATCGGCATCG**CC**GGGGCGAGAGAAACCTGATCTATATCAC
AGTGATCGACTCCACCGGCAAGATCCTGGAGCAGCGGAGCCTGAACACCATCCAGCAGTTTGATTACCAGAA
GAAGCTGGACAACAGGGAGAAGGAGAGGGTGGCAGCAAGGCAGGCCTGGTCTGTGGTGGGCACAATCAAG
GATCTGAAGCAGGGCTATCTGAGCCAGGTCATCCACGAGATCGTGGACCTGATGATCCACTACCAGGCCGTG
GTGGTGCTGGAGAACCTGAATTTCGGCTTTAAGAGCAAGAGGACCGGCATCGCCGAGAAGGCCGTGTACCA
GCAGTTCGAGAAGATGCTGATCGATAAGCTGAATTGCCTGGTGCTGAAGGACTATCCAGCAGAGAAAGTGGG
AGGCGTGCTGAACCCATACCAGCTGACAGACCAGTTCACCTCCTTTGCCAAGATGGGCACCCAGTCTGGCTTC
CTGTTTTACGTGCCTGCCCCATATACATCTAAGATCGATCCCCTGACCGGCTTCGTGGACCCCTTCGTGTGGAA
AACCATCAAGAATCACGAGAGCCGCAAGCACTTCCTGGAGGGCTTCGACTTTCTGCACTACGACGTGAAAACC
GGCGACTTCATCCTGCACTTTAAGATGAACAGAAATCTGTCCTTCCAGAGGGGCCTGCCCGGCTTTATGCCTGC
ATGGGATATCGTGTTCGAGAAGAACGAGACACAGTTTGACGCCAAGGGCACCCCTTTCATCGCCGGCAAGAG
AATCGTGCCAGTGATCGAGAATCACAGATTCACCGGCAGATACCGGGACCTGTATCCTGCCAACGAGCTGATC
GCCCTGCTGGAGGAGAAGGGCATCGTGTTCAGGGATGGCTCCAACATCCTGCCAAAGCTGCTGGAGAATGAC
GATTCTCACGCCATCGACACCATGGTGGCCCTGATCCGCAGCGTGCTGCAGATGCGGAACTCCAATGCCGCCA
CAGGCGAGGACTATATCAACAGCCCCGTGCGCGATCTGAATGGCGTGTGCTTCGACTCCCGGTTTCAGAACCC
AGAGTGGCCCATGGACGCCGATGCCAATGGCGCCTACCACATCGCCCTGAAGGGCCAGCTGCTGCTGAATCA
CCTGAAGGAGAGCAAGGATCTGAAGCTGCAGAACGGCATCTCCAATCAGGACTGGCTGGCCTACATCCAGGA
GCTGCGCAAC

**Humanised nucleotide sequence dCas12b (A1709C mutation in bold and underlined) (SEQ ID No. 55)**

ATGGCCGTGAAGAGCATGAAGGTGAAGCTGCGCCTGGACAACATGCCCGAGATCCGCGCCGGCCTGTGGAA
GCTGCACACCGAGGTGAACGCCGGCGTGCGCTACTACACCGAGTGGCTGAGCCTGCTGCGCCAGGAGAACCT
GTACCGCCGCAGCCCCAACGGCGACGGCGAGCAGGAGTGCTACAAGACCGCCGAGGAGTGCAAGGCCGAGC
TGCTGGAGCGCCTGCGCGCCCGCCAGGTGGAGAACGGCCACTGCGGCCCCGCCGGCAGCGACGACGAGCTG
CTGCAGCTGGCCCGCCAGCTGTACGAGCTGCTGGTGCCCCAGGCCATCGGCGCCAAGGGCGACGCCCAGCAG
ATCGCCCGCAAGTTCCTGAGCCCCCTGGCCGACAAGGACGCCGTGGGCGGCCTGGGCATCGCCAAGGCCGGC
AACAAGCCCGCTGGGTGCGCATGCGCGAGGCCGGCGAGCCCGGCTGGGAGGAGGAGAAGGCCAAGGCCG
AGGCCCGCAAGAGCACCGACCGCACCGCCGACGTGCTGCGCGCCCTGGCCGACTTCGGCCTGAAGCCCCTGA
TGCGCGTGTACACCGACAGCGACATGAGCAGCGTGCAGTGGAAGCCCCTGCGCAAGGGCCAGGCCGTGCGC
ACCTGGGACCGCGACATGTTCCAGCAGGCCATCGAGCGCATGATGAGCTGGGAGAGCTGGAACCAGCGCGT
GGGCGAGGCCTACGCCAAGCTGGTGGAGCAGAAGAGCCGCTTCGAGCAGAAGAACTTCGTGGGCCAGGAGC
ACCTGGTGCAGCTGGTGAACCAGCTGCAGCAGGACATGAAGGAGGCCAGCCACGGCCTGGAGAGCAAGGA
GCAGACCGCCCACTACCTGACCGGCCGCGCCCTGCGCGGCAGCGACAAGGTGTTCGAGAAGTGGGAGAAGC
TGGACCCCGACGCCCCCTTCGACCTGTACGACACCGAGATCAAGAACGTGCAGCGCCGCAACACCCGCCGCTT
CGGCAGCCACGACCTGTTCGCCAAGCTGGCCGAGCCCAAGTACCAGGCCCTGTGGCGCGAGGACGCCAGCTT
CCTGACCCGCTACGCCGTGTACAACAGCATCGTGCGCAAGCTGAACCACGCCAAGATGTTCGCCACCTTCACC
CTGCCCGACGCCACCGCCCACCCCATCTGGACCCGCTTCGACAAGCTGGGCGGCAACCTGCACCAGTACACCT
TCCTGTTCAACGAGTTCGGCGAGGGCCGCCACGCCATCCGCTTCCAGAAGCTGCTGACCGTGGAGGACGGCG
TGGCCAAGGAGGTGGACGACGTGACCGTGCCCATCAGCATGAGCGCCCAGCTGGACGACCTGCTGCCCCGCG

ACCCCCACGAGCTGGTGGCCCTGTACTTCCAGGACTACGGCGCCGAGCAGCACCTGGCCGGCGAGTTCGGCG
GCGCCAAGATCCAGTACCGCCGCGACCAGCTGAACCACCTGCACGCCCGCCGCGGCGCCCGCGACGTGTACC
TGAACCTGAGCGTGCGCGTGCAGAGCCAGAGCGAGGCCCGCGGCGAGCGCCGCCCCCCCTACGCCGCCGTG
TTCCGCCTGGTGGGCGACAACCACCGCGCCTTCGTGCACTTCGACAAGCTGAGCGACTACCTGGCCGAGCACC
CCGACGACGGCAAGCTGGGCAGCGAGGGCCTGCTGAGCGGCCTGCGCGTGATGAGCGTGG**C**CCTGGGCCTG
CGCACCAGCGCCAGCATCAGCGTGTTCCGCGTGGCCCGCAAGGACGAGCTGAAGCCCAACAGCGAGGGCCG
CGTGCCCTTCTGCTTCCCCATCGAGGGCAACGAGAACCTGGTGGCCGTGCACGAGCGCAGCCAGCTGCTGAA
GCTGCCCGGCGAGACCGAGAGCAAGGACCTGCGCGCCATCCGCGAGGAGCGCCAGCGCACCCTGCGCCAGC
TGCGCACCCAGCTGGCCTACCTGCGCCTGCTGGTGCGCTGCGGCAGCGAGGACGTGGGCCGCCGCGAGCGC
AGCTGGGCCAAGCTGATCGAGCAGCCCATGGACGCCAACCAGATGACCCCCGACTGGCGCGAGGCCTTCGAG
GACGAGCTGCAGAAGCTGAAGAGCCTGTACGGCATCTGCGGCGACCGCGAGTGGACCGAGGCCGTGTACGA
GAGCGTGCGCCGCGTGTGGCGCCACATGGGCAAGCAGGTGCGCGACTGGCGCAAGGACGTGCGCAGCGGC
GAGCGCCCCAAGATCCGCGGCTACCAGAAGGACGTGGTGGGCGGCAACAGCATCGAGCAGATCGAGTACCT
GGAGCGCCAGTACAAGTTCCTGAAGAGCTGGAGCTTCTTCGGCAAGGTGAGCGGCCAGGTGATCCGCGCCG
AGAAGGGCAGCCGCTTCGCCATCACCCTGCGCGAGCACATCGACCACGCCAAGGAGGACCGCCTGAAGAAG
CTGGCCGACCGCATCATCATGGAGGCCCTGGGCTACGTGTACGCCCTGGACGACGAGCGCGGCAAGGGCAA
GTGGGTGGCCAAGTACCCCCCCCTGCCAGCTGATCCTGCTGGAGGAGCTGAGCGAGTACCAGTTCAACAACGA
CCGCCCCCCCAGCGAGAACAACCAGCTGATGCAGTGGAGCCACCGCGGCGTGTTCCAGGAGCTGCTGAACCA
GGCCCAGGTGCACGACCTGCTGGTGGGCACCATGTACGCCGCCTTCAGCAGCCGCTTCGACGCCCGCACCGG
CGCCCCCGGCATCCGCTGCCGCCGCGTGCCCGCCCGCTGCGCCCGCGAGCAGAACCCCGAGCCCTTCCCCTGG
TGGCTGAACAAGTTCGTGGCCGAGCACAAGCTGGACGGCTGCCCCCTGCGCGCCGACGACCTGATCCCCACC
GGCGAGGGCGAGTTCTTCGTGAGCCCCTTCAGCGCCGAGGAGGGCGACTTCCACCAGATCCACGCCGACCTG
AACGCCGCCCAGAACCTGCAGCGCCGCCTGTGGAGCGACTTCGACATCAGCCAGATCCGCCTGCGCTGCGAC
TGGGGCGAGGTGGACGGCGAGCCCGTGCTGATCCCCCGCACCACCGGCAAGCGCACCGCCGACAGCTACGG
CAACAAGGTGTTCTACACCAAGACCGGCGTGACCTACTACGAGCGCGAGCGCGGCAAGAAGCGCCGCAAGG
TGTTCGCCCAGGAGGAGCTGAGCGAGGAGGAGGCCGAGCTGCTGGTGGAGGCCGACGAGGCCCGCGAGAA
GAGCGTGGTGCTGATGCGCGACCCCAGCGGCATCATCAACCGCGGCGACTGGACCCGCCAGAAGGAGTTCTG
GAGCATGGTGAACCAGCGCATCGAGGGCTACCTGGTGAAGCAGATCCGCAGCCGCGTGCGCCTGCAGGAGA
GCGCCTGCGAGAACACCGGCGACATC

Humanised nucleotide sequence dCas12d (A2624C/A2912C/G2913C/A3593C mutations in bold and underlined) (SEQ ID No. 56)

ATGAACGAAAAGAAGACCGCCACACAGAGAAGGAACGCGCGCAGAAGAAGAGGAGAGAGAGCCAGAACCA
AGAGCCAGGAGTTGAGAGGCTACCGGCTGCACGACGCCAGAATCGAGTTCAGCGGCGGACTCGGCAGCATG
AGAACAGTGAAGGTGGAACTGCTGAACCCCGACAGCTCCAGAGAGGATCCACAGAGAGGCCAGGGCCTGCA
GGGTAAAGTGGCCAAGGCCGTGTTCGACGACTACAGAGCCCTGTACGGCCCTATGAACATCGAGGATTATCT
GAGCGACCCTGACTGCCCTTCTTTTCTGGGCCTGTGGGTTAAGGCCGTTTGTCTGGGCGTGATCATGAGCAGA
AAGACCGCCACCGACTTCGGCGAATTACGCGGAGGCTCTAAGAGCGGACAGGCGTTCGATAGCATTCCCGAG
CACCTGAGAAGACAACTGATCAAACTGAAGTGGCTGGACTGGTACGACAAGGGCATCCGGAAGTCCTCCAGC
AAGGCCTCTCGTCTGAAGTCCCTGACCGACGTGTTCGCCAATCCTAAGCAGCCTGACCAGGGAGTTATGGCCG
CGTGGGAGCAGGGCGAGAAGCTTGCTGAAAGCTCCAGAGACATTGCGGCCCTGGGAAGGCGGGAATTTAAG
GACAAGCTGTTTGCTATCCCTCCACCAACCTCTTCCGTGGTGTTGGATGACGACGTGAAGGCTACCAAGGTGA
GCCGCGATTGGCAGTGGGCCGTGGACCCCCAGTTCAAGCTGCCGAGCACCGACCTGGATATCACCAGAGCCC
TGGAGGAAGTGGACCGGCAGTGGTTCGAACGCCTGGGCAATAACAGAGGCATGGTGCAGCAGTTCTTCGCT
ATCGGCGATAACGGCAACCACCTGAACAACGGCCTGTTCGGCCACTTCTTCGCCTCGATCAGAAGTGCCAACC

TGGCCGATATCGTGGCCGAAATGGGCACCGCCTTTGGTTTTAGCGCTGAGGAAAGAGATATCGTGCGGCAGC
GGCTGGAGACCCTGCACGAGTACGCCCAGGGCCTGCCCGAGAAACCTGTCCTGGCTAGCAGATGGGCCGAGT
ACAGAACAGATATGACAGCCAAGCTGGGCAGTTGGTACAGCAACAGAACCTCCAAGGGCGCCGCATCTATCA
CACAGGTGTGGGGCACAATCAACACCGAGACCGGAGAGGTGAAGGATGATGGACTAGTCAGAACCCTGGAA
AACATCCAGAGCGATTTGCCTGACAGCTGTAGCATTAAAGAAGGCATCCTGCAAGAGACCCTGGACTTCATCG
GAGATAGACGGAGCAGCACAGACAGAGCTTTCACAGACGAACTGGAGCTGTACCTGGCTACCCTACGGAGC
GACCTGAACACCTGGTGCCAGGAGCAGAGCGCCTTGTGGGAGGAAAAGCAGCGGCAGGTCGCTACGCCTGC
CTCTGATGAGAAAGCAAAAAGGCCGACAATCCATGGGCCGGCAAGGGCTCCAAAACAGATAAGTGGCTGG
GCGCCCTGCATACCCGGATCCAAAGCAGCCCCCTGTTCTGGGGCGTGGACAAGCTGGAGCTGTGGAAGACCC
TGGCCAACCTGAAGCAAGCCATCAGAGACGAGATAGACAAGCTGAACGAGCAGGTGGAGGTGTTCGGGAGA
AGCGCCTATGACGAACCCGTAGGCAAAGATGCGGACTCAGGAGAGGGAGATCGGAGAGTGGACCAGCTGA
GCTACCTGAGTGCCAGACTGGGTGATCAGGCTCATGAAGAGGTCAGACAACGGCTGGACGCCATCGCCCTGG
CCCTCGGCGTGAAGTTCTCCGAGCGGGATGATCTTCACCGGTTTTTCGTGTCTAGCAGAGCCAGACGGCGGGC
TGCCCTGCTGGCGATGCCCAATACCATCACAGTGGGCAAGCTGCGGGAGCTGGCTGACCTGACACCACTGTG
GGAACGGATCAAGAAAAAGCCTGAGGAGCCAAGACTGCTGGCCGATACAGTGGCCCTCAGCAAGGTGGTGA
ACAGCGCTTGTGCCAGCCGGGCTAATCCTAGCGACCAGATCGAGCTTACAACCATCCACTCTCGGCTCGACGG
CTACTCCAAGAACATCGGACACACAGAGTTCATCAGCAGAGCTACGGTCCAGAGCACCAACGGAGCCCAGAA
CACCGTCGCCCTGGACAGCCTGGTGAGCCCCAGACTCTTCTACTACAACTTCCCCAATATCGTGGAAAGCGCA
GAACCCCACGTGTCGCACCTGGAAGTGGCCACAAGAGGCAATCTGGGGTCTTTCGAAGAATTCGCCGCCAAG
GAACACCGGACCTTCGACAGAGAAAACCCTCAGAAAGACTCCAGAAACCGGATCGATAGCGTTAACCCTCTG
GCCGTGGCCAGTAGCCGGTACCAGATCCAGTTCTTCACCTGGTGGGCCGGCCTGCACAGGAGCAAGGAGACA
GCCCTGGAGGTCGGCGGATCTTTTACCATCGCCGAACGGCAAGTGCGTCTGGATTGGTCCCAGGAGAAACCC
CAGGCCGTGGTGTCTGAAGAACTGAGGGTGTTCGTGTCTCAACCTTTTACCATCGTGCCTGATGACAAGAAGC
GGCCTGCTACCAGCGGCACCCGGTACATCGGCGTGG**<u>C</u>**CATCGGCGAGTACGGCCTCGCCTGGAGCTGCTGGG
AATTCGCCCCTGGCTACTGGAATGGTTCTGTTGTGAATCCCAGCAAGGTGACATGCCTGGACTACGGATTCCT
GGCCGAACCAGGCCAGAGAAGAATCGTGGAGAGAGTGAAGAAGCTGAGAGAGAGCCAGGCCACCAAGACA
TTCACCAGCCCTGATACCTACATCGCTCGTCTGAGAGAGAATGTGGTGGCTACATACCAAGCCCAGCTGGAAG
CGCTGATGATGGCCTACAACGCCCAACTGGTGTTCG**<u>CC</u>**TCCGAGATCTCTGCCTTTGAAACAGGAGGCAACCG
GGTCAAGAAAATCTACGACGCAATTAAAAGGTCTAGCGTGTTTGGCCGCAGCGACGCTGAGGCCACCGACAA
TAACCAGCACTGGGGCAAGAACGGCAACAGAAGCTCCGTGAAGGACCCCGACAAGCTGAGGCTGAATGAGG
CCGGCCAGGTGGCCGCCAGAGTGCCTTGGGCCGAGCCTGTGTCCGCATGGATGACCAGCCAAACATGCAGCG
CCTGCGGCCGGGTGTACGTGAGAGCCTACCGAGGCAAGAACTCTAACGAACCTGATAGCGGCGCTACAGGC
GAAGTGAGATATTTCGACAACAAGCAGCAGAAGATCCTGACCAAGACCATCGGCGCCGACACCGTGTGGGTG
ACCGATCAGGAGAGAAAAGAGTTCGAGCGGGGCGTTTATAACGCCATGCGCCCCAACGCCTTCATGCCTGAC
GGCAGATGGACCGCCGCTGGCGAGATTCTGGAAGCCGCCCTCAAAAGCAGAGGAACCCTGGACGGCGGCAG
AGGCTTCGCTGGACTGCACCTGACATCTAAGGCCCAGGTGCACGAGTACATCGAGGGCACAGGCAAGAGCCA
CCGGGACGCCCACGGCAACTCCGCAATCTTCATCTGCCCTTACACCGACTGCGGCCATATCGCCGACGCCG**<u>CC</u>**
CTGCAGGCCAGCTACAACATCGCCCTGAGAGGCTTCGCATACGCCATCGTACGGAAGAAGCACCCTGAGCTG
TTCGCAGGATCTGGCAGCTCTACTGACGGCGACGAGGGGGGAGGCAAAAGCCTCAGCAGAAGCAGGCCTT
TATCGACGAGATTGTCAGAGCCGCCGGCAGAGCCTCC

**Amino acid sequence <u>dAsCas12a (D908A mutation in bold and underlined)</u> (SEQ ID No. 57)**

MTQFEGFTNLYQVSKTLRFELIPQGKTLKHIQEQGFIEEDKARNDHYKELKPIIDRIYKTYADQCLQLVQLDWENLSA
AIDSYRKEKTEETRNALIEEQATYRNAIHDYFIGRTDNLTDAINKRHAEIYKGLFKAELFNGKVLKQLGTVTTTEHENA
LLRSFDKFTTYFSGFYENRKNVFSAEDISTAIPHRIVQDNFPKFKENCHIFTRLITAVPSLREHFENVKKAIGIFVSTSIEE
VFSFPFYNQLLTQTQIDLYNQLLGGISREAGTEKIKGLNEVLNLAIQKNDETAHIIASLPHRFIPLFKQILSDRNTLSFILE
EFKSDEEVIQSFCKYKTLLRNENVLETAEALFNELNSIDLTHIFISHKKLETISSALCDHWDTLRNALYERRISELTGKITK
SAKEKVQRSLKHEDINLQEIISAAGKELSEAFKQKTSEILSHAHAALDQPLPTTLKKQEEKEILKSQLDSLLGLYHLLDW
FAVDESNEVDPEFSARLTGIKLEMEPSLSFYNKARNYATKKPYSVEKFKLNFQMPTLASGWDVNKEKNNGAILFVK
NGLYYLGIMPKQKGRYKALSFEPTEKTSEGFDKMYYDYFPDAAKMIPKCSTQLKAVTAHFQTHTTPILLSNNFIEPLE
ITKEIYDLNNPEKEPKKFQTAYAKKTGDQKGYREALCKWIDFTRDFLSKYTKTTSIDLSSLRPSSQYKDLGEYYAELNPL
LYHISFQRIAEKEIMDAVETGKLYLFQIYNKDFAKGHHGKPNLHTLYWTGLFSPENLAKTSIKLNGQAELFYRPKSRM
KRMAHRLGEKMLNKKLKDQKTPIPDTLYQELYDYVNHRLSHDLSDEARALLPNVITKEVSHEIIKDRRFTSDKFFFHV
PITLNYQAANSPSKFNQRVNAYLKEHPETPIIGI**A**RGERNLIYITVIDSTGKILEQRSLNTIQQFDYQKKLDNREKERVA
ARQAWSVVGTIKDLKQGYLSQVIHEIVDLMIHYQAVVVLENLNFGFKSKRTGIAEKAVYQQFEKMLIDKLNCLVLKD
YPAEKVGGVLNPYQLTDQFTSFAKMGTQSGFLFYVPAPYTSKIDPLTGFVDPFVWKTIKNHESRKHFLEGFDFLHYD
VKTGDFILHFKMNRNLSFQRGLPGFMPAWDIVFEKNETQFDAKGTPFIAGKRIVPVIENHRFTGRYRDLYPANELIA
LLEEKGIVFRDGSNILPKLLENDDSHAIDTMVALIRSVLQMRNSNAATGEDYINSPVRDLNGVCFDSRFQNPEWPM
DADANGAYHIALKGQLLLNHLKESKDLKLQNGISNQDWLAYIQELRN

Catalytically dead AsCas12a described in: Chem. Commun., 2020,56, 2123-2126.

**Amino acid sequence dAaCas12b (D570A <u>mutation in bold and underlined) (SEQ ID No. 58)</u>**

MAVKSMKVKLRLDNMPEIRAGLWKLHTEVNAGVRYYTEWLSLLRQENLYRRSPNGDGEQECYKTAEECKAELLR
LRARQVENGHCGPAGSDDELLQLARQLYELLVPQAIGAKGDAQQIARKFLSPLADKDAVGGLGIAKAGNKPRWVR
MREAGEPGWEEEKAKAEARKSTDRTADVLRALADFGLKPLMRVYTDSDMSSVQWKPLRKGQAVRTWDRDMF
QQAIERMMSWESWNQRVGEAYAKLVEQKSRFEQKNFVGQEHLVQLVNQLQQDMKEASHGLESKEQTAHYLTG
RALRGSDKVFEKWEKLDPDAPFDLYDTEIKNVQRRNTRRFGSHDLFAKLAEPKYQALWREDASFLTRYAVYNSIVR
KLNHAKMFATFTLPDATAHPIWTRFDKLGGNLHQYTFLFNEFGEGRHAIRFQKLLTVEDGVAKEVDDVTVPISMSA
QLDDLLPRDPHELVALYFQDYGAEQHLAGEFGGAKIQYRRDQLNHLHARRGARDVYLNLSVRVQSQSEARGERRP
PYAAVFRLVGDNHRAFVHFDKLSDYLAEHPDDGKLGSEGLLSGLRVMSV**A**LGLRTSASISVFRVARKDELKPNSEGR
VPFCFPIEGNENLVAVHERSQLLKLPGETESKDLRAIREERQRTLRQLRTQLAYLRLLVRCGSEDVGRRERSWAKLIE
QPMDANQMTPDWREAFEDELQKLKSLYGICGDREWTEAVYESVRRVWRHMGKQVRDWRKDVRSGERPKIRG
YQKDVVGGNSIEQIEYLERQYKFLKSWSFFGKVSGQVIRAEKGSRFAITLREHIDHAKEDRLKKLADRIIMEALGYVV
ALDDERGKGKWVAKYPPCQLILLEELSEYQFNNDRPPSENNQLMQWSHRGVFQELLNQAQVHDLLVGTMYAAF
SSRFDARTGAPGIRCRRVPARCAREQNPEPFPWWLNKFVAEHKLDGCPLRADDLIPTGEGEFFVSPFSAEEGDFHQ
IHADLNAAQNLQRRLWSDFDISQIRLRCDWGEVDGEPVLIPRTTGKRTADSYGNKVFYTKTGVTYYERERGKKRRK
VFAQEELSEEEAELLVEADEAREKSVVLMRDPSGIINRGDWTRQKEFWSMVNQRIEGYLVKQIRSRVRLQESACEN
TGDI

Catalytically dead AaCas12b described in: CRISPR-Cas12b enables efficient plant genome engineering. Ming M, Ren Q, Pan C, He Y, Zhang Y, Liu S, Zhong Z, Wang J, Malzahn AA, Wu J, Zheng X, Zhang Y, and Qi Y.. Nature Plants (2020) 10.1038/s41477-020-0614-6

**Amino acid sequence dCas12d15 (D875A/E971A/D1198A <u>mutations in bold and underlined) (SEQ ID No. 59)</u>**

MNEKKTATQRRNARRRRGERARTKSQELRGYRLHDARIEFSGGLGSMRTVKVELLNPDSSREDPQRGQGLQGKV
AKAVFDDYRALYGPMNIEDYLSDPDCPSFLGLWVKAVCLGVIMSRKTATDFGELRGGSKSGQAFDSIPEHLRRQLIK
LKWLDWYDKGIRKSSSKASRLKSLTDVFANPKQPDQGVMAAWEQGEKLAESSRDIAALGRREFKDKLFAIPPPTSS
VVLDDDVKATKVSRDWQWAVDPQFKLPSTDLDITRALEEVDRQWFERLGNNRGMVQQFFAIGDNGNHLNNGL
FGHFFASIRSANLADIVAEMGTAFGFSAEERDIVRQRLETLHEYAQGLPEKPVLASRWAEYRTDMTAKLGSWYSNR
TSKGAASITQVWGTINTETGEVKDDGLVRTLENIQSDLPDSCSIKEGILQETLDFIGDRRSSTDRAFTDELELYLATLRS
DLNTWCQEQSALWEEKQRQVATPASDEKSKKADNPWAGKGSKTDKWLGALHTRIQSSPLFWGVDKLELWKTLA
NLKQAIRDEIDKLNEQVEVFGRSAYDEPVGKDADSGEGDRRVDQLSYLSARLGDQAHEEVRQRLDAIALALGVKFS
ERDDLHRFFVSSRARRRAALLAMPNTITVGKLRELADLTPLWERIKKKPEEPRLLADTVALSKVVNSACASRANPSD
QIELTTIHSRLDGYSKNIGHTEFISRATVQSTNGAQNTVALDSLVSPRLFYYNFPNIVESAEPHVSHLEVATRGNLGSF
EEFAAKEHRTFDRENPQKDSRNRIDSVNPLAVASSRYQIQFFTWWAGLHRSKETALEVGGSFTIAERQVRLDWSQ
EKPQAVVSEELRVFVSQPFTIVPDDKKRPATSGTRYIGV**A**IGEYGLAWSCWEFAPGYWNGSVVNPSKVTCLDYGFL
AEPGQRRIVERVKKLRESQATKTFTSPDTYIARLRENVVATYQAQLEALMMAYNAQLVF**A**SEISAFETGGNRVKKIY
DAIKRSSVFGRSDAEATDNNQHWGKNGNRSSVKDPDKLRLNEAGQVAARVPWAEPVSAWMTSQTCSACGRVY
VRAYRGKNSNEPDSGATGEVRYFDNKQQKILTKTIGADTVWVTDQERKEFERGVYNAMRPNAFMPDGRWTAA
GEILEAALKSRGTLDGGRGFAGLHLTSKAQVHEYIEGTGKSHRDAHGNSAIFICPYTDCGHIADA**A**LQASYNIALRGF
AYAIVRKKHPELFAGSGSSTDGDEGGGKKPQQKQAFIDEIVRAAGRAS

Note: This is a putative catalytically dead Cas12d and has not yet been experimentally validated.

**Bacillus phage PBS2 Uracil-DNA glycosylase inhibitor (SEQ ID No. 60)**

MTNLSDIIEKETGKQLVIQESILMLPEEVEEVIGNKPESDILVHTAYDESTDENVMLLTSDAPEYKPWALVIQDSNGE
NKIKML

**Hybrid Type II crRNA:tracrRNA guide RNA sequence: 5'-(20nt guide) (SEQ ID No. 61)**

GUUUAAGAGCUAUGCUGGAAACAGCAUAGCAAGUUUAAAUAAGGCUAGUCCGUUAUCAACUUGAAAAA
GUGGCACCGAGUCGGUGCUUUUUUU-3'

**RNA scaffold expression cassette (*S. pyogenes*), containing a 20-nucleotide programmable sequence, a CRISPR RNA motif, and an MS2 operator motif (SEQ ID No. 62):**

N$_{20}$<u>GTTTTAGAGCTAGAAATAGCAAGTTAAAATAAGGCTAGTCCGTTATCAACTTGAAAAAGTGGCACCGAGT</u>
<u>CGGTGC</u>**GCGCACATGAGGATCACCCATGTGC***TTTTTTTG*

(N$_{20:}$ programmable sequence; Underlined: CRISPR RNA motif; Bold: MS2 motif; Italic: terminator)

**The above RNA scaffold containing one MS2 loop (1xMS2). Shown below is an RNA scaffold containing two MS2 loops (2xMS2), where MS2 scaffolds are underlined (SEQ ID No. 63)**

GTTTTAGAGCTAGAAATAGCAAGTTAAAATAAGGCTAGTCCGTTATCAACTTGAAAAAGTGGCACCGAGTCG
GTGCgggagc<u>ACATGAGGATCACCCATGT</u>gccacgagcg<u>ACATGAGGATCACCCATGT</u>cgctcgtgttcccTTTTTTTTCT
CCGCT

NLS-Anolis(P16A-E17A)-Apobec1-Linker-MCP **(SEQ ID No. 64)**

AUGGCCCCCAAGAAGAAGCGGAAAGUGAUGGAGCCGGAGGCUUUUCAGCGCAACUUUGACCCUCGGGAA
UUUGCCGCCUGUACACUCCUCUUGUAUGAGAUCCACUGGGACAAUAACACAUCUAGAAAUUGGGUGUAC
GAAUAAGCCUGGGCUCCACGCUGAGGAGAAUUUCUUGCAGAUAUUUAAUGAGAAAAUUGACAUUAAAC
AGGAUACGCCGUGCUCUAUAACAUGGUUCCUUUCUUGGAGCCCCUGUUACCCUUGUAGCCAAGCAAUA
AUAAAAUUCUUGGAGGCACACCCGAAUGUCAGUCUGGAGAUUAAGGCUGCGCGGCUGUAUAUGCAUCA
AAUAGACUGUAACAAGGAGGGACUCAGAAAUCUGGGCCGGAAUCGAGUGUCAAUAAUGAACCUGCCUG
AUUAUAGGCAUUGCUGGACUACGUUUGUUGUGCCAAGGGGAGCAAACGAAGAUUACUGGCCACAAGAC
UUUCUGCCUGCGAUCACAAAUUACUCCCGAGAACUCGACUCCAUACUGCAGGAUGAGCUGAAGACACCC
CUGGGCGACACCACACACCUCUCCACCUUGCCCAGCACCAGAGCUGCUGGGGAGGCCCUAUGGCCAGCA
ACUUCACACAGUUUGUGCUGGUGGAUAAUGGAGGAACCGGCGACGUGACAGUGGCACCAUCUAACUUU
GCCAAUGGCAUCGCCGAGUGGAUCAGCUCCAACUCUCGGAGCCAGGCCUAUAAGGUGACCUGUAGCGUG
CGGCAGUCUAGCGCCCAGAAUAGAAAGUAUACAAUCAAGGUGGAGGUGCCUAAGGGCGCCUGGAGAUC
CUACCUGAACAUGGAGCUGACCAUCCCAAUCUUUGCCACAAAUUCUGAUUGCGAGCUGAUCGUGAAGGC
CAUGCAGGGCCUGCUGAAGGACGGCAACCCUAUCCCAAGCGCCAUCGCCGCCAAUAGCGGAAUCUAC

NLS-Rattus-Apobec1-Linker-MCP **(SEQ ID No. 65)**

AUGGCCCCCAAGAAGAAGCGGAAAGUGUCCUCAGAGACUGGGCCUGUCGCCGUCGAUCCAACCCUGCGC
CGCCGGAUUGAACCUCACGAGUUUGAAGUGUUCUUUGACCCCCGGGAGCUGAGAAAGGAGACAUGCCU
GCUGUACGAGAUCAACUGGGGAGGCAGGCACUCCAUCUGGAGGCACACCUCUCAGAACACAAAUAAGCA
CGUGGAGGUGAACUUCAUCGAGAAGUUUACCACAGAGCGGUACUUCUGCCCCAAUACCAGAUGUAGCA
UCACAUGGUUUCUGAGCUGGUCCCCUUGCGGAGAGUGUAGCAGGGCCAUCACCGAGUUCCUGUCCAGA
UAUCCACACGUGACACUGUUUAUCUACAUCGCCAGGCUGUAUCACCACGCAGACCCAAGGAAUAGGCAG
GGCCUGCGCGAUCUGAUCAGCUCCGGCGUGACCAUCCAGAUCAUGACAGAGCAGGAGUCCGGCUACUGC
UGGCGGAACUUCGUGAAUUAUUCUCCUAGCAACGAGGCCCACUGGCCUAGGUACCCACACCUGUGGGUG
CGCCUGUACGUGCUGGAGCUGUAUUGCAUCAUCCUGGGCCUGCCCCCUUGUCUGAAUAUCCUGCGGAG
AAAGCAGCCCCAGCUGACCUUCUUUACAAUCGCCCUGCAGUCUUGUCACUAUCAGAGGCUGCCACCCCAC
AUCCUGUGGGCCACAGGCCUGAAGGAGCUGAAGACACCCCUGGGCGACACCACACACCUCUCCACCUU
GCCCAGCACCAGAGCUGCUGGGGAGGCCCUAUGGCCAGCAACUUCACACAGUUUGUGCUGGUGGAUAAU
GGAGGAACCGGCGACGUGACAGUGGCACCAUCUAACUUUGCCAAUGGCAUCGCCGAGUGGAUCAGCUCC
AACUCUCGGAGCCAGGCCUAUAAGGUGACCUGUAGCGUGCGGCAGUCUAGCGCCCAGAAUAGAAAGUAU
ACAAUCAAGGUGGAGGUGCCUAAGGGCGCCUGGAGAUCCUACCUGAACAUGGAGCUGACCAUCCCAAUC
UUUGCCACAAAUUCUGAUUGCGAGCUGAUCGUGAAGGCCAUGCAGGGCCUGCUGAAGGACGGCAACCC
UAUCCCAAGCGCCAUCGCCGCCAAUAGCGGAAUCUAC

NLS-Homo-AID-L25-MCP **(SEQ ID No. 66)**

AUGGCCCCCAAGAAGAAGCGGAAAGUGAUGGAUAGCCUGCUGAUGAACCGGAGAAAGUUCCUGUAUCA
GUUUAAGAAUGUGCGCUGGGCAAAGGGCAGGCGCGAGACCUACCUGUGCUAUGUGGUGAAGCGGAGA

GAUUCCGCCACAUCCUUCUCUCUGGACUUUGGCUACCUGCGGAACAAGAAUGGCUGCCACGUGGAGCUG
CUGUUCCUGAGAUACAUCUCUGACUGGGAUCUGGACCCAGGCAGGUGUUAUCGCGUGACCUGGUUCAC
AAGCUGGUCCCCUGCUACGAUUGUGCAAGGCACGUGGCAGACUUUCUGAGGGGAAACCCAAAUCUGUC
CCUGCGGAUCUUCACCGCCAGACUGUAUUUUUGCGAGGAUAGGAAGGCAGAGCCAGAGGGACUGAGGC
GCCUGCACAGGGCCGGCGUGCAGAUCGCCAUCAUGACCUUCAAGGACUACUUUUAUUGUUGGAACACCU
UCGUGGAGAAUCACGAGCGGACCUUCAAGGCCUGGGAGGGACUGCACGAGAACUCCGUGCGGCUGUCU
AGACAGCUGCGGAGAAUCCUGCUGCCUCUGUACGAGGUGGACGAUCUGAGGGAUGCCUUCCGCACCCU
GGGACUGGAGCUGAAGACACCCCUGGGCGACACCACACACACCUCUCCACCUUGCCCAGCACCAGAGCUG
CUGGGAGGCCCUAUGGCCAGCAACUUCACACAGUUUGUGCUGGUGGAUAAUGGAGGAACCGGCGACGU
GACAGUGGCACCAUCUAACUUUGCCAAUGGCAUCGCCGAGUGGAUCAGCUCCAACUCUCGGAGCCAGGC
CUAUAAGGUGACCUGUAGCGUGCGGCAGUCUAGCGCCCAGAAUAGAAAGUAUACAAUCAAGGUGGAGG
UGCCUAAGGGCGCCUGGAGAUCCUACCUGAACAUGGAGCUGACCAUCCCAAUCUUUGCCACAAAUUCUG
AUUGCGAGCUGAUCGUGAAGGCCAUGCAGGGCCUGCUGAAGGACGGCAACCCUAUCCCAAGCGCCAUCG
CCGCCAAUAGCGGAAUCUAC

NLS-nCas9(D10A)-UGI-UGI-NLS **(SEQ ID No. 67)**

AUGCCAAAGAAGAAGCGGAAAGUCGACAAGAAGUACAGCAUCGGCCUGGCCAUCGGCACCAACUCUGUG
GGCUGGGCCGUGAUCACCGACGAGUACAAGGUGCCCAGCAAGAAAUUCAAGGUGCUGGGCAACACCGAC
CGGCACAGCAUCAAGAAGAACCUGAUCGGAGCCCUGCUGUUCGACAGCGGCGAAACAGCCGAGGCCACCC
GGCUGAAGAGAACCGCCAGAAGAAGAUACACCAGACGGAAGAACCGGAUCUGCUAUCUGCAAGAGAUCU
UCAGCAACGAGAUGGCCAAGGUGGACGACAGCUUCUUCCACAGACUGGAAGAGUCCUUCCUGGUGGAA
GAGGAUAAGAAGCACGAGCGGCACCCCAUCUUCGGCAACAUCGUGGACGAGGUGGCCUACCACGAGAAG
UACCCCACCAUCUACCACCUGAGAAAGAAACUGGUGGACAGCACCGACAAGGCCGACCUGCGGCUGAUCU
AUCUGGCCCUGGCCCACAUGAUCAAGUUCCGGGGCCACUUCCUGAUCGAGGGCGACCUGAACCCCGACA
ACAGCGACGUGGACAAGCUGUUCAUCCAGCUGGUGCAGACCUACAACCAGCUGUUCGAGGAAAACCCCA
UCAACGCCAGCGGCGUGGACGCCAAGGCCAUCCUGUCUGCCAGACUGAGCAAGAGCAGACGGCUGGAAA
AUCUGAUCGCCCAGCUGCCCGGCGAGAAGAAGAAUGGCCUGUUCGGAAACCUGAUUGCCCUGAGCCUGG
GCCUGACCCCCAACUUCAAGAGCAACUUCGACCUGGCCGAGGAUGCCAAACUGCAGCUGAGCAAGGACAC
CUACGACGACGACCUGGACAACCUGCUGGCCCAGAUCGGCGACCAGUACGCCGACCUGUUUCUGGCCGCC
AAGAACCUGUCCGACGCCAUCCUGCUGAGCGACAUCCUGAGAGUGAACACCGAGAUCACCAAGGCCCCCC
UGAGCGCCUCUAUGAUCAAGAGAUACGACGAGCACCACCAGGACCUGACCCUGCUGAAAGCUCUCGUGC
GGCAGCAGCUGCCUGAGAAGUACAAAGAGAUUUUCUUCGACCAGAGCAAGAACGGCUACGCCGGCUACA
UUGACGGCGGAGCCAGCCAGGAAGAGUUCUACAAGUUCAUCAAGCCCAUCCUGGAAAAGAUGGACGGCA
CCGAGGAACUGCUCGUGAAGCUGAACAGAGAGGACCUGCUGCGGAAGCAGCGGACCUUCGACAACGGCA
GCAUCCCCCACCAGAUCCACCUGGGAGAGCUGCACGCCAUUCUGCGGCGGCAGGAAGAUUUUUACCCAU
UCCUGAAGGACAACCGGGAAAAGAUCGAGAAGAUCCUGACCUUCCGCAUCCCCUACUACGUGGGCCCUC
UGGCCAGGGGAAACAGCAGAUUCGCCUGGAUGACCAGAAAGAGCGAGGAAACCAUCACCCCCUGGAACU
UCGAGGAAGUGGUGGACAAGGGCGCUUCCGCCCAGAGCUUCAUCGAGCGGAUGACCAACUUCGAUAAG
AACCUGCCCAACGAGAAGGUGCUGCCCAAGCACAGCCUGCUGUACGAGUACUUCACCGUGUAUAACGAG
CUGACCAAAGUGAAAUACGUGACCGAGGGAAUGAGAAAGCCCGCCUUCCUGAGCGGCGAGCAGAAAAAG
GCCAUCGUGGACCUGCUGUUCAAGACCAACCGGAAAGUGACCGUGAAGCAGCUGAAAGAGGACUACUUC
AAGAAAAUCGAGUGCUUCGACUCCGUGGAAAUCUCCGGCGUGGAAGAUCGGUUCAACGCCUCCCUGGGC
ACAUACCACGAUCUGCUGAAAAUUAUCAAGGACAAGGACUUCCUGGACAAUGAGGAAACGAGGACAUU
CUGGAAGAUAUCGUGCUGACCCUGACACUGUUUGAGGACAGAGAGAUGAUCGAGGAACGGCUGAAAAC
CUAUGCCCACCUGUUCGACGACAAAGUGAUGAAGCAGCUGAAGCGGCGGAGAUACACCGGCUGGGGCAG

GCUGAGCCGGAAGCUGAUCAACGGCAUCCGGGACAAGCAGUCCGGCAAGACAAUCCUGGAUUUCCUGAA
GUCCGACGGCUUCGCCAACAGAAACUUCAUGCAGCUGAUCCACGACGACAGCCUGACCUUUAAAGAGGA
CAUCCAGAAAGCCCAGGUGUCCGGCCAGGGCGAUAGCCUGCACGAGCACAUUGCCAAUCUGGCCGGCAG
CCCCGCCAUUAAGAAGGGCAUCCUGCAGACAGUGAAGGUGGUGGACGAGCUCGUGAAAGUGAUGGGCC
GGCACAAGCCCGAGAACAUCGUGAUCGAAAUGGCCAGAGAGAACCAGACCACCCAGAAGGGACAGAAGAA
CAGCCGCGAGAGAAUGAAGCGGAUCGAAGAGGGCAUCAAAGAGCUGGGCAGCCAGAUCCUGAAAGAACA
CCCCGUGGAAAACACCCAGCUGCAGAACGAGAAGCUGUACCUGUACUACCUGCAGAAUGGGCGGGAUAU
GUACGUGGACCAGGAACUGGACAUCAACCGGCUGUCCGACUACGAUGUGGACCAUAUCGUGCCUCAGAG
CUUUCUGAAGGACGACUCCAUCGACAACAAGGUGCUGACCAGAAGCGACAAGAACCGGGGCAAGAGCGA
CAACGUGCCCUCCGAAGAGGUCGUGAAGAAGAUGAAGAACUACUGGCGGCAGCUGCUGAACGCCAAGCU
GAUUACCCAGAGAAAGUUCGACAAUCUGACCAAGGCCGAGAGAGGCGGCCUGAGCGAACUGGAUAAGGC
CGGCUUCAUCAAGAGACAGCUGGUGGAAACCCGGCAGAUCACAAAGCACGUGGCACAGAUCCUGGACUC
CCGGAUGAACACUAAGUACGACGAGAAUGACAAGCUGAUCCGGGAAGUGAAAGUGAUCACCCUGAAGUC
CAAGCUGGUGUCCGAUUUCCGGAAGGAUUUCCAGUUUUACAAAGUGCGCGAGAUCAACAACUACCACCA
CGCCCACGACGCCUACCUGAACGCCGUCGUGGGAACCGCCCUGAUCAAAAAGUACCCUAAGCUGGAAAGC
GAGUUCGUGUACGGCGACUACAAGGUGUACGACGUGCGGAAGAUGAUCGCCAAGAGCGAGCAGGAAAU
CGGCAAGGCUACCGCCAAGUACUUCUUCUACAGCAACAUCAUGAACUUUUUCAAGACCGAGAUUACCCU
GGCCAACGGCGAGAUCCGGAAGCGGCCUCUGAUCGAGACAAACGGCGAAACCGGGGAGAUCGUGUGGGA
UAAGGGCCGGGAUUUUGCCACCGUGCGGAAAGUGCUGAGCAUGCCCCAAGUGAAUAUCGUGAAAAAGA
CCGAGGUGCAGACAGGCGGCUUCAGCAAAGAGUCUAUCCUGCCCAAGAGGAACAGCGAUAAGCUGAUCG
CCAGAAAGAAGGACUGGGACCCUAAGAAGUACGGCGGCUUCGACAGCCCCACCGUGGCCUAUUCUGUGC
UGGUGGUGGCCAAAGUGGAAAAGGGCAAGUCCAAGAAACUGAAGAGUGUGAAAGAGCUGCUGGGGAUC
ACCAUCAUGGAAAGAAGCAGCUUCGAGAAGAAUCCCAUCGACUUUCUGGAAGCCAAGGGCUACAAAGAA
GUGAAAAAGGACCUGAUCAUCAAGCUGCCUAAGUACUCCCUGUUCGAGCUGGAAAACGGCCGGAAGAGA
AUGCUGGCCUCUGCCGGCGAACUGCAGAAGGGAAACGAACUGGCCCUGCCCUCCAAAUAUGUGAACUUC
CUGUACCUGGCCAGCCACUAUGAGAAGCUGAAGGGCUCCCCCGAGGAUAAUGAGCAGAAACAGCUGUUU
GUGGAACAGCACAAGCACUACCUGGACGAGAUCAUCGAGCAGAUCAGCGAGUUCUCCAAGAGAGUGAUC
CUGGCCGACGCUAAUCUGGACAAAGUGCUGUCCGCCUACAACAAGCACCGGGAUAAGCCCAUCAGAGAG
CAGGCCGAGAAUAUCAUCCACCUGUUUACCCUGACCAAUCUGGGAGCCCCUGCCGCCUUCAAGUACUUU
GACACCACCAUCGACCGGAAGAGGUACACCAGCACCAAAGAGGUGCUGGACGCCACCCUGAUCCACCAGA
GCAUCACCGGCCUGUACGAGACACGGAUCGACCUGUCUCAGCUGGGAGGUGACAGCGGCGGGAGCGGCG
GGAGCGGGGGGAGCACUAAUCUGAGCGACAUCAUUGAGAAGGAGACUGGGAAACAGCUGGUCAUUCAG
GAGUCCAUCCUGAUGCUGCCUGAGGAGGUGGAGGAAGUGAUCGGCAACAAGCCAGAGUCUGACAUCCU
GGUGCACACCGCCUACGACGAGUCCACAGAUGAGAAUGUGAUGCUGCUGACCUCUGACGCCCCCGAGUA
UAAGCCUUGGGCCCUGGUCAUCCAGGAUUCUAACGGCGAGAAUAAGAUCAAGAUGCUGAGCGGAGGAU
CCGGAGGAUCUGGAGGCAGCACCAACCUGUCUGACAUCAUCGAGAAGGAGACAGGCAAGCAGCUGGUCA
UCCAGGAGAGCAUCCUGAUGCUGCCCGAAGAAGUCGAAGAAGUGAUCGGAAACAAGCCUGAGAGCGAUA
UCCUGGUCCAUACCGCCUACGACGAGAGUACCGACGAAAAUGUGAUGCUGCUGACAUCCGACGCCCCAG
AGUAUAAGCCCUGGGCUCUGGUCAUCCAGGAUUCCAACGGAGAGAACAAAAUCAAAAUGCUGUCUGGCG
GCUCAAAAAGAACCGCCGACGGCAGCGAAUUCGAGCCCAAGAAGAAGAGGAAAGUC

Qbeta phage operator stem-loop **(SEQ ID No. 68)**
5'- ATGCTGTCTAAGACAGCAT -3'

Qbeta coat protein [Q65H] **(SEQ ID No. 69)**

MAKLETVTLGNIGKDGKQTLVLNPRGVNPTNGVASLSQAGAVPALEKRVTVSVSQPSRNRKNYKVHVKIQNPTAC

TANGSCDPSVTRQAYADVTFSFTQYSTDEERAFVRTELAALLASPLLIDAIDQLNPAY

BoxB **(SEQ ID No. 70)**
5'- GCCCTGAAGAAGGGC -3'

Lambda bacteriophage protein N (LambdaN-(1-22) **(SEQ ID No. 71)**
MNARTRRRERRAEKQAQWKAAN

Csy4 binding motif **(SEQ ID No. 72)**
5'- CTGCCGTATAGGCAGC -3'

Csy4[H29A] **(SEQ ID No. 73)**

MDHYLDIRLRPDPEFPPAQLMSVLFGKLAQALVAQGGDRIGVSFPDLDESRSRLGERLRIHASADDLRALLARPWL

EGLRDHLQFGEPAVVPHPTPYRQVSRVQAKSNPERLRRRLMRRHDLSEEEARKRIPDTVARALDLPFVTLRSQSTG

QHFRLFIRHGPLQVTAEEGGFTCYGLSKGGFVPWF

## Claims

1. A method for genetically modifying a cell by base editing, the method comprising introduction into the cell and/or expression in the cell of:

   i) a sequence-targeting protein;
   ii) an RNA-ligand binding complex comprising

      a) a guide RNA, and
      b) a ligand binding moiety; and

   iii) two or more effector proteins each comprising

      c) a ligand capable of reversibly binding to the ligand binding moiety, and
      d) an effector domain,

   wherein the two or more effector proteins each comprise different effector domains.

2. The method according to claim 1, wherein the sequence-targeting protein is a Cas protein.

3. The method according to any preceding claim, wherein the ligand binding moiety is an RNA motif and the ligand capable of binding to the ligand binding moiety is an RNA binding domain that recognizes the RNA motif.

4. The method according to any preceding claim, wherein the cell undergoes base editing at a single genomic locus or at two or more genomic loci.

5. The method according to any preceding claim, wherein the sequence-targeting protein is a Type II Cas protein or a Type V Cas protein.

6. The method according to any preceding claim, wherein the sequence-targeting protein is a Type II Cas protein that is nuclease null or has nickase activity or a Type V Cas protein that is nuclease null or has nickase activity.

7. The method according to any one of claims 3 to 6, wherein the RNA motif and the RNA binding domain are a pair selected from the group consisting of:

   a telomerase Ku binding motif and Ku protein or an RNA-binding section thereof,

a telomerase Sm7 binding motif and Sm7 protein or an RNA-binding section thereof,
a MS2 phage operator stem-loop and MS2 coat protein (MCP) or an RNA-binding section thereof,
a PP7 phage operator stem-loop and PP7 coat protein (PCP) or an RNA-binding section thereof,
a BoxB binding motif and a Lambda bacteriophage protein N (LambdaN-(1-22)) or an RNA-binding section thereof,
a Csy4 binding motif and a Csy4[H29A] or an RNA-binding section thereof; or
a Qbeta phage operator stem-loop and a Qbeta coat protein [Q65H] or an RNA-binding section thereof.

8. The method according to any preceding claim, wherein the effector domain is selected from the group consisting of an enzyme or a protein fragment.

9. The method according to claim 8, wherein the effector domain has cytidine or adenosine deamination activity.

10. The method according to any preceding claim, wherein the genetic modification involves at least two genetic mutations within the same genetic locus.

11. The method according to any preceding claim, wherein the RNA-ligand binding complex comprises one or more ligand binding moieties, and wherein the one or more ligand binding moieties are RNA motif(s).

12. The method according to any one of claims 3 to 11, wherein at least one RNA motif is an MS2 aptamer or a PP7 aptamer, optionally wherein the MS2 aptamer has an extended stem.

13. The method according to any preceding claim, wherein the sequence targeting protein comprises nCas9 with one or two uracil DNA glycosylase (UNG) inhibitor peptide(s) (UGIs) and the ligand binding moiety is a single MS2 RNA motif or a PP7 RNA motif, wherein the ligand binding moiety is located at the 3' end of the RNA-ligand binding complex.

14. A kit for genetically modifying a cell by base editing comprising:

i) a sequence-targeting protein;
ii) an RNA-ligand binding complex comprising

a) a guide RNA, and
b) a ligand binding moiety; and

iii) two or more effector proteins each comprising

c) a ligand capable of reversibly binding to the ligand binding moiety, and
d) an effector domain,

wherein the two or more effector proteins each comprise different effector domains.

**Patentansprüche**

1. Verfahren zum genetischen Modifizieren einer Zelle durch Basen-Editieren, das Verfahren umfassend die Einführung in die Zelle und/oder die Expression in der Zelle von:

i) einem Sequenz-Targeting-Protein;
ii) einem RNA-Liganden-Bindungskomplex, umfassend

a) eine Führungs-RNA und
b) eine Liganden-Bindungsgruppe; und

iii) zwei oder mehr Effektorproteinen, jeweils umfassend

c) einen Liganden, der reversibel an die Liganden-Bindungsgruppe binden kann, und
d) eine Effektordomäne,

wobei die zwei oder mehr Effektorproteine jeweils unterschiedliche Effektordomänen umfassen.

2. Verfahren gemäß Anspruch 1, wobei das Sequenz-Targeting-Protein ein Cas-Protein ist.

3. Verfahren gemäß irgendeinem der vorhergehenden Ansprüche, wobei die Liganden-Bindungsgruppe ein RNA-Motiv ist und der Ligand, der an die Liganden-Bindungsgruppe binden kann, eine RNA-Bindungsdomäne ist, die das RNA-Motiv erkennt.

4. Verfahren gemäß irgendeinem der vorhergehenden Ansprüche, wobei die Zelle an einem einzelnen genomischen Locus oder an zwei oder mehr genomischen Loci einer Basen-Editierung unterzogen wird.

5. Verfahren gemäß irgendeinem der vorhergehenden Ansprüche, wobei das Sequenz-Targeting-Protein ein Typ-II-Cas-Protein oder ein Typ-V-Cas-Protein ist.

6. Verfahren gemäß irgendeinem der vorhergehenden Ansprüche, wobei das Sequenz-Targeting-Protein ein Typ-II-Cas-Protein ist, das Nuklease-Null ist oder Nickase-Aktivität hat, oder ein Typ-V-Cas-Protein ist, das Nuklease-Null ist oder Nickase-Aktivität hat.

7. Verfahren gemäß irgendeinem der Ansprüche 3 bis 6, wobei das RNA-Motiv und die RNA-Bindungsdomäne ein Paar sind, das aus der Gruppe ausgewählt ist, bestehend aus:

   einem Telomerase-Ku-Bindungsmotiv und Ku-Protein oder einer RNA-bindenden Sektion davon,
   ein Telomerase-Sm7-Bindungsmotiv und Sm7-Protein oder einer RNA-bindenden Sektion davon,
   eine MS2-Phagen-Operator-Stammschleife und MS2-Hüllprotein (MCP) oder einer RNA-bindenden Sektion davon,
   eine PP7-Phagen-Operator-Stammschleife und PP7-Hüllprotein (PCP) oder einer RNA-bindenden Sektion davon,
   ein BoxB-Bindungsmotiv und ein Lambda-Bakteriophagen-Protein N (LambdaN (1-22)) oder einer RNA-bindenden Sektion davon, einem Csy4-Bindungsmotiv und einem Csy4[H29A] oder einer RNA-bindenden Sektion davon; oder
   einer Qbeta-Phagen-Operator-Stammschleife und einem Qbeta-Hüllprotein [Q65H] oder einer RNA-bindenden Sektion davon.

8. Verfahren gemäß irgendeinem der vorhergehenden Ansprüche, wobei die Effektordomäne aus der Gruppe bestehend aus einem Enzym oder einem Proteinfragment ausgewählt ist.

9. Verfahren gemäß Anspruch 8, wobei die Effektor-Domäne Cytidin- oder Adenosin-Desaminierungsaktivität aufweist.

10. Verfahren gemäß irgendeinem der vorhergehenden Ansprüche, wobei die genetische Modifikation mindestens zwei genetische Mutationen innerhalb desselben genetischen Locus umfasst.

11. Verfahren gemäß irgendeinem der vorhergehenden Ansprüche, wobei der RNA-Ligand-Bindungskomplex eine oder mehrere Ligandenbindungskomponenten umfasst und wobei die eine oder die mehreren Ligandenbindungskomponenten ein oder mehrere RNA-Motive sind.

12. Verfahren gemäß irgendeinem der Ansprüche 3 bis 11, wobei mindestens ein RNA-Motiv ein MS2-Aptamer oder ein PP7-Aptamer ist, optional wobei das MS2-Aptamer einen verlängerten Stamm hat.

13. Verfahren gemäß irgendeinem der vorhergehenden Ansprüche, wobei das Sequenz-Targeting-Protein nCas9 mit einem oder zwei Uracil-DNA-Glykosylase (UNG)-Inhibitorpeptid(en) (UGIs) umfasst und die Liganden-Bindungsgruppe ein einzelnes MS2-RNA-Motiv oder ein PP7-RNA-Motiv ist, wobei sich die Liganden-Bindungsgruppe am 3'-Ende des RNA-Ligand-Bindungskomplexes befindet.

14. Kit zum genetischen Modifizieren einer Zelle durch Basen-Editierung, umfassend:

   i) ein Sequenz-Targeting-Protein;
   ii) einen RNA-Ligand-Bindungskomplex, umfassend

a) eine Führungs-RNA und
b) eine Liganden-Bindungsgruppe; und

iii) zwei oder mehr Effektorproteine, jeweils umfassend

c) einen Liganden, der reversibel an die Liganden-Bindungsgruppe binden kann, und
d) eine Effektordomäne

umfassen, wobei die zwei oder mehr Effektorproteine jeweils unterschiedliche Effektordomänen umfassen.

**Revendications**

1. Procédé de modification génétique d'une cellule par correction génomique, le procédé comprenant l'introduction dans la cellule et/ou l'expression dans la cellule de :

i) une protéine de ciblage de séquence ;
ii) un complexe de liaison ARN-ligand comprenant

a) un ARN guide, et
b) un fragment de liaison au ligand ; et

iii) deux protéines effectrices, ou plus, comprenant chacune

c) un ligand apte à se lier de manière réversible au fragment de liaison au ligand, et
d) un domaine effecteur,

dans lequel les deux protéines effectrices, ou plus, comprennent chacune des domaines effecteurs différents.

2. Procédé selon la revendication 1, dans lequel la protéine de ciblage de séquence est une protéine Cas.

3. Procédé selon une quelconque revendication précédente, dans lequel le fragment de liaison au ligand est un motif d'ARN et le ligand apte à se lier au fragment de liaison au ligand est un domaine de liaison à l'ARN qui reconnaît le motif d'ARN.

4. Procédé selon une quelconque revendication précédente, dans lequel la cellule subit une correction génomique en un seul locus génomique ou en deux loci génomiques, ou plus.

5. Procédé selon une quelconque revendication précédente, dans lequel la protéine de ciblage de séquence est une protéine Cas de type II ou une protéine Cas de type V.

6. Procédé selon une quelconque revendication précédente, dans lequel la protéine de ciblage de séquence est une protéine Cas de type II sans nucléase ou qui présente une activité nickase ou une protéine Cas de type V sans nucléase ou qui présente une activité nickase.

7. Procédé selon l'une quelconque des revendications 3 à 6, dans lequel le motif d'ARN et le domaine de liaison à l'ARN sont une paire sélectionnée dans le groupe consistant en :

un motif de liaison de Ku et de télomérase et une protéine Ku ou une section de liaison à l'ARN de celle-ci,
un motif de liaison de Sm7 et de télomérase et une protéine Sm7 ou une section de liaison à l'ARN de celle-ci,
une tige-boucle d'opérateur de phage MS2 et une protéine d'enveloppe MS2 (MCP) ou une section de liaison à l'ARN de celle-ci,
une tige-boucle d'opérateur de phage PP7 et une protéine d'enveloppe PP7 (PCP) ou une section de liaison à l'ARN de celle-ci,
un motif de liaison BoxB et une protéine N de bactériophage lambda (LambdaN-(1-22)) ou une section de liaison à l'ARN de celle-ci,
un motif de liaison Csy4 et une Csy4[H29A] ou une section de liaison à l'ARN de celle-ci ; ou
une tige-boucle d'opérateur de phage Qbêta et une protéine d'enveloppe Qbêta [Q65H] ou une section de liaison à l'ARN de celle-ci.

8. Procédé selon une quelconque revendication précédente, dans lequel le domaine effecteur est sélectionné dans le groupe consistant en une enzyme ou un fragment de protéine.

9. Procédé selon la revendication 8, dans lequel le domaine effecteur présente une activité de désamination par cytidine ou adénosine.

10. Procédé selon une quelconque revendication précédente, dans lequel la modification génétique implique au moins deux mutations génétiques au sein du même locus génétique.

11. Procédé selon une quelconque revendication précédente, dans lequel le complexe de liaison ARN-ligand comprend un ou plusieurs fragments de liaison au ligand, et dans lequel les un ou plusieurs fragments de liaison au ligand sont un ou des motifs d'ARN.

12. Procédé selon l'une quelconque des revendications 3 à 11, dans lequel au moins un motif d'ARN est un aptamère MS2 ou un aptamère PP7, éventuellement dans lequel l'aptamère MS2 présente une tige allongée.

13. Procédé selon une quelconque revendication précédente, dans lequel la protéine de ciblage de séquence comprend une nCas9 avec un ou plusieurs peptides inhibiteurs de l'uracile ADN glycosylase (UNG) (UGI) et le fragment de liaison au ligand est un motif d'ARN MS2 unique ou un motif d'ARN PP7, dans lequel le fragment de liaison au ligand est situé à l'extrémité 3' du complexe de liaison ARN-ligand.

14. Kit de modification génétique d'une cellule par correction génomique comprenant :

   i) une protéine de ciblage de séquence ;
   ii) un complexe de liaison ARN-ligand comprenant

      a) un ARN guide, et
      b) un fragment de liaison au ligand ; et

   iii) deux protéines effectrices, ou plus, comprenant chacune

      c) un ligand apte à se lier de manière réversible au fragment de liaison au ligand, et
      d) un domaine effecteur,

   dans lequel les deux protéines effectrices, ou plus, comprennent chacune des domaines effecteurs différents.

Figure 1

Figure adapted from: Mir, A., Alterman, J.F., Hassler, M.R. *et al.* Heavily and fully modified RNAs guide efficient SpyCas9-mediated genome editing. *Nat Commun* **9**, 2641 (2018).

Figure 2

Figure 3

Figure 4

Figure 5

Figure 6

EP 4 274 893 B1

| | C | A | C | C | T | A | C | C | T | A | A | G | A | A | C | C | A | T | C | C |
|---|---|---|---|---|---|---|---|---|---|---|---|---|---|---|---|---|---|---|---|---|
| Apobec1-MCP-only | 0 | 0 | 0 | 21 | 0 | 0 | 45 | 49 | 0 | 0 | 0 | 0 | 0 | 0 | 0 | 0 | 0 | 0 | 0 | 0 |
| AID-MCP-only | 0 | 0 | 22 | 26 | 0 | 0 | 24 | 28 | 0 | 0 | 0 | 0 | 0 | 0 | 0 | 0 | 0 | 0 | 0 | 0 |
| Apobec1-MCP + AID-MCP | 0 | 0 | 20 | 28 | 0 | 0 | 44 | 48 | 0 | 0 | 0 | 0 | 0 | 0 | 0 | 0 | 0 | 0 | 0 | 0 |
| Neither Deaminase | 0 | 0 | 0 | 4 | 0 | 0 | 0 | 4 | 0 | 0 | 0 | 0 | 0 | 0 | 0 | 0 | 0 | 0 | 0 | 0 |

100
80
60
40
20
0

Figure 7

Figure 8

Figure 8 continued

Figure 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017011721 A
- WO 2018129129 A
- US 62901584 B
- US 20140273233 A
- WO 2013176772 A
- WO 2014099750 A
- US 20140179006 A
- US 20140273226 A
- WO 2014144592 A
- US 8202983 B
- WO 2014144761 A
- US 5489677 A

- US 5602240 A
- US 5034506 A
- US 3687808 A
- US 10113163 B
- US 4625014 A
- US 5057301 A
- US 5514363 A
- US 20150182596 A
- US 20100063258 A
- WO 2012142515 A
- US 6165476 A
- US 5856456 A

**Non-patent literature cited in the description**

- **KOONIN ; MAKAROVA.** origins and evolution of crispr cas systems. *Review Philos Trans R Soc Lond B Biol Sci,* 13 May 2019, vol. 374, 1772
- **TYPE-V YAN et al.** *Science,* 2019, vol. 363, 88-92
- **HARRINGTON et al.** *Science,* 2018, vol. 362, 839-842
- **CREIGHTON.** Proteins: Structures and Molecular Principles. W.H. Freeman & Co, 1983
- **FREDERICK M. AUSUBEL et al.** Current Protocols in Molecular Biology. John Wiley & Sons, 2003
- **SAMBROOK et al.** Molecular Cloning, A Laboratory Manual. Cold Spring Harbor Press, 2001
- *Science,* 16 September 2016, vol. 353 (6305
- **RAUCH et al.** Programmable RNA-Guided RNA Effector Proteins Built from Human Parts. *Cell,* 27 June 2019, vol. 178 (1), 122-134.e12
- **WANG, J ; ZHANG, C ; FENG, B.** The rapidly advancing Class 2 CRISPR-Cas technologies: a customizable toolbox for molecular manipulations. *J. Cell. Mol. Med,* 2020, vol. 24, 3256-3270
- **MAKAROVA, K.S ; WOLF, Y.I ; IRANZO, J et al.** Evolutionary classification of CRISPR-Cas systems: a burst of class 2 and derived variants. *Nat Rev Microbiol,* 2020, vol. 18, 67-83
- **TONG, B et al.** The versatile type V CRISPR effectors and their application prospects. Front. *Cell Dev. Biol,* 2021, vol. 8, 1835
- **WANG et al.** Uracil-DNA glycosylase inhibitor gene of bacteriophage PBS2 encodes a binding protein specific for uracil-DNA glycosylase. *J Biol. Chem.,* 1989, vol. 264, 1163-1171

- **LUNDQUIST et al.** Site-directed mutagenesis and characterization of uracil-DNA glycosylase inhibitor protein. Role of specific carboxylic amino acids in complex formation with Escherichia coli uracil-DNA glycosylase. *J Biol. Chem.,* 1997, vol. 272, 21408-21419
- **RAVISHANKAR et al.** X-ray analysis of a complex of Escherichia coli uracil DNA glycosylase (EcUDG) with a proteinaceous inhibitor. The structure elucidation of a prokaryotic UDG. *Nucleic Acids Res.,* 1998, vol. 26, 4880-4887
- **PUTNAM et al.** Protein mimicry of DNA from crystal structures of the uracil-DNA glycosylase inhibitor protein and its complex with Escherichia coli uracil-DNA glycosylase. *J Mol. Biol.,* 1999, vol. 287, 331-346
- **CONG et al.** *Science,* 2012, vol. 339 (6121), 819-823
- **JINEK et al.** *Science,* 2012, vol. 337 (6096), 816-821
- **MALI et al.** *Science,* 2013, vol. 339 (6121), 823-826
- **GASIUNAS et al.** *Proc Natl Acad Sci USA.,* 2012, vol. 109 (39), E2579-E2586
- **CHO et al.** *Nature Biotechnology,* vol. 31, 230-232
- **HOU et al.** *Proc Natl Acad Sci USA.,* 24 September 2013, vol. 110 (39), 15644-9
- **MOJICA et al.** *Microbiology,* March 2009, vol. 155, 733-40, www.addgene.org/CRISPR
- **CHEN et al.** *Cell,* 19 December 2013, vol. 155 (7), 1479-91

- **FU, B.X.H ; SMITH, J.D. ; FUCHS, R.T et al.** Target-dependent nickase activities of the CRISPR-Cas nucleases Cpf1 and Cas9. *Nat Microbiol,* 2019, vol. 4, 888-897, https://doi.org/10.1038/s41564-019-0382-0
- **MARTIN et al.** *Helv. Chim. Acta,* 1995, vol. 78, 486-504
- The Concise Encyclopedia Of Polymer Science And Engineering. John Wiley & Sons, 1990, 858-859
- **ENGLISCH et al.** *Angewandte Chemie,* 1991, vol. 30, 613
- **SANGHVI, Y. S.** Antisense Research and Applications. CRC Press, 1993, 289-302
- Antisense Research and Applications. CRC Press, Boca Raton, 1993, 276-278
- **LANGE et al.** *J. Biol. Chem,* 2007, vol. 282, 5101-5105
- **AUSUBEL et al.** Current Protocols in Molecular Biology. John Wiley & Sons, 2003
- **JING, M ; BOWSER, M. T.** Methods for measuring aptamer-protein equilibria: A review. *Analytica Chimica Acta,* 2011, vol. 686 (1-2), 9-18
- **DERENZO et al.** Genetic Modification Strategies to Enhance CAR T Cell Persistence for Patients With Solid Tumors. *Front. Immunol,* 15 February 2019
- **SAMBROOK et al.** Molecular Cloning: A Laboratory Manual. Cold Spring Harbor Press, 2001
- **SAMBROOK ; RUSSELL.** Molecular Cloning: A Laboratory Manual. Cold Spring Harbor Press, 2001
- **SANTIAGO et al.** *PNAS,* 2008, vol. 105, 5809-5814
- **MOEHLE et al.** *PNAS,* 2007, vol. 104, 3055-3060
- **URNOV et al.** *Nature,* 2005, vol. 435, 646-651
- **LOMBARDO et al.** *Nat. Biotechnology,* 2007, vol. 25, 1298-1306
- **GASIUNAS et al.** *Proc Natl Acad Sci USA.,* 25 September 2012, vol. 109 (39), E2579-E2586
- Laboratory Techniques in Biochemistry and Molecular Biology-Hybridization with Nucleic Acid Probes. **TIJSSEN.** Overview of principles of hybridization and the strategy of nucleic acid probe assay. Elsevier, 1993
- **ADAMS et al.** The Biochemistry of the Nucleic Acids. 1992
- *Chem. Commun.,* 2020, vol. 56, 2123-2126
- **MING M ; REN Q ; PAN C ; HE Y ; ZHANG Y ; LIU S ; ZHONG Z ; WANG J ; MALZAHN AA ; WU J.** *Nature Plants,* 2020